# EUROPEAN PATENT SPECIFICATION

(11) **EP 2 953 973 B1**
(45) Date of publication and mention of the grant of the patent: **10.07.2019**
(21) Application number: 14703337.7
(22) Date of filing: 05.02.2014
(51) Int. Cl.: C07K 16/28

(54) **MUSCARINIC ACETYLCHOLINE RECEPTOR BINDING AGENTS AND USES THEREOF**
MUSKARINISCHE ACETYLCHOLINREZEPTORBINDER UND VERWENDUNGEN DAVON
AGENTS SE LIANT AUX RÉCEPTEURS MUSCARINIQUES DE L'ACÉTYLCHOLINE ET LEURS UTILISATIONS

(30) Priority: 05.02.2013 US 201361761136 P; 03.10.2013 US 201361961058 P
(43) Date of publication of application: 16.12.2015
(73) Proprietor: VIB vzw, 9052 Ghent (BE); Vrije Universiteit Brussel, 1050 Brussel (BE); The Board Of Trustees Of The University Of the Leland Stanford Junior University, Palo Alto, CA 94306-1106 (US)
(72) Inventor: STEYAERT, Jan, B-1652 Beersel (BE); PARDON, Els, B-3210 Lubbeek (BE); KOBILKA, Brian, Palo Alto, California 94306 (US); RING, Aaron, Palo Alto, California 94306 (US); KRUSE, Andrew, Roslindale, MA 02131 (US); MANGLIK, Aasish, Menlo Park, California 94025 (US)
(74) Representative: Nederlandsch Octrooibureau
(86) International application number: PCT/EP2014/052265
(87) International publication number: WO 2014/122183

(56) References cited:
- WO-A1-2012/007593
- J.-C. PETER ET AL: "Modulation of the M2 Muscarinic Acetylcholine Receptor Activity with Monoclonal Anti-M2 Receptor Antibody Fragments", JOURNAL OF BIOLOGICAL CHEMISTRY, vol. 279, no. 53, 14 October 2004 (2004-10-14), pages 55697-55706, XP055108410, ISSN: 0021-9258, DOI: 10.1074/jbc.M407213200
- ANDREW C. KRUSE ET AL: "Activation and allosteric modulation of a muscarinic acetylcholine receptor", NATURE, vol. 504, no. 7478, 1 December 2013 (2013-12-01), pages 101-106, XP055108116, ISSN: 0028-0836, DOI: 10.1038/nature12735
- KAZUKO HAGA ET AL: "Structure of the human M2 muscarinic acetylcholine receptor bound to an antagonist", NATURE, vol. 482, no. 7386, 25 January 2012 (2012-01-25), pages 547-551, XP055108185, ISSN: 0028-0836, DOI: 10.1038/nature10753
- K. J. GREGORY ET AL: "Identification of Orthosteric and Allosteric Site Mutations in M2 Muscarinic Acetylcholine Receptors That Contribute to Ligand-selective Signaling Bias", JOURNAL OF BIOLOGICAL CHEMISTRY, vol. 285, no. 10, 5 January 2010 (2010-01-05), pages 7459-7474, XP055108388, ISSN: 0021-9258, DOI: 10.1074/jbc.M109.094011
- ANDREW C. KRUSE ET AL: "Structure and dynamics of the M3 muscarinic acetylcholine receptor", NATURE, vol. 482, no. 7386, 22 February 2012 (2012-02-22), pages 552-556, XP055108187, ISSN: 0028-0836, DOI: 10.1038/nature10867

### SEQUENCE LISTING

<110> VIB VZW
   VRIJE UNIVERSITEIT BRUSSEL
   THE BOARD OF TRUSTEES OF THE LELAND STANFORD JUNIOR
   UNIVERSITY)
<120> Muscarinic acetylcholine receptor binding agents and uses thereof
<130> JaSt/M2R/471
<150> US 61/761,136
   <151> 2013-02-05
<150> US 61/961,058
   <151> 2013-10-03
<160> 166
<170> PatentIn version 3.5
<210> 1
   <211> 121
   <212> PRT
   <213> Lama glama
<400> 1
   Gln Val Gln Leu Gln Glu Ser Gly Gly Gly Leu Val Gln Ala Gly Asp
   1               5                   10                  15
   Ser Leu Arg Leu Ser Cys Ala Ala Ser Gly Phe Asp Phe Asp Asn Phe
               20                  25                  30
   Asp Asp Tyr Ala Ile Gly Trp Phe Arg Gln Ala Pro Gly Gln Glu Arg
           35                  40                  45
   Glu Gly Val Ser Cys Ile Asp Pro Ser Asp Gly Ser Thr Ile Tyr Ala
       50                  55                  60
   Asp Ser Ala Lys Gly Arg Phe Thr Ile Ser Ser Asp Asn Ala Glu Asn
   65                  70                  75                  80
   Thr Val Tyr Leu Gln Met Asn Ser Leu Lys Pro Glu Asp Thr Ala Val
                   85                  90                  95
   Tyr Val Cys Ser Ala Trp Thr Leu Phe His Ser Asp Glu Tyr Trp Gly
               100                 105                 110
   Gln Gly Thr Gln Val Thr Val Ser Ser
           115                 120
<210> 2
   <211> 121
   <212> PRT
   <213> Lama glama
<400> 2
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly His Thr Phe Ser Ser Ala
               20                  25                  30
   Arg Met Tyr Trp Val Arg Gln Ala Pro Gly Lys Glu Arg Glu Phe Val
           35                  40                  45
   Ala Ala Ile Ser Arg Ser Gly Phe Thr Tyr Ser Ala Asp Ser Val Lys
       50                  55                  60
   Gly Arg Phe Thr Ile Ser Arg Asp Ile Ala Asn Asn Thr Val Tyr Leu
   65                  70                  75                  80
   Gln Met Asn Ser Leu Gln Pro Glu Asp Thr Ala Ile Tyr Thr Cys Tyr
                   85                  90                  95
   Ala Ala Tyr Leu Asp Glu Phe Tyr Asn Asp Tyr Thr His Tyr Trp Gly
               100                 105                 110
   Leu Gly Thr Gln Val Thr Val Ser Ser
           115                 120
<210> 3
   <211> 122
   <212> PRT
   <213> Lama glama
<400> 3
   Gln Val Gln Leu Gln Glu Ser Gly Gly Gly Leu Val Gln Ala Gly Gly
   1               5                   10                  15
   Ser Leu Arg Leu Ser Cys Ala Phe Ser Gly Arg Thr Phe Ser Asn Tyr
               20                  25                  30
   Gly Met Gly Trp Phe Arg Gln Gly Pro Gly Lys Glu Arg Glu Phe Val
           35                  40                  45
   Ala Ser Ile Ser Trp Ser Gly Thr Met Thr Gln Tyr Ala Asp Ser Val
       50                  55                  60
   Lys Gly Arg Phe Thr Ile Ser Arg Asp Asn Ala Lys Asn Thr Val Tyr
   65                  70                  75                  80
   Leu Gln Met Asn Asn Leu Lys Pro Glu Asp Thr Ala Val Tyr Tyr Cys
                   85                  90                  95
   Ala Lys Tyr Phe Val Ser Trp Tyr Pro Glu Gly Ala Leu Gly Ser Trp
               100                 105                 110
   Gly Gln Gly Thr Gln Val Thr Val Ser Ser
           115                 120
<210> 4
   <211> 122
   <212> PRT
   <213> Lama glama
<400> 4
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Arg Thr Phe Ser Asn Tyr
               20                  25                  30
   Gly Met Gly Trp Phe Arg Gln Gly Pro Gly Lys Glu Arg Glu Phe Val
           35                  40                  45
   Ala Gly Ile Ser Trp Ser Gly Arg Ser Thr Tyr Tyr Ser Asp Ser Val
       50                  55                  60
   Lys Gly Arg Phe Thr Ile Ser Arg Asp Asn Ala Lys His Thr Met Tyr
   65                  70                  75                  80
   Leu Gln Met Asn Ser Leu Lys Pro Glu Asp Thr Ala Val Tyr Tyr Cys
                   85                  90                  95
   Thr Ala Lys Thr Tyr Gly Ala Ala Arg Asp Pro Val Tyr Asp Tyr Trp
               100                 105                 110
   Gly Pro Gly Thr Gln Val Thr Val Ser Ser
           115                 120
<210> 5
   <211> 123
   <212> PRT
   <213> Lama glama
<400> 5
   Gln Val Gln Leu Gln Glu Ser Gly Gly Gly Leu Val Gln Ala Gly Gly
   1               5                   10                  15
   Ser Leu Arg Leu Ser Cys Val Ala Ser Val Arg Thr Phe Ser Thr Tyr
               20                  25                  30
   Ser Met Gly Trp Phe Arg Gln Ala Pro Gly Lys Glu Arg Glu Phe Leu
           35                  40                  45
   Ala Gly Ile Ser Gly Ser Gly Asp Arg Thr Trp Tyr Arg Thr Ser Val
       50                  55                  60
   Lys Gly Arg Phe Ala Ile Ser Arg Asp Asn Gly Lys Asn Thr Ala Tyr
   65                  70                  75                  80
   Leu Gln Met Asn Ser Leu Glu Pro Glu Asp Thr Ala Val Tyr Tyr Cys
                   85                  90                  95
   Ala Ala Arg Ser Pro Lys Cys His Ser Arg Ser Thr Tyr Tyr Asp Tyr
               100                 105                 110
   Trp Gly Gln Gly Thr Gln Val Thr Val Ser Ser
           115                 120
<210> 6
   <211> 118
   <212> PRT
   <213> Lama glama
<400> 6
   Gln Val Gln Leu Gln Glu Ser Gly Gly Gly Leu Val His Ala Gly Gly
   1               5                   10                  15
   Ser Leu Arg Leu Ser Cys Thr Ala Ser Gly Arg Thr Ser Ser Arg Gly
               20                  25                  30
   Gly Met Gly Trp Phe Arg Gln Ala Pro Gly Lys Asp Arg Glu Phe Val
           35                  40                  45
   Ala Ala Ile Thr Trp Asn Ile Gly Ile Thr Tyr Tyr Glu Asp Ser Val
       50                  55                  60
   Lys Gly Arg Phe Thr Val Ser Arg Asp Asn Ala Lys Asn Thr Leu Tyr
   65                  70                  75                  80
   Leu Gln Met Asn Ser Leu Lys Pro Glu Asp Thr Ala Val Tyr Tyr Cys
                   85                  90                  95
   Tyr Gly Gly Gly Gly Tyr Tyr Gly Gln Asp Ser Trp Gly Gln Gly Thr
               100                 105                 110
   Gln Val Thr Val Ser Ser
                           115
<210> 7
   <211> 118
   <212> PRT
   <213> Lama glama
<400> 7
   Gln Val Gln Leu Gln Glu Ser Gly Gly Gly Leu Val Gln Ala Gly Gly
   1               5                   10                  15
   Ser Leu Arg Leu Ser Cys Thr Ala Ser Gly Arg Thr Ser Ser Arg Gly
               20                  25                  30
   Gly Met Ser Trp Phe Arg Gln Ala Pro Gly Lys Asp Arg Glu Phe Val
           35                  40                  45
   Ala Ala Ile Ser Trp Asn Ile Gly Ile Thr Tyr Tyr Gly Asp Ser Val
       50                  55                  60
   Lys Gly Arg Phe Thr Val Ser Arg Asp Asn Ala Lys Asn Thr Val Tyr
   65                  70                  75                  80
   Leu Gln Met Asn Ser Leu Lys Pro Glu Asp Thr Ala Leu Tyr Tyr Cys
                   85                  90                  95
   Ala Ala Gly Pro Arg Tyr Glu Asn Pro His Tyr Trp Gly Gln Gly Thr
               100                 105                 110
   Gln Val Thr Val Ser Ser
           115
<210> 8
   <211> 122
   <212> PRT
   <213> Lama glama
<400> 8
   Gln Val Gln Leu Gln Glu Ser Gly Gly Gly Leu Val Gln Ala Gly Gly
   1               5                   10                  15
   Ser Leu Arg Leu Ser Cys Ala Ala Ser Arg Arg Thr Gly Asn Met Tyr
               20                  25                  30
   Asn Met Ala Trp Phe Arg Gln Ala Pro Gly Lys Glu Arg Glu Phe Val
           35                  40                  45
   Ala Ala Ile Asn Trp Ser Gly Lys Asn Thr Tyr Tyr Ala Asp Ser Val
       50                  55                  60
   Lys Gly Arg Phe Thr Ile Ser Arg Asp Asn Ala Lys Thr Thr Val Tyr
   65                  70                  75                  80
   Leu Glu Met Asn Ser Leu Lys Pro Glu Asp Thr Ala Val Tyr Tyr Cys
                   85                  90                  95
   Ala Ala Gly Gly Cys Val Val Lys Ala Arg Asn Glu Cys Asp Phe Trp
               100                 105                 110
   Gly Gln Gly Thr Gln Val Thr Val Ser Ser
           115                 120
<210> 9
   <211> 124
   <212> PRT
   <213> Lama glama
<400> 9
   Gln Val Gln Leu Gln Glu Ser Gly Gly Gly Leu Val Gln Ala Gly Ala
   1               5                   10                  15
   Ser Leu Arg Leu Ser Cys Ala Ala Ser Gly Leu Thr Phe His Asp Tyr
               20                  25                  30
   Asn Met Gly Trp Phe Arg Gln Ala Pro Gly Lys Glu Arg Glu Ser Val
           35                  40                  45
   Ala Ala Ile Ser Arg Ser Gly Phe His Thr Tyr Tyr Ala Asp Ser Val
       50                  55                  60
   Lys Gly Arg Phe Thr Ile Ser Arg Asp Asn Ser Lys Asn Thr Met Tyr
   65                  70                  75                  80
   Leu Gln Met Asn Ser Leu Lys Pro Glu Asp Thr Ala Val Tyr Tyr Cys
                   85                  90                  95
   Ala Ala Arg Ser Thr Tyr Asn Ser Gly Arg Tyr Ser Arg Glu Tyr Asp
               100                 105                 110
   Tyr Trp Gly Gln Gly Thr Gln Val Thr Val Ser Ser
           115                 120
<210> 10
   <211> 121
   <212> PRT
   <213> Lama glama
<400> 10
   Gln Val Gln Leu Gln Glu Ser Gly Gly Gly Leu Val Gln Ala Gly Gly
   1 5 10 15
   Ser Leu Arg Leu Phe Cys Ala Ala Ser Gly Arg Thr Phe Ser Asn Tyr
   20 25 30
   Asn Met Gly Trp Phe Arg Leu Ala Pro Gly Lys Glu Arg Glu Phe Val
   35 40 45
   Ala Ala Ile Ser Arg Ser Ala Gly Ser Thr Ser Tyr Ala Asp Ser Val
   50 55 60
   Lys Gly Arg Phe Thr Ile Ser Arg Asp Asn Thr Asn Asn Ile Val Tyr
   65 70 75 80
   Leu Gln Met Asn Ser Val Glu Pro Glu Asp Thr Ala Val Tyr Tyr Cys
   85 90 95
   Ala Lys Tyr Thr Arg Thr Tyr Pro Tyr Tyr Gly Met Asn Tyr Trp Gly
   100 105 110
   Lys Gly Thr Gln Val Thr Val Ser Ser
   115 120
<210> 11
   <211> 122
   <212> PRT
   <213> Lama glama
<400> 11
   Gln Val Gln Leu Gln Glu Ser Gly Gly Gly Leu Val Gln Pro Glu Gly
   1 5 10 15
   Ser Leu Thr Leu Ala Cys Asp Thr Ser Gly Phe Thr Met Asn Tyr Tyr
   20 25 30
   Ala Ile Ala Trp Phe Arg Gln Ala Pro Glu Lys Glu Arg Glu Gly Leu
   35 40 45
   Ala Thr Ile Ser Ser Ile Asp Gly Arg Thr Tyr Tyr Ala Asp Ser Val
   50 55 60
   Lys Gly Arg Phe Thr Ile Ser Arg Asp Ser Ala Lys Asn Met Val Tyr
   65 70 75 80
   Leu Gln Met Asn Asn Leu Arg Pro Glu Asp Thr Ala Val Tyr Tyr Cys
   85 90 95
   Ser Ala Gly Pro Asp Tyr Ser Asp Tyr Gly Asp Glu Ser Glu Tyr Trp
               100                 105                 110
   Gly Gln Gly Thr Gln Val Thr Val Ser Ser
           115                 120
<210> 12
   <211> 122
   <212> PRT
   <213> Lama glama
<400> 12
   Gln Val Gln Leu Gln Glu Ser Gly Gly Gly Leu Val Gln Pro Gly Gly
   1               5                   10                  15
   Ser Leu Arg Leu Ser Cys Thr Ala Ser Gly Arg Ser Ile Ser Asn Ile
               20                  25                  30
   Tyr Ala Thr Thr Trp Tyr Arg Gln Ala Pro Gly Lys Gln Arg Glu Leu
           35                  40                  45
   Val Ala Val Phe Gly Tyr Ser Gly Gly Thr Thr Asn Tyr Ala Asp Ser
       50                  55                  60
   Val Lys Gly Arg Phe Thr Ile Ser Arg Asp Asp Ala Lys Asn Thr Val
   65                  70                  75                  80
   Tyr Leu Gln Met Asn Asn Leu Lys Pro Glu Asp Thr Ala Val Tyr Tyr
                   85                  90                  95
   Cys Asn Ala Val Lys Tyr Ile Pro Gly Arg Gly Glu Tyr Asp Tyr Trp
               100                 105                 110
   Gly Gln Gly Thr Gln Val Thr Val Ser Ser
           115                 120
<210> 13
   <211> 121
   <212> PRT
   <213> Lama glama
<400> 13
   Gln Val Gln Leu Gln Glu Ser Gly Gly Gly Ala Val Gln Ala Gly Asp
   1               5                   10                  15
   Ser Leu Arg Leu Ser Cys Ala Ala Ser Ala Arg Ser Phe Val Ser Tyr
               20                  25                  30
   Ala Met Gly Trp Phe Arg Gln Ala Pro Gly Lys Glu Arg Glu Phe Val
           35                  40                  45
   Ala Ser Ile Ser Trp Ser Gly Thr Met Thr Gln Tyr Ala Asp Ser Val
       50                  55                  60
   Lys Gly Arg Phe Thr Ile Ser Arg Asp Asp Ala Lys Asn Thr Val Tyr
   65                  70                  75                  80
   Leu Gln Met Asn Asn Leu Lys Pro Glu Asp Thr Ala Val Tyr Tyr Cys
                   85                  90                  95
   Asn Ala Val Lys Tyr Ile Pro Gly Arg Gly Glu Tyr Asp Tyr Trp Gly
               100                 105                 110
   Gln Gly Thr Gln Val Thr Val Ser Ser
           115                 120
<210> 14
   <211> 121
   <212> PRT
   <213> Lama glama
<400> 14
   Gln Val Gln Leu Gln Glu Ser Gly Gly Gly Leu Val Gln Ala Gly Gly
   1               5                   10                  15
   Ser Leu Arg Leu Ser Cys Thr Ala Ser Val Arg Thr Phe Ser Asn Tyr
               20                  25                  30
   Gly Met Gly Trp Phe Arg Gln Gly Pro Gly Lys Glu Arg Glu Phe Val
           35                  40                  45
   Ala Ser Ile Ser Trp Ser Gly Thr Met Thr Gln Tyr Ala Asp Ser Val
       50                  55                  60
   Lys Gly Arg Phe Thr Ile Ser Arg Asp Asn Ala Lys Ser Thr Val Tyr
   65                  70                  75                  80
   Leu Gln Met Asn Asn Leu Lys Pro Glu Asp Thr Ala Val Tyr Tyr Cys
                   85                  90                  95
   Asn Ala Val Lys Tyr Ile Pro Gly Arg Gly Glu Tyr Asp Tyr Trp Gly
               100                 105                 110
   Gln Gly Thr Gln Val Thr Val Ser Ser
           115                 120
<210> 15
   <211> 122
   <212> PRT
   <213> Lama glama
<400> 15
   Gln Val Gln Leu Gln Glu Ser Gly Gly Gly Leu Val Gln Ala Gly Ala
   1               5                   10                  15
   Ser Leu Asn Leu Ser Cys Ala Ala Ser Gly Gly Thr Phe Arg His Tyr
               20                  25                  30
   Gly Met Gly Trp Phe Arg Gln Ala Pro Gly Lys Glu Arg Glu Phe Val
           35                  40                  45
   Ala Ala Ile Ser Trp Thr Gly Gly Val Thr Phe Tyr Gly Asp Ser Val
       50                  55                  60
   Lys Gly Arg Phe Thr Ile Ser Arg Asp Asp Glu Lys Asn Thr Val Asp
   65                  70                  75                  80
   Leu Gln Met Asn Asn Leu Lys Ala Glu Asp Thr Ala Val Tyr Tyr Cys
                   85                  90                  95
   Asn Val Arg Gly Gly Arg Pro Ala Ser Arg Asp Asp Pro Gly Tyr Trp
               100                 105                 110
   Gly Gln Gly Thr Gln Val Thr Val Ser Ser
           115                 120
<210> 16
   <211> 117
   <212> PRT
   <213> Lama glama
<400> 16
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Arg Thr Phe Ser Asn Tyr
               20                  25                  30
   Ala Met Ser Trp Phe Arg Gln Ala Pro Gly Lys Glu Arg Gly Leu Val
           35                  40                  45
   Ala Thr Ile Tyr Arg Ser Gly Glu Gly Thr Tyr Tyr Leu Pro Ser Ala
       50                  55                  60
   Lys Gly Arg Phe Thr Val Ser Arg Asp Asn Ala Lys Asn Thr Ala Tyr
   65                  70                  75                  80
   Leu Gln Met Asn Ser Leu Lys Ala Glu Asp Thr Ala Val Tyr Tyr Cys
                   85                  90                  95
   Ala Val Met Ser Arg Gly Thr Trp Ser Met Trp Gly Gln Gly Thr Gln
               100                 105                 110
   Val Thr Val Ser Ser
           115
<210> 17
   <211> 123
   <212> PRT
   <213> Lama glama
<400> 17
   Gln Val Gln Leu Gln Glu Ser Gly Gly Gly Leu Val Gln Ala Gly Gly
   1               5                   10                  15
   Ser Leu Arg Leu Ser Cys Ala Ala Ser Gly Phe Ser Phe Asp Asp Tyr
               20                  25                  30
   Ala Ile Gly Trp Phe Arg Gln Ala Pro Gly Lys Glu Arg Glu Phe Val
           35                  40                  45
   Ala Arg Ile Asn Arg Ser Gly Tyr Asn Thr Phe Tyr Thr Asp Ser Val
       50                  55                  60
   Lys Gly Arg Phe Thr Ile Ser Arg Glu Asn Ala Lys Asn Thr Val Tyr
   65                  70                  75                  80
   Leu Gln Met Asn Asn Leu Lys Pro Glu Asp Thr Ala Val Tyr Tyr Cys
                   85                  90                  95
   Gly Ala Arg Tyr Ser Gly Ser Pro Phe Tyr Ser Gly Ala Tyr Asp Tyr
               100                 105                 110
   Trp Gly Gln Gly Thr Gln Val Thr Val Ser Ser
           115                 120
<210> 18
   <211> 114
   <212> PRT
   <213> Lama glama
<400> 18
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Ser Ile Ala Asn Leu Asn
               20                  25                  30
   Ser Val Gly Trp Tyr Arg Gln Ala Pro Gly Lys Glu Arg Glu Trp Val
           35                  40                  45
   Ala Ala Ile Leu Ala Gly Gly Phe Ala Thr Tyr Ala Asp Ser Val Lys
       50                  55                  60
   Gly Arg Phe Thr Ile Ser Arg Asp Asn Ala Lys Asn Thr Val Tyr Leu
   65                  70                  75                  80
   Gln Met Asn Ser Leu Lys Leu Glu Asp Thr Ala Val Tyr Tyr Cys Asn
                   85                  90                  95
   Thr Pro Asp Arg Pro Gly Ala Trp Gly Gln Gly Thr Gln Val Thr Val
               100                 105                 110
   Ser Ser
<210> 19
   <211> 122
   <212> PRT
   <213> Lama glama
<400> 19
   Gln Val Gln Leu Gln Glu Ser Gly Gly Gly Leu Val Glu Ser Gly Gly
   1               5                   10                  15
   Ser Leu Arg Leu Ser Cys Ala Ala Ser Gly Phe Thr Ala Asp Asp Tyr
               20                  25                  30
   Thr Met Ser Trp Val Arg Gln Ala Pro Gly Lys Gly Leu Glu Trp Val
           35                  40                  45
   Ser Thr Ile Ala Ala Ser Ser Val Ile Thr Phe Tyr Ala Asp Ser Val
       50                  55                  60
   Glu Gly Arg Phe Thr Ile Ser Arg Asp Asn Ala Glu Asn Ile Val Tyr
   65                  70                  75                  80
   Leu Gln Met Asn Gly Leu Lys Pro Glu Asp Thr Ala Val Tyr Tyr Cys
                   85                  90                  95
   Asn Thr Tyr Pro Pro Leu Trp Gly Arg Thr Pro Asp Glu Asp Tyr Trp
               100                 105                 110
   Gly Gln Gly Thr Gln Val Thr Val Ser Ser
   115 120
<210> 20
   <211> 25
   <212> PRT
   <213> Lama glama
<400> 20
   Gln Val Gln Leu Gln Glu Ser Gly Gly Gly Leu Val Gln Ala Gly Asp
   1 5 10 15
   Ser Leu Arg Leu Ser Cys Ala Ala Ser
   20 25
<210> 21
   <211> 25
   <212> PRT
   <213> Lama glama
<400> 21
   Gln Val Gln Leu Gln Glu Ser Gly Gly Gly Leu Val Gln Ala Gly Gly
   1 5 10 15
   Ser Leu Arg Leu Ser Cys Ala Ala Ser
   20 25
<210> 22
   <211> 25
   <212> PRT
   <213> Lama glama
<400> 22
   Gln Val Gln Leu Gln Glu Ser Gly Gly Gly Leu Val Gln Ala Gly Gly
   1 5 10 15
   Ser Leu Arg Leu Ser Cys Ala Phe Ser
   20 25
<210> 23
   <211> 25
   <212> PRT
   <213> Lama glama
<400> 23
   Gln Val Gln Leu Gln Glu Ser Gly Gly Gly Leu Val Gln Ala Gly Gly
   1 5 10 15
   Ser Leu Arg Leu Ser Cys Ala Ala Ser
   20 25
<210> 24
   <211> 25
   <212> PRT
   <213> Lama glama
<400> 24
   Gln Val Gln Leu Gln Glu Ser Gly Gly Gly Leu Val Gln Ala Gly Gly
   1               5                   10                  15
   Ser Leu Arg Leu Ser Cys Val Ala Ser
               20                  25
<210> 25
   <211> 25
   <212> PRT
   <213> Lama glama
<400> 25
   Gln Val Gln Leu Gln Glu Ser Gly Gly Gly Leu Val His Ala Gly Gly
   1               5                   10                  15
   Ser Leu Arg Leu Ser Cys Thr Ala Ser
               20                  25
<210> 26
   <211> 25
   <212> PRT
   <213> Lama glama
<400> 26
   Gln Val Gln Leu Gln Glu Ser Gly Gly Gly Leu Val Gln Ala Gly Gly
   1               5                   10                  15
   Ser Leu Arg Leu Ser Cys Thr Ala Ser
               20                  25
<210> 27
   <211> 25
   <212> PRT
   <213> Lama glama
<400> 27
   Gln Val Gln Leu Gln Glu Ser Gly Gly Gly Leu Val Gln Ala Gly Gly
   1               5                   10                  15
   Ser Leu Arg Leu Ser Cys Ala Ala Ser
               20                  25
<210> 28
   <211> 25
   <212> PRT
   <213> Lama glama
<400> 28
   Gln Val Gln Leu Gln Glu Ser Gly Gly Gly Leu Val Gln Ala Gly Ala
   1 5 10 15
   Ser Leu Arg Leu Ser Cys Ala Ala Ser
   20 25
<210> 29
   <211> 25
   <212> PRT
   <213> Lama glama
<400> 29
   Gln Val Gln Leu Gln Glu Ser Gly Gly Gly Leu Val Gln Ala Gly Gly
   1 5 10 15
   Ser Leu Arg Leu Phe Cys Ala Ala Ser
   20 25
<210> 30
   <211> 25
   <212> PRT
   <213> Lama glama
<400> 30
   Gln Val Gln Leu Gln Glu Ser Gly Gly Gly Leu Val Gln Pro Glu Gly
   1 5 10 15
   Ser Leu Thr Leu Ala Cys Asp Thr Ser
   20 25
<210> 31
   <211> 11
   <212> PRT
   <213> Lama glama
<400> 31
   Gly Phe Asp Phe Asp Asn Phe Asp Asp Tyr Ala
   1 5 10
<210> 32
   <211> 8
   <212> PRT
   <213> Lama glama
<400> 32
   Gly His Thr Phe Ser Ser Ala Arg
   1 5
<210> 33
   <211> 8
   <212> PRT
   <213> Lama glama
<400> 33
   Gly Arg Thr Phe Ser Asn Tyr Gly
   1 5
<210> 34
   <211> 8
   <212> PRT
   <213> Lama glama
<400> 34
   Gly Arg Thr Phe Ser Asn Tyr Gly
   1 5
<210> 35
   <211> 8
   <212> PRT
   <213> Lama glama
<400> 35
   Val Arg Thr Phe Ser Thr Tyr Ser
   1 5
<210> 36
   <211> 8
   <212> PRT
   <213> Lama glama
<400> 36
   Gly Arg Thr Ser Ser Arg Gly Gly
   1 5
<210> 37
   <211> 8
   <212> PRT
   <213> Lama glama
<400> 37
   Gly Arg Thr Ser Ser Arg Gly Gly
   1 5
<210> 38
   <211> 8
   <212> PRT
   <213> Lama glama
<400> 38
   Arg Arg Thr Gly Asn Met Tyr Asn
   1 5
<210> 39
   <211> 8
   <212> PRT
   <213> Lama glama
<400> 39
   Gly Leu Thr Phe His Asp Tyr Asn
   1 5
<210> 40
   <211> 8
   <212> PRT
   <213> Lama glama
<400> 40
   Gly Arg Thr Phe Ser Asn Tyr Asn
   1 5
<210> 41
   <211> 8
   <212> PRT
   <213> Lama glama
<400> 41
   Gly Phe Thr Met Asn Tyr Tyr Ala
   1 5
<210> 42
   <211> 17
   <212> PRT
   <213> Lama glama
<400> 42
   Ile Gly Trp Phe Arg Gln Ala Pro Gly Gln Glu Arg Glu Gly Val Ser
   1 5 10 15
   Cys
<210> 43
   <211> 17
   <212> PRT
   <213> Lama glama
<400> 43
   Met Tyr Trp Val Arg Gln Ala Pro Gly Lys Glu Arg Glu Phe Val Ala
   1 5 10 15
   Ala
<210> 44
   <211> 17
   <212> PRT
   <213> Lama glama
<400> 44
   Met Gly Trp Phe Arg Gln Gly Pro Gly Lys Glu Arg Glu Phe Val Ala
   1 5 10 15
   Ser
<210> 45
   <211> 17
   <212> PRT
   <213> Lama glama
<400> 45
   Met Gly Trp Phe Arg Gln Gly Pro Gly Lys Glu Arg Glu Phe Val Ala
   1 5 10 15
   Gly
<210> 46
   <211> 17
   <212> PRT
   <213> Lama glama
<400> 46
   Met Gly Trp Phe Arg Gln Ala Pro Gly Lys Glu Arg Glu Phe Leu Ala
   1 5 10 15
   Gly
<210> 47
   <211> 17
   <212> PRT
   <213> Lama glama
<400> 47
   Met Gly Trp Phe Arg Gln Ala Pro Gly Lys Asp Arg Glu Phe Val Ala
   1 5 10 15
   Ala
<210> 48
   <211> 17
   <212> PRT
   <213> Lama glama
<400> 48
   Met Ser Trp Phe Arg Gln Ala Pro Gly Lys Asp Arg Glu Phe Val Ala
   1 5 10 15
   Ala
<210> 49
   <211> 17
   <212> PRT
   <213> Lama glama
<400> 49
   Met Ala Trp Phe Arg Gln Ala Pro Gly Lys Glu Arg Glu Phe Val Ala
   1 5 10 15
   Ala
<210> 50
   <211> 17
   <212> PRT
   <213> Lama glama
<400> 50
   Met Gly Trp Phe Arg Gln Ala Pro Gly Lys Glu Arg Glu Ser Val Ala
   1 5 10 15
   Ala
<210> 51
   <211> 17
   <212> PRT
   <213> Lama glama
<400> 51
   Met Gly Trp Phe Arg Leu Ala Pro Gly Lys Glu Arg Glu Phe Val Ala
   1 5 10 15
   Ala
<210> 52
   <211> 17
   <212> PRT
   <213> Lama glama
<400> 52
   Ile Ala Trp Phe Arg Gln Ala Pro Glu Lys Glu Arg Glu Gly Leu Ala
   1 5 10 15
   Thr
<210> 53
   <211> 8
   <212> PRT
   <213> Lama glama
<400> 53
   Ile Asp Pro Ser Asp Gly Ser Thr
   1 5
<210> 54
   <211> 7
   <212> PRT
   <213> Lama glama
<400> 54
   Ile Ser Arg Ser Gly Phe Thr
   1 5
<210> 55
   <211> 8
   <212> PRT
   <213> Lama glama
<400> 55
   Ile Ser Trp Ser Gly Thr Met Thr
   1 5
<210> 56
   <211> 8
   <212> PRT
   <213> Lama glama
<400> 56
   Ile Ser Trp Ser Gly Arg Ser Thr
   1 5
<210> 57
   <211> 8
   <212> PRT
   <213> Lama glama
<400> 57
   Ile Ser Gly Ser Gly Asp Arg Thr
   1 5
<210> 58
   <211> 8
   <212> PRT
   <213> Lama glama
<400> 58
   Ile Thr Trp Asn Ile Gly Ile Thr
   1 5
<210> 59
   <211> 8
   <212> PRT
   <213> Lama glama
<400> 59
   Ile Ser Trp Asn Ile Gly Ile Thr
   1 5
<210> 60
   <211> 8
   <212> PRT
   <213> Lama glama
<400> 60
   Ile Asn Trp Ser Gly Lys Asn Thr
   1 5
<210> 61
   <211> 8
   <212> PRT
   <213> Lama glama
<400> 61
   Ile Ser Arg Ser Gly Phe His Thr
   1 5
<210> 62
   <211> 8
   <212> PRT
   <213> Lama glama
<400> 62
   Ile Ser Arg Ser Ala Gly Ser Thr
   1 5
<210> 63
   <211> 8
   <212> PRT
   <213> Lama glama
<400> 63
   Ile Ser Ser Ile Asp Gly Arg Thr
   1 5
<210> 64
   <211> 38
   <212> PRT
   <213> Lama glama
<400> 64
   Ile Tyr Ala Asp Ser Ala Lys Gly Arg Phe Thr Ile Ser Ser Asp Asn
   1               5                   10                  15
   Ala Glu Asn Thr Val Tyr Leu Gln Met Asn Ser Leu Lys Pro Glu Asp
               20                  25                  30
   Thr Ala Val Tyr Val Cys
           35
<210> 65
   <211> 38
   <212> PRT
   <213> Lama glama
<400> 65
   Tyr Ser Ala Asp Ser Val Lys Gly Arg Phe Thr Ile Ser Arg Asp Ile
   1               5                   10                  15
   Ala Asn Asn Thr Val Tyr Leu Gln Met Asn Ser Leu Gln Pro Glu Asp
               20                  25                  30
   Thr Ala Ile Tyr Thr Cys
           35
<210> 66
   <211> 38
   <212> PRT
   <213> Lama glama
<400> 66
   Gln Tyr Ala Asp Ser Val Lys Gly Arg Phe Thr Ile Ser Arg Asp Asn
   1               5                   10                  15
   Ala Lys Asn Thr Val Tyr Leu Gln Met Asn Asn Leu Lys Pro Glu Asp
               20                  25                  30
   Thr Ala Val Tyr Tyr Cys
           35
<210> 67
   <211> 38
   <212> PRT
   <213> Lama glama
<400> 67
   Tyr Tyr Ser Asp Ser Val Lys Gly Arg Phe Thr Ile Ser Arg Asp Asn
   1               5                   10                  15
   Ala Lys His Thr Met Tyr Leu Gln Met Asn Ser Leu Lys Pro Glu Asp
               20                  25                  30
   Thr Ala Val Tyr Tyr Cys
           35
<210> 68
   <211> 38
   <212> PRT
   <213> Lama glama
<400> 68
   Trp Tyr Arg Thr Ser Val Lys Gly Arg Phe Ala Ile Ser Arg Asp Asn
   1               5                   10                  15
   Gly Lys Asn Thr Ala Tyr Leu Gln Met Asn Ser Leu Glu Pro Glu Asp
               20                  25                  30
   Thr Ala Val Tyr Tyr Cys
           35
<210> 69
   <211> 38
   <212> PRT
   <213> Lama glama
<400> 69
   Tyr Tyr Glu Asp Ser Val Lys Gly Arg Phe Thr Val Ser Arg Asp Asn
   1               5                   10                  15
   Ala Lys Asn Thr Leu Tyr Leu Gln Met Asn Ser Leu Lys Pro Glu Asp
               20                  25                  30
   Thr Ala Val Tyr Tyr Cys
           35
<210> 70
   <211> 38
   <212> PRT
   <213> Lama glama
<400> 70
   Tyr Tyr Gly Asp Ser Val Lys Gly Arg Phe Thr Val Ser Arg Asp Asn
   1               5                   10                  15
   Ala Lys Asn Thr Val Tyr Leu Gln Met Asn Ser Leu Lys Pro Glu Asp
               20                  25                  30
   Thr Ala Leu Tyr Tyr Cys
           35
<210> 71
   <211> 38
   <212> PRT
   <213> Lama glama
<400> 71
   Tyr Tyr Ala Asp Ser Val Lys Gly Arg Phe Thr Ile Ser Arg Asp Asn
   1               5                   10                  15
   Ala Lys Thr Thr Val Tyr Leu Glu Met Asn Ser Leu Lys Pro Glu Asp
               20                  25                  30
   Thr Ala Val Tyr Tyr Cys
           35
<210> 72
   <211> 38
   <212> PRT
   <213> Lama glama
<400> 72
   Tyr Tyr Ala Asp Ser Val Lys Gly Arg Phe Thr Ile Ser Arg Asp Asn
   1               5                   10                  15
   Ser Lys Asn Thr Met Tyr Leu Gln Met Asn Ser Leu Lys Pro Glu Asp
               20                  25                  30
   Thr Ala Val Tyr Tyr Cys
           35
<210> 73
   <211> 38
   <212> PRT
   <213> Lama glama
<400> 73
   Ser Tyr Ala Asp Ser Val Lys Gly Arg Phe Thr Ile Ser Arg Asp Asn
   1               5                   10                  15
   Thr Asn Asn Ile Val Tyr Leu Gln Met Asn Ser Val Glu Pro Glu Asp
               20                  25                  30
   Thr Ala Val Tyr Tyr Cys
           35
<210> 74
   <211> 38
   <212> PRT
   <213> Lama glama
<400> 74
   Tyr Tyr Ala Asp Ser Val Lys Gly Arg Phe Thr Ile Ser Arg Asp Ser
   1               5                   10                  15
   Ala Lys Asn Met Val Tyr Leu Gln Met Asn Asn Leu Arg Pro Glu Asp
               20                  25                  30
   Thr Ala Val Tyr Tyr Cys
           35
<210> 75
   <211> 11
   <212> PRT
   <213> Lama glama
<400> 75
   Ser Ala Trp Thr Leu Phe His Ser Asp Glu Tyr
   1               5                   10
<210> 76
   <211> 15
   <212> PRT
   <213> Lama glama
<400> 76
   Tyr Ala Ala Tyr Leu Asp Glu Phe Tyr Asn Asp Tyr Thr His Tyr
   1               5                   10                  15
<210> 77
   <211> 15
   <212> PRT
   <213> Lama glama
<400> 77
   Ala Lys Tyr Phe Val Ser Trp Tyr Pro Glu Gly Ala Leu Gly Ser
   1               5                   10                  15
<210> 78
   <211> 15
   <212> PRT
   <213> Lama glama
<400> 78
   Thr Ala Lys Thr Tyr Gly Ala Ala Arg Asp Pro Val Tyr Asp Tyr
   1 5 10 15
<210> 79
   <211> 16
   <212> PRT
   <213> Lama glama
<400> 79
   Ala Ala Arg Ser Pro Lys Cys His Ser Arg Ser Thr Tyr Tyr Asp Tyr
   1 5 10 15
<210> 80
   <211> 11
   <212> PRT
   <213> Lama glama
<400> 80
   Tyr Gly Gly Gly Gly Tyr Tyr Gly Gln Asp Ser
   1 5 10
<210> 81
   <211> 11
   <212> PRT
   <213> Lama glama
<400> 81
   Ala Ala Gly Pro Arg Tyr Glu Asn Pro His Tyr
   1 5 10
<210> 82
   <211> 15
   <212> PRT
   <213> Lama glama
<400> 82
   Ala Ala Gly Gly Cys Val Val Lys Ala Arg Asn Glu Cys Asp Phe
   1 5 10 15
<210> 83
   <211> 17
   <212> PRT
   <213> Lama glama
<400> 83
   Ala Ala Arg Ser Thr Tyr Asn Ser Gly Arg Tyr Ser Arg Glu Tyr Asp
   1 5 10 15
   Tyr
<210> 84
   <211> 14
   <212> PRT
   <213> Lama glama
<400> 84
   Ala Lys Tyr Thr Arg Thr Tyr Pro Tyr Tyr Gly Met Asn Tyr
   1 5 10
<210> 85
   <211> 15
   <212> PRT
   <213> Lama glama
<400> 85
   Ser Ala Gly Pro Asp Tyr Ser Asp Tyr Gly Asp Glu Ser Glu Tyr
   1 5 10 15
<210> 86
   <211> 11
   <212> PRT
   <213> Lama glama
<400> 86
   Trp Gly Gln Gly Thr Gln Val Thr Val Ser Ser
   1 5 10
<210> 87
   <211> 11
   <212> PRT
   <213> Lama glama
<400> 87
   Trp Gly Leu Gly Thr Gln Val Thr Val Ser Ser
   1 5 10
<210> 88
   <211> 11
   <212> PRT
   <213> Lama glama
<400> 88
   Trp Gly Gln Gly Thr Gln Val Thr Val Ser Ser
   1 5 10
<210> 89
   <211> 11
   <212> PRT
   <213> Lama glama
<400> 89
   Trp Gly Pro Gly Thr Gln Val Thr Val Ser Ser
   1 5 10
<210> 90
   <211> 11
   <212> PRT
   <213> Lama glama
<400> 90
   Trp Gly Gln Gly Thr Gln Val Thr Val Ser Ser
   1 5 10
<210> 91
   <211> 11
   <212> PRT
   <213> Lama glama
<400> 91
   Trp Gly Gln Gly Thr Gln Val Thr Val Ser Ser
   1 5 10
<210> 92
   <211> 11
   <212> PRT
   <213> Lama glama
<400> 92
   Trp Gly Gln Gly Thr Gln Val Thr Val Ser Ser
   1 5 10
<210> 93
   <211> 11
   <212> PRT
   <213> Lama glama
<400> 93
   Trp Gly Gln Gly Thr Gln Val Thr Val Ser Ser
   1 5 10
<210> 94
   <211> 11
   <212> PRT
   <213> Lama glama
<400> 94
   Trp Gly Gln Gly Thr Gln Val Thr Val Ser Ser
   1 5 10
<210> 95
   <211> 11
   <212> PRT
   <213> Lama glama
<400> 95
   Trp Gly Lys Gly Thr Gln Val Thr Val Ser Ser
   1               5                   10
<210> 96
   <211> 11
   <212> PRT
   <213> Lama glama
<400> 96
   Trp Gly Gln Gly Thr Gln Val Thr Val Ser Ser
   1               5                   10
<210> 97
   <211> 25
   <212> PRT
   <213> Lama glama
<400> 97
   Gln Val Gln Leu Gln Glu Ser Gly Gly Gly Leu Val Gln Pro Gly Gly
   1               5                   10                  15
   Ser Leu Arg Leu Ser Cys Thr Ala Ser
               20                  25
<210> 98
   <211> 25
   <212> PRT
   <213> Lama glama
<400> 98
   Gln Val Gln Leu Gln Glu Ser Gly Gly Gly Ala Val Gln Ala Gly Asp
   1               5                   10                  15
   Ser Leu Arg Leu Ser Cys Ala Ala Ser
               20                  25
<210> 99
   <211> 25
   <212> PRT
   <213> Lama glama
<400> 99
   Gln Val Gln Leu Gln Glu Ser Gly Gly Gly Leu Val Gln Ala Gly Gly
   1 5 10 15
   Ser Leu Arg Leu Ser Cys Thr Ala Ser
   20 25
<210> 100
   <211> 25
   <212> PRT
   <213> Lama glama
<400> 100
   Gln Val Gln Leu Gln Glu Ser Gly Gly Gly Leu Val Gln Ala Gly Ala
   1 5 10 15
   Ser Leu Asn Leu Ser Cys Ala Ala Ser
   20 25
<210> 101
   <211> 25
   <212> PRT
   <213> Lama glama
<400> 101
   Gln Val Gln Leu Gln Glu Ser Gly Gly Gly Leu Val Gln Ala Gly Gly
   1 5 10 15
   Ser Leu Arg Leu Ser Cys Ala Ala Ser
   20 25
<210> 102
   <211> 25
   <212> PRT
   <213> Lama glama
<400> 102
   Gln Val Gln Leu Gln Glu Ser Gly Gly Gly Leu Val Gln Ala Gly Gly
   1 5 10 15
   Ser Leu Arg Leu Ser Cys Ala Ala Ser
   20 25
<210> 103
   <211> 25
   <212> PRT
   <213> Lama glama
<400> 103
   Gln Val Gln Leu Gln Glu Ser Gly Gly Gly Leu Val Gln Pro Gly Gly
   1 5 10 15
   Ser Leu Arg Leu Ser Cys Ala Ala Ser
   20 25
<210> 104
   <211> 25
   <212> PRT
   <213> Lama glama
<400> 104
   Gln Val Gln Leu Gln Glu Ser Gly Gly Gly Leu Val Glu Ser Gly Gly
   1 5 10 15
   Ser Leu Arg Leu Ser Cys Ala Ala Ser
   20 25
<210> 105
   <211> 9
   <212> PRT
   <213> Lama glama
<400> 105
   Gly Arg Ser Ile Ser Asn Ile Tyr Ala
   1 5
<210> 106
   <211> 8
   <212> PRT
   <213> Lama glama
<400> 106
   Ala Arg Ser Phe Val Ser Tyr Ala
   1 5
<210> 107
   <211> 8
   <212> PRT
   <213> Lama glama
<400> 107
   Val Arg Thr Phe Ser Asn Tyr Gly
   1 5
<210> 108
   <211> 8
   <212> PRT
   <213> Lama glama
<400> 108
   Gly Gly Thr Phe Arg His Tyr Gly
   1 5
<210> 109
   <211> 8
   <212> PRT
   <213> Lama glama
<400> 109
   Gly Arg Thr Phe Ser Asn Tyr Ala
   1 5
<210> 110
   <211> 8
   <212> PRT
   <213> Lama glama
<400> 110
   Gly Phe Ser Phe Asp Asp Tyr Ala
   1 5
<210> 111
   <211> 8
   <212> PRT
   <213> Lama glama
<400> 111
   Gly Ser Ile Ala Asn Leu Asn Ser
   1 5
<210> 112
   <211> 8
   <212> PRT
   <213> Lama glama
<400> 112
   Gly Phe Thr Ala Asp Asp Tyr Thr
   1 5
<210> 113
   <211> 17
   <212> PRT
   <213> Lama glama
<400> 113
   Thr Thr Trp Tyr Arg Gln Ala Pro Gly Lys Gln Arg Glu Leu Val Ala
   1 5 10 15
   Val
<210> 114
   <211> 17
   <212> PRT
   <213> Lama glama
<400> 114
   Met Gly Trp Phe Arg Gln Ala Pro Gly Lys Glu Arg Glu Phe Val Ala
   1 5 10 15
   Ser
<210> 115
   <211> 17
   <212> PRT
   <213> Lama glama
<400> 115
   Met Gly Trp Phe Arg Gln Gly Pro Gly Lys Glu Arg Glu Phe Val Ala
   1 5 10 15
   Ser
<210> 116
   <211> 17
   <212> PRT
   <213> Lama glama
<400> 116
   Met Gly Trp Phe Arg Gln Ala Pro Gly Lys Glu Arg Glu Phe Val Ala
   1 5 10 15
   Ala
<210> 117
   <211> 17
   <212> PRT
   <213> Lama glama
<400> 117
   Met Ser Trp Phe Arg Gln Ala Pro Gly Lys Glu Arg Gly Leu Val Ala
   1 5 10 15
   Thr
<210> 118
   <211> 17
   <212> PRT
   <213> Lama glama
<400> 118
   Ile Gly Trp Phe Arg Gln Ala Pro Gly Lys Glu Arg Glu Phe Val Ala
   1               5                   10                  15
   Arg
<210> 119
   <211> 17
   <212> PRT
   <213> Lama glama
<400> 119
   Val Gly Trp Tyr Arg Gln Ala Pro Gly Lys Glu Arg Glu Trp Val Ala
   1               5                   10                  15
   Ala
<210> 120
   <211> 17
   <212> PRT
   <213> Lama glama
<400> 120
   Met Ser Trp Val Arg Gln Ala Pro Gly Lys Gly Leu Glu Trp Val Ser
   1               5                   10                  15
   Thr
<210> 121
   <211> 8
   <212> PRT
   <213> Lama glama
<400> 121
   Phe Gly Tyr Ser Gly Gly Thr Thr
   1               5
<210> 122
   <211> 8
   <212> PRT
   <213> Lama glama
<400> 122
   Ile Ser Trp Ser Gly Thr Met Thr
   1               5
<210> 123
   <211> 8
   <212> PRT
   <213> Lama glama
<400> 123
   Ile Ser Trp Ser Gly Thr Met Thr
   1 5
<210> 124
   <211> 8
   <212> PRT
   <213> Lama glama
<400> 124
   Ile Ser Trp Thr Gly Gly Val Thr
   1 5
<210> 125
   <211> 8
   <212> PRT
   <213> Lama glama
<400> 125
   Ile Tyr Arg Ser Gly Glu Gly Thr
   1 5
<210> 126
   <211> 8
   <212> PRT
   <213> Lama glama
<400> 126
   Ile Asn Arg Ser Gly Tyr Asn Thr
   1 5
<210> 127
   <211> 7
   <212> PRT
   <213> Lama glama
<400> 127
   Ile Leu Ala Gly Gly Phe Ala
   1 5
<210> 128
   <211> 8
   <212> PRT
   <213> Lama glama
<400> 128
   Ile Ala Ala Ser Ser Val Ile Thr
   1 5
<210> 129
   <211> 38
   <212> PRT
   <213> Lama glama
<400> 129
   Asn Tyr Ala Asp Ser Val Lys Gly Arg Phe Thr Ile Ser Arg Asp Asp
   1               5                   10                  15
   Ala Lys Asn Thr Val Tyr Leu Gln Met Asn Asn Leu Lys Pro Glu Asp
               20                  25                  30
   Thr Ala Val Tyr Tyr Cys
           35
<210> 130
   <211> 38
   <212> PRT
   <213> Lama glama
<400> 130
   Gln Tyr Ala Asp Ser Val Lys Gly Arg Phe Thr Ile Ser Arg Asp Asp
   1               5                   10                  15
   Ala Lys Asn Thr Val Tyr Leu Gln Met Asn Asn Leu Lys Pro Glu Asp
               20                  25                  30
   Thr Ala Val Tyr Tyr Cys
           35
<210> 131
   <211> 38
   <212> PRT
   <213> Lama glama
<400> 131
   Gln Tyr Ala Asp Ser Val Lys Gly Arg Phe Thr Ile Ser Arg Asp Asn
   1               5                   10                  15
   Ala Lys Ser Thr Val Tyr Leu Gln Met Asn Asn Leu Lys Pro Glu Asp
               20                  25                  30
   Thr Ala Val Tyr Tyr Cys
           35
<210> 132
   <211> 38
   <212> PRT
   <213> Lama glama
<400> 132
   Phe Tyr Gly Asp Ser Val Lys Gly Arg Phe Thr Ile Ser Arg Asp Asp
   1               5                   10                  15
   Glu Lys Asn Thr Val Asp Leu Gln Met Asn Asn Leu Lys Ala Glu Asp
               20                  25                  30
   Thr Ala Val Tyr Tyr Cys
           35
<210> 133
   <211> 38
   <212> PRT
   <213> Lama glama
<400> 133
   Tyr Tyr Leu Pro Ser Ala Lys Gly Arg Phe Thr Val Ser Arg Asp Asn
   1               5                   10                  15
   Ala Lys Asn Thr Ala Tyr Leu Gln Met Asn Ser Leu Lys Ala Glu Asp
               20                  25                  30
   Thr Ala Val Tyr Tyr Cys
           35
<210> 134
   <211> 38
   <212> PRT
   <213> Lama glama
<400> 134
   Phe Tyr Thr Asp Ser Val Lys Gly Arg Phe Thr Ile Ser Arg Glu Asn
   1               5                   10                  15
   Ala Lys Asn Thr Val Tyr Leu Gln Met Asn Asn Leu Lys Pro Glu Asp
               20                  25                  30
   Thr Ala Val Tyr Tyr Cys
           35
<210> 135
   <211> 37
   <212> PRT
   <213> Lama glama
<400> 135
   Tyr Ala Asp Ser Val Lys Gly Arg Phe Thr Ile Ser Arg Asp Asn Ala
   1 5 10 15
   Lys Asn Thr Val Tyr Leu Gln Met Asn Ser Leu Lys Leu Glu Asp Thr
   20 25 30
   Ala Val Tyr Tyr Cys
   35
<210> 136
   <211> 38
   <212> PRT
   <213> Lama glama
<400> 136
   Phe Tyr Ala Asp Ser Val Glu Gly Arg Phe Thr Ile Ser Arg Asp Asn
   1 5 10 15
   Ala Glu Asn Ile Val Tyr Leu Gln Met Asn Gly Leu Lys Pro Glu Asp
   20 25 30
   Thr Ala Val Tyr Tyr Cys
   35
<210> 137
   <211> 14
   <212> PRT
   <213> Lama glama
<400> 137
   Asn Ala Val Lys Tyr Ile Pro Gly Arg Gly Glu Tyr Asp Tyr
   1 5 10
<210> 138
   <211> 14
   <212> PRT
   <213> Lama glama
<400> 138
   Asn Ala Val Lys Tyr Ile Pro Gly Arg Gly Glu Tyr Asp Tyr
   1 5 10
<210> 139
   <211> 14
   <212> PRT
   <213> Lama glama
<400> 139
   Asn Ala Val Lys Tyr Ile Pro Gly Arg Gly Glu Tyr Asp Tyr
   1 5 10
<210> 140
   <211> 15
   <212> PRT
   <213> Lama glama
<400> 140
   Asn Val Arg Gly Gly Arg Pro Ala Ser Arg Asp Asp Pro Gly Tyr
   1 5 10 15
<210> 141
   <211> 10
   <212> PRT
   <213> Lama glama
<400> 141
   Ala Val Met Ser Arg Gly Thr Trp Ser Met
   1 5 10
<210> 142
   <211> 16
   <212> PRT
   <213> Lama glama
<400> 142
   Gly Ala Arg Tyr Ser Gly Ser Pro Phe Tyr Ser Gly Ala Tyr Asp Tyr
   1 5 10 15
<210> 143
   <211> 9
   <212> PRT
   <213> Lama glama
<400> 143
   Asn Thr Pro Asp Arg Pro Gly Ala Ser
   1 5
<210> 144
   <211> 15
   <212> PRT
   <213> Lama glama
<400> 144
   Asn Thr Tyr Pro Pro Leu Trp Gly Arg Thr Pro Asp Glu Asp Tyr
   1 5 10 15
<210> 145
   <211> 11
   <212> PRT
   <213> Lama glama
<400> 145
   Trp Gly Gln Gly Thr Gln Val Thr Val Ser Ser
   1 5 10
<210> 146
   <211> 11
   <212> PRT
   <213> Lama glama
<400> 146
   Trp Gly Gln Gly Thr Gln Val Thr Val Ser Ser
   1 5 10
<210> 147
   <211> 11
   <212> PRT
   <213> Lama glama
<400> 147
   Trp Gly Gln Gly Thr Gln Val Thr Val Ser Ser
   1 5 10
<210> 148
   <211> 11
   <212> PRT
   <213> Lama glama
<400> 148
   Trp Gly Gln Gly Thr Gln Val Thr Val Ser Ser
   1 5 10
<210> 149
   <211> 11
   <212> PRT
   <213> Lama glama
<400> 149
   Trp Gly Gln Gly Thr Gln Val Thr Val Ser Ser
   1 5 10
<210> 150
   <211> 11
   <212> PRT
   <213> Lama glama
<400> 150
   Trp Gly Gln Gly Thr Gln Val Thr Val Ser Ser
   1 5 10
<210> 151
   <211> 11
   <212> PRT
   <213> Lama glama
<400> 151
   Trp Gly Gln Gly Thr Gln Val Thr Val Ser Ser
   1               5                   10
<210> 152
   <211> 11
   <212> PRT
   <213> Lama glama
<400> 152
   Trp Gly Gln Gly Thr Gln Val Thr Val Ser Ser
   1               5                   10
<210> 153
   <211> 466
   <212> PRT
   <213> Homo sapiens
<400> 153
   Met Asn Asn Ser Thr Asn Ser Ser Asn Asn Ser Leu Ala Leu Thr Ser
   1               5                   10                  15
   Pro Tyr Lys Thr Phe Glu Val Val Phe Ile Val Leu Val Ala Gly Ser
               20                  25                  30
   Leu Ser Leu Val Thr Ile Ile Gly Asn Ile Leu Val Met Val Ser Ile
           35                  40                  45
   Lys Val Asn Arg His Leu Gln Thr Val Asn Asn Tyr Phe Leu Phe Ser
       50                  55                  60
   Leu Ala Cys Ala Asp Leu Ile Ile Gly Val Phe Ser Met Asn Leu Tyr
   65                  70                  75                  80
   Thr Leu Tyr Thr Val Ile Gly Tyr Trp Pro Leu Gly Pro Val Val Cys
                   85                  90                  95
   Asp Leu Trp Leu Ala Leu Asp Tyr Val Val Ser Asn Ala Ser Val Met
               100                 105                 110
   Asn Leu Leu Ile Ile Ser Phe Asp Arg Tyr Phe Cys Val Thr Lys Pro
           115                 120                 125
   Leu Thr Tyr Pro Val Lys Arg Thr Thr Lys Met Ala Gly Met Met Ile
       130                 135                 140
   Ala Ala Ala Trp Val Leu Ser Phe Ile Leu Trp Ala Pro Ala Ile Leu
   145                 150                 155                 160
   Phe Trp Gln Phe Ile Val Gly Val Arg Thr Val Glu Asp Gly Glu Cys
                   165                 170                 175
   Tyr Ile Gln Phe Phe Ser Asn Ala Ala Val Thr Phe Gly Thr Ala Ile
               180                 185                 190
   Ala Ala Phe Tyr Leu Pro Val Ile Ile Met Thr Val Leu Tyr Trp His
           195                 200                 205
   Ile Ser Arg Ala Ser Lys Ser Arg Ile Lys Lys Asp Lys Lys Glu Pro
       210                 215                 220
   Val Ala Asn Gln Asp Pro Val Ser Pro Ser Leu Val Gln Gly Arg Ile
   225                 230                 235                 240
   Val Lys Pro Asn Asn Asn Asn Met Pro Ser Ser Asp Asp Gly Leu Glu
                   245                 250                 255
   His Asn Lys Ile Gln Asn Gly Lys Ala Pro Arg Asp Pro Val Thr Glu
               260                 265                 270
   Asn Cys Val Gln Gly Glu Glu Lys Glu Ser Ser Asn Asp Ser Thr Ser
           275                 280                 285
   Val Ser Ala Val Ala Ser Asn Met Arg Asp Asp Glu Ile Thr Gln Asp
       290                 295                 300
   Glu Asn Thr Val Ser Thr Ser Leu Gly His Ser Lys Asp Glu Asn Ser
   305                 310                 315                 320
   Lys Gln Thr Cys Ile Arg Ile Gly Thr Lys Thr Pro Lys Ser Asp Ser
                   325                 330                 335
   Cys Thr Pro Thr Asn Thr Thr Val Glu Val Val Gly Ser Ser Gly Gln
               340                 345                 350
   Asn Gly Asp Glu Lys Gln Asn Ile Val Ala Arg Lys Ile Val Lys Met
           355                 360                 365
   Thr Lys Gln Pro Ala Lys Lys Lys Pro Pro Pro Ser Arg Glu Lys Lys
       370                 375                 380
   Val Thr Arg Thr Ile Leu Ala Ile Leu Leu Ala Phe Ile Ile Thr Trp
   385                 390                 395                 400
   Ala Pro Tyr Asn Val Met Val Leu Ile Asn Thr Phe Cys Ala Pro Cys
                   405                 410                 415
   Ile Pro Asn Thr Val Trp Thr Ile Gly Tyr Trp Leu Cys Tyr Ile Asn
               420                 425                 430
   Ser Thr Ile Asn Pro Ala Cys Tyr Ala Leu Cys Asn Ala Thr Phe Lys
   435 440 445
   Lys Thr Phe Lys His Leu Leu Met Cys His Tyr Lys Asn Ile Gly Ala
   450 455 460
   Thr Arg
   465
<210> 154
   <211> 466
   <212> PRT
   <213> Mus musculus
<400> 154
   Met Asn Asn Ser Thr Asn Ser Ser Asn Asn Gly Leu Ala Ile Thr Ser
   1 5 10 15
   Pro Tyr Lys Thr Phe Glu Val Val Phe Ile Val Leu Val Ala Gly Ser
   20 25 30
   Leu Ser Leu Val Thr Ile Ile Gly Asn Ile Leu Val Met Val Ser Ile
   35 40 45
   Lys Val Asn Arg His Leu Gln Thr Val Asn Asn Tyr Phe Leu Phe Ser
   50 55 60
   Leu Ala Cys Ala Asp Leu Ile Ile Gly Val Phe Ser Met Asn Leu Tyr
   65 70 75 80
   Thr Leu Tyr Thr Val Ile Gly Tyr Trp Pro Leu Gly Pro Val Val Cys
   85 90 95
   Asp Leu Trp Leu Ala Leu Asp Tyr Val Val Ser Asn Ala Ser Val Met
   100 105 110
   Asn Leu Leu Ile Ile Ser Phe Asp Arg Tyr Phe Cys Val Thr Lys Pro
   115 120 125
   Leu Thr Tyr Pro Val Lys Arg Thr Thr Lys Met Ala Gly Met Met Ile
   130 135 140
   Ala Ala Ala Trp Val Leu Ser Phe Ile Leu Trp Ala Pro Ala Ile Leu
   145 150 155 160
   Phe Trp Gln Phe Ile Val Gly Val Arg Thr Val Glu Asp Gly Glu Cys
   165 170 175
   Tyr Ile Gln Phe Phe Ser Asn Ala Ala Val Thr Phe Gly Thr Ala Ile
   180 185 190
   Ala Ala Phe Tyr Leu Pro Val Ile Ile Met Thr Val Leu Tyr Trp His
   195 200 205
   Ile Ser Arg Ala Ser Lys Ser Arg Ile Lys Lys Glu Lys Lys Glu Pro
   210 215 220
   Val Ala Asn Gln Asp Pro Val Ser Pro Ser Leu Val Gln Gly Arg Ile
   225 230 235 240
   Val Lys Pro Asn Asn Asn Asn Met Pro Gly Gly Asp Gly Gly Leu Glu
   245 250 255
   His Asn Lys Ile Gln Asn Gly Lys Ala Pro Arg Asp Gly Gly Thr Glu
   260 265 270
   Asn Cys Val Gln Gly Glu Glu Lys Glu Ser Ser Asn Asp Ser Thr Ser
   275 280 285
   Val Ser Ala Val Ala Ser Asn Met Arg Asp Asp Glu Ile Thr Gln Asp
   290 295 300
   Glu Asn Thr Val Ser Thr Ser Leu Gly His Ser Lys Asp Asp Asn Ser
   305 310 315 320
   Arg Gln Thr Cys Ile Lys Ile Val Thr Lys Thr Gln Lys Gly Asp Ala
   325 330 335
   Cys Thr Pro Thr Ser Thr Thr Val Glu Leu Val Gly Ser Ser Gly Gln
   340 345 350
   Asn Gly Asp Glu Lys Gln Asn Ile Val Ala Arg Lys Ile Val Lys Met
   355 360 365
   Thr Lys Gln Pro Ala Lys Lys Lys Pro Pro Pro Ser Arg Glu Lys Lys
   370 375 380
   Val Thr Arg Thr Ile Leu Ala Ile Leu Leu Ala Phe Ile Ile Thr Trp
   385 390 395 400
   Ala Pro Tyr Asn Val Met Val Leu Ile Asn Thr Phe Cys Ala Pro Cys
   405 410 415
   Ile Pro Asn Thr Val Trp Thr Ile Gly Tyr Trp Leu Cys Tyr Ile Asn
   420 425 430
   Ser Thr Ile Asn Pro Ala Cys Tyr Ala Leu Cys Asn Ala Thr Phe Lys
   435 440 445
   Lys Thr Phe Lys His Leu Leu Met Cys His Tyr Lys Asn Ile Gly Ala
   450 455 460
   Thr Arg
   465
<210> 155
   <211> 466
   <212> PRT
   <213> Rattus norvegicus
<400> 155
   Met Asn Asn Ser Thr Asn Ser Ser Asn Asn Gly Leu Ala Ile Thr Ser
   1               5                   10                  15
   Pro Tyr Lys Thr Phe Glu Val Val Phe Ile Val Leu Val Ala Gly Ser
               20                  25                  30
   Leu Ser Leu Val Thr Ile Ile Gly Asn Ile Leu Val Met Val Ser Ile
           35                  40                  45
   Lys Val Asn Arg His Leu Gln Thr Val Asn Asn Tyr Phe Leu Phe Ser
       50                  55                  60
   Leu Ala Cys Ala Asp Leu Ile Ile Gly Val Phe Ser Met Asn Leu Tyr
   65                  70                  75                  80
   Thr Leu Tyr Thr Val Ile Gly Tyr Trp Pro Leu Gly Pro Val Val Cys
                   85                  90                  95
   Asp Leu Trp Leu Ala Leu Asp Tyr Val Val Ser Asn Ala Ser Val Met
               100                 105                 110
   Asn Leu Leu Ile Ile Ser Phe Asp Arg Tyr Phe Cys Val Thr Lys Pro
           115                 120                 125
   Leu Thr Tyr Pro Val Lys Arg Thr Thr Lys Met Ala Gly Met Met Ile
       130                 135                 140
   Ala Ala Ala Trp Val Leu Ser Phe Ile Leu Trp Ala Pro Ala Ile Leu
   145                 150                 155                 160
   Phe Trp Gln Phe Ile Val Gly Val Arg Thr Val Glu Asp Gly Glu Cys
                   165                 170                 175
   Tyr Ile Gln Phe Phe Ser Asn Ala Ala Val Thr Phe Gly Thr Ala Ile
               180                 185                 190
   Ala Ala Phe Tyr Leu Pro Val Ile Ile Met Thr Val Leu Tyr Trp His
           195                 200                 205
   Ile Ser Arg Ala Ser Lys Ser Arg Ile Lys Lys Glu Lys Lys Glu Pro
       210                 215                 220
   Val Ala Asn Gln Asp Pro Val Ser Pro Ser Leu Val Gln Gly Arg Ile
   225                 230                 235                 240
   Val Lys Pro Asn Asn Asn Asn Met Pro Gly Gly Asp Gly Gly Leu Glu
                   245                 250                 255
   His Asn Lys Ile Gln Asn Gly Lys Ala Pro Arg Asp Gly Val Thr Glu
               260                 265                 270
   Asn Cys Val Gln Gly Glu Glu Lys Glu Ser Ser Asn Asp Ser Thr Ser
           275                 280                 285
   Val Ser Ala Val Ala Ser Asn Met Arg Asp Asp Glu Ile Thr Gln Asp
       290                 295                 300
   Glu Asn Thr Val Ser Thr Ser Leu Gly His Ser Arg Asp Asp Asn Ser
   305                 310                 315                 320
   Lys Gln Thr Cys Ile Lys Ile Val Thr Lys Ala Gln Lys Gly Asp Val
                   325                 330                 335
   Cys Thr Pro Thr Ser Thr Thr Val Glu Leu Val Gly Ser Ser Gly Gln
               340                 345                 350
   Asn Gly Asp Glu Lys Gln Asn Ile Val Ala Arg Lys Ile Val Lys Met
           355                 360                 365
   Thr Lys Gln Pro Ala Lys Lys Lys Pro Pro Pro Ser Arg Glu Lys Lys
       370                 375                 380
   Val Thr Arg Thr Ile Leu Ala Ile Leu Leu Ala Phe Ile Ile Thr Trp
   385                 390                 395                 400
   Ala Pro Tyr Asn Val Met Val Leu Ile Asn Thr Phe Cys Ala Pro Cys
                   405                 410                 415
   Ile Pro Asn Thr Val Trp Thr Ile Gly Tyr Trp Leu Cys Tyr Ile Asn
   420 425 430
   Ser Thr Ile Asn Pro Ala Cys Tyr Ala Leu Cys Asn Ala Thr Phe Lys
   435 440 445
   Lys Thr Phe Lys His Leu Leu Met Cys His Tyr Lys Asn Ile Gly Ala
   450 455 460
   Thr Arg
   465
<210> 156
   <211> 10
   <212> PRT
   <213> Artificial sequence
<220>
   <223> Linker
<400> 156
   Gly Ser Gly Ser Gly Ser Gly Ser Gly Ser
   1 5 10
<210> 157
   <211> 20
   <212> PRT
   <213> Artificial sequence
<220>
   <223> Linker
<400> 157
   Gly Ser Gly Ser Gly Ser Gly Ser Gly Ser Gly Ser Gly Ser Gly Ser
   1 5 10 15
   Gly Ser Gly Ser
   20
<210> 158
   <211> 15
   <212> PRT
   <213> Artificial sequence
<220>
   <223> Linker
<400> 158
   Gly Gly Gly Gly Ser Gly Gly Gly Gly Ser Gly Gly Gly Gly Ser
   1 5 10 15
<210> 159
   <211> 17
   <212> PRT
   <213> Lama glama
<400> 159
   Ala His His Ser Glu Asp Pro Ser Ser Lys Ala Pro Lys Ala Pro Met
   1               5                   10                  15
   Ala
<210> 160
   <211> 16
   <212> PRT
   <213> Homo sapiens
<400> 160
   Ser Pro Ser Thr Pro Pro Thr Pro Ser Pro Ser Thr Pro Pro Ala Ser
   1               5                   10                  15
<210> 161
   <211> 4
   <212> PRT
   <213> Artificial sequence
<220>
   <223> Factor Xa cleavage site
<400> 161
   Ile Glu Gly Arg
   1
<210> 162
   <211> 4
   <212> PRT
   <213> Artificial sequence
<220>
   <223> thrombin cleavage site
<400> 162
   Leu Val Pro Arg
   1
<210> 163
   <211> 5
   <212> PRT
   <213> Artificial sequence
<220>
   <223> enterokinase cleaving site
<400> 163
   Asp Asp Asp Asp Lys
   1 5
<210> 164
   <211> 8
   <212> PRT
   <213> Artificial sequence
<220>
   <223> PreScission cleavage site
<400> 164
   Leu Glu Val Leu Phe Gln Gly Pro
   1 5
<210> 165
   <211> 92
   <212> DNA
   <213> Artificial Sequence
<220>
   <223> Primer
<400> 165
   cattttcaat taagatgcag ttacttcgct gtttttcaat attttctgtt attgctagcg 60
   ttttagcaat ggcccaggtg cagctgcagg ag 92
<210> 166
   <211> 81
   <212> DNA
   <213> Artificial Sequence
<220>
   <223> Primer
<400> 166
   ccaccagatc caccaccacc caaggtcttc ttcggagata agcttttgtt cggatcctga 60
   ggagacggtg acctgggtcc c 81

## Description

### FIELD OF THE INVENTION

Many transmembrane receptors such as G protein-coupled receptors (GPCRs) exist in many interconvertible three-dimensional conformations depending on their activity or ligand-binding state. Agents that specifically bind to a transmembrane receptor in a conformationally-specific way can be used to induce a conformational change in the transmembrane receptor. Such agents have therapeutic applications and can be used in X-ray crystallography studies of the transmembrane receptor. Such agents can also be used to improve drug discovery via compound screening and/or structure based drug design.

### BACKGROUND

Muscarinic acetylcholine receptors (M1 - M5) are members of the G protein coupled receptor (GPCR) family that regulate the activity of a diverse array of central and peripheral functions in the human body, including the parasympathetic actions of acetylcholine (Wess et al. 2007). The M2 muscarinic receptor subtype plays a key role in modulating cardiac function and many important central processes such as cognition and pain perception (Wess et al. 2007). As it was among the first GPCRs to be purified (Peterson et al. 1984) and cloned (Kubo et al. 1986), the M2 receptor has long served as a model system in GPCR biology and pharmacology. Muscarinic receptors have attracted particular interest due to their ability to bind small molecule allosteric modulators (Mohr et al. 2003). Since allosteric sites can comprise receptor regions that are less conserved in sequence and structure than the orthosteric binding site, some ligands binding to allosteric sites in muscarinic receptors show substantial subtype selectivity (Digby et al. 2010; Keov et al. 2011). Such agents hold great promise for the development of novel muscarinic drugs for the treatment of various clinical conditions including diseases of the central nervous system and metabolic disorders. Though crystal structures were recently obtained for inactive states of the M2 and M3 muscarinic receptors (Haga et al. 2012; Kruse et al. 2012), experimental data regarding the structural basis for muscarinic receptor activation and allosteric modulation by drug-like molecules has not been reported. Such information could greatly facilitate the development of novel agents with increased potency and selectivity.

The binding of an activating ligand (agonist) to the extracellular side of a GPCR results in conformational changes that enable the receptor to activate heterotrimeric G proteins. Despite the importance of this process, only the β-adrenergic receptor and rhodopsin have been crystallized and their structures solved in agonist-bound active-state conformations (Choe et al. 2011; Rasmussen et al. 2011a; Rasmussen et al. 2011b; Deupi et al. 2012; Scheerer et al. 2008). Crystallization of agonist-bound active-state GPCRs has been extremely challenging due to their inherent conformational flexibility. Fluorescence and NMR experiments have shown that the conformational stabilization of the agonist-bound active-state conformation requires that the receptor must form a complex with an agonist and its G protein, or some other binding protein that stabilizes the active conformation (Yao et al. 2009, Nygaard et al. 2013).

The development of new straightforward tools for structural and pharmacological analysis of GPCR drug targets is therefore needed.

### SUMMARY OF THE INVENTION

In a first aspect, the present invention relates to an active conformation-selective VHH antibody that is directed against and capable of specifically binding to an active conformation of a muscarinic receptor M2 (M2R), wherein the VHH antibody comprises an amino acid sequence that comprises four framework regions (FR1 to FR4) and three complementary determining regions (CDR1 to CDR3) according to the formula: FR1-CDR1-FR2-CDR2-FR3-CDR3-FR4, wherein CDR1 is SEQ ID NO: 31, and CDR2 is SEQ ID NO: 53, and CDR3 is SEQ ID NO: 75; or wherein CDR1 is SEQ ID NO: 32, and CDR2 is SEQ ID NO: 54, and CDR3 is SEQ ID NO: 76; or wherein CDR1 is SEQ ID NO: 41, and CDR2 is SEQ ID NO: 63, and CDR3 is SEQ ID NO: 85. It will be appreciated that M2R can be of any origin, preferably from mammalian origin, in particular from human origin.

The present invention particularly envisages that the active conformation-selective VHH antibody is capable of stabilizing M2R in an active conformation. The above described VHH antibody binds a conformational epitope of the receptor. The VHH antibody binds to an intracellular conformational epitope of the receptor. In one specific embodiment, the above described VHH antibody is a G protein mimetic.

Also envisaged is a polypeptide comprising the above described VHH antibody.

In one embodiment, the above described VHH antibody may also be immobilized on a solid support.

In another aspect, the invention relates to a complex comprising muscarinic receptor M2 (M2R) and an active conformation-selective M2R VHH antibody as described above. The complex may further comprise at least one other conformation-selective receptor ligand. Also, the complex may be crystalline. In a further aspect, the invention also encompasses a non-cellular composition comprising the above described complex. Such a non-cellular composition may be a membrane composition. Another aspect relates to an isolated cellular composition comprising the above described complex.

Further, the present invention relates to a nucleic acid molecule comprising a nucleic acid sequence encoding an amino acid sequence of any of the above described VHH antibody. Also envisaged is a host cell comprising a nucleic acid sequence of the invention.

The above described active conformation-selective compounds targeting muscarinic receptor M2 can be used in a range of applications, including capturing and/or purification of receptor in an active conformation, ligand screening and (structure-based) drug discovery, crystallization studies, but also as therapeutic or diagnostic agents. Other applications and uses will become clear to the skilled person from the further disclosure herein.

### BRIEF DESCRIPTION OF THE FIGURES

The skilled artisan will understand that the drawings, described below, are for illustration purposes only. The drawings are not intended to limit the scope of the present teachings in any way.
**Figure 1****.** Results of selection of M2 Gi mimetic nanobodies from a post-immune llama VHH library.
**Figure 2****.** Summary of sequences of selected M2 Gi mimetics and their effect on an M2 receptor radioligand binding assay. As a non-limiting example, Nb9_8, causes a substantial enhancement of iperoxo affinity in a competition binding assay, similar to the G protein Gᵢ.
**Figure 3****.** Results of selections for functional M2 nanobody ligands from a postimmune llama VHH library using the Gi mimetic Nb9-8.
**Figure 4**. Summary of sequences of selected functional, extracellular M2 nanobody ligands and their effect on M2 receptor in a radioligand binding assay.
**Figure 5.** The overall structure of the active-state Mz receptor (orange) in complex with the orthosteric agonist iperoxo and the active-state stabilizing nanobody Nb9-8 is shown.
**Figure 6.** Data collection and refinement statistics.

### DEFINITIONS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments described herein are capable of operation in other sequences than described or illustrated herein.

Unless otherwise defined herein, scientific and technical terms and phrases used in connection with the present disclosure shall have the meanings that are commonly understood by those of ordinary skill in the art. Generally, nomenclatures used in connection with, and techniques of molecular and cellular biology, structural biology, biophysics, pharmacology, genetics and protein and nucleic acid chemistry described herein are those well-known and commonly used in the art. Singleton, et al., Dictionary of Microbiology and Molecular Biology, 2D ED., John Wiley and Sons, New York (1994), and Hale & Marham, The Harper Collins Dictionary of Biology, Harper Perennial, NY (1991) provide one of skill with general dictionaries of many of the terms used in this disclosure. The methods and techniques described herein are generally performed according to conventional methods well known in the art and as described in various general and more specific references that are cited and discussed throughout the present specification unless otherwise indicated. See, for example, Sambrook et al. Molecular Cloning: A Laboratory Manual, 3th ed., Cold Spring Harbor Laboratory Press, Cold Spring Harbor, N.Y. (2001); Ausubel et al., Current Protocols in Molecular Biology, Greene Publishing Associates (1992, and Supplements to 2002); Rup, Biomolecular crystallography: principles, Practice and Applications to Structural Biology, 1st edition, Garland Science, Taylor & Francis Group, LLC, an informa Business, N.Y. (2009); Limbird, Cell Surface Receptors, 3d ed., Springer (2004).

As used herein, the terms "polypeptide", "protein", "peptide" are used interchangeably herein, and refer to a polymeric form of amino acids of any length, which can include coded and non-coded amino acids, chemically or biochemically modified or derivatized amino acids, and polypeptides having modified peptide backbones. Throughout the application, the standard one letter notation of amino acids will be used. Typically, the term "amino acid" will refer to "proteinogenic amino acid", i.e. those amino acids that are naturally present in proteins. Most particularly, the amino acids are in the L isomeric form, but D amino acids are also envisaged.

As used herein, the terms "nucleic acid molecule", "polynucleotide", "polynucleic acid", "nucleic acid" are used interchangeably and refer to a polymeric form of nucleotides of any length, either deoxyribonucleotides or ribonucleotides, or analogs thereof. Polynucleotides may have any three-dimensional structure, and may perform any function, known or unknown. Non-limiting examples of polynucleotides include a gene, a gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, control regions, isolated RNA of any sequence, nucleic acid probes, and primers. The nucleic acid molecule may be linear or circular.

Any of the peptides, polypeptides, nucleic acids, compound, etc., disclosed herein may be "isolated" or "purified". "Isolated" is used herein to indicate that the material referred to is (i) separated from one or more substances with which it exists in nature (e.g., is separated from at least some cellular material, separated from other polypeptides, separated from its natural sequence context), and/or (ii) is produced by a process that involves the hand of man such as recombinant DNA technology, chemical synthesis, etc.; and/or (iii) has a sequence, structure, or chemical composition not found in nature. "Isolated" is meant to include compounds that are within samples that are substantially enriched for the compound of interest and/or in which the compound of interest is partially or substantially purified. "Purified" as used herein denote that the material referred to is removed from its natural environment and is at least 60% free, at least 75% free, or at least 90% free from other components with which it is naturally associated, also referred to as being "substantially pure".

The term "sequence identity" as used herein refers to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. Determining the percentage of sequence identity can be done manually, or by making use of computer programs that are available in the art. Examples of useful algorithms are PILEUP (Higgins & Sharp, CABIOS 5:151 (1989), BLAST and BLAST 2.0 (Altschul et al. J. Mol. Biol. 215: 403 (1990). Software for performing BLAST analyses is publicly available through the National Center for Biotechnology Information (http://www.ncbi.nlm.nih.gov/).

"Similarity" refers to the percentage number of amino acids that are identical or constitute conservative substitutions. Similarity may be determined using sequence comparison programs such as GAP (Deveraux et al. 1984). In this way, sequences of a similar or substantially different length to those cited herein might be compared by insertion of gaps into the alignment, such gaps being determined, for example, by the comparison algorithm used by GAP. As used herein, "conservative substitution" is the substitution of amino acids with other amino acids whose side chains have similar biochemical properties (e.g. are aliphatic, are aromatic, are positively charged, ...) and is well known to the skilled person. Non-conservative substitution is then the substitution of amino acids with other amino acids whose side chains do not have similar biochemical properties (e.g. replacement of a hydrophobic with a polar residue). Conservative substitutions will typically yield sequences which are not identical anymore, but still highly similar. By conservative substitutions is intended combinations such as gly, ala; val, ile, leu, met; asp, glu; asn, gln; ser, thr; lys, arg; cys, met; and phe, tyr, trp.

A "deletion" is defined here as a change in either amino acid or nucleotide sequence in which one or more amino acid or nucleotide residues, respectively, are absent as compared to an amino acid sequence or nucleotide sequence of a parental polypeptide or nucleic acid. Within the context of a protein, a deletion can involve deletion of about 2, about 5, about 10, up to about 20, up to about 30 or up to about 50 or more amino acids. A protein or a fragment thereof may contain more than one deletion. Within the context of a GPCR, a deletion may also be a loop deletion, or an N- and/or C-terminal deletion. As will be clear to the skilled person, an N- and/or C-terminal deletion of a GPCR is also referred to as a truncation of the amino acid sequence of the GPCR or a truncated GPCR.

An "insertion" or "addition" is that change in an amino acid or nucleotide sequences which has resulted in the addition of one or more amino acid or nucleotide residues, respectively, as compared to an amino acid sequence or nucleotide sequence of a parental protein. "Insertion" generally refers to addition to one or more amino acid residues within an amino acid sequence of a polypeptide, while "addition" can be an insertion or refer to amino acid residues added at an N- or C-terminus, or both termini. Within the context of a protein or a fragment thereof, an insertion or addition is usually of about 1, about 3, about 5, about 10, up to about 20, up to about 30 or up to about 50 or more amino acids. A protein or fragment thereof may contain more than one insertion.

A "substitution", as used herein, results from the replacement of one or more amino acids or nucleotides by different amino acids or nucleotides, respectively as compared to an amino acid sequence or nucleotide sequence of a parental protein or a fragment thereof. It is understood that a protein or a fragment thereof may have conservative amino acid substitutions which have substantially no effect on the protein's activity. By conservative substitutions is intended combinations such as gly, ala; val, ile, leu, met; asp, glu; asn, gin; ser, thr; lys, arg; cys, met; and phe, tyr, trp.

The term "recombinant" when used in reference to a cell, nucleic acid, protein or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express nucleic acids or polypeptides that are not found within the native (non-recombinant) form of the cell or express native genes that are otherwise abnormally expressed, under expressed, over expressed or not expressed at all.

As used herein, the term "expression" refers to the process by which a polypeptide is produced based on the nucleic acid sequence of a gene. The process includes both transcription and translation.

The term "operably linked" as used herein refers to a linkage in which the regulatory sequence is contiguous with the gene of interest to control the gene of interest, as well as regulatory sequences that act in trans or at a distance to control the gene of interest. For example, a DNA sequence is operably linked to a promoter when it is ligated to the promoter downstream with respect to the transcription initiation site of the promoter and allows transcription elongation to proceed through the DNA sequence. A DNA for a signal sequence is operably linked to DNA coding for a polypeptide if it is expressed as a pre-protein that participates in the transport of the polypeptide. Linkage of DNA sequences to regulatory sequences is typically accomplished by ligation at suitable restriction sites or adapters or linkers inserted in lieu thereof using restriction endonucleases known to one of skill in the art.

The term "regulatory sequence" as used herein, and also referred to as "control sequence", refers to polynucleotide sequences which are necessary to affect the expression of coding sequences to which they are operably linked. Regulatory sequences are sequences which control the transcription, post-transcriptional events and translation of nucleic acid sequences. Regulatory sequences include appropriate transcription initiation, termination, promoter and enhancer sequences; efficient RNA processing signals such as splicing and polyadenylation signals; sequences that stabilize cytoplasmic mRMA; sequences that enhance translation efficiency (e.g., ribosome binding sites); sequences that enhance protein stability; and when desired, sequences that enhance protein secretion. The nature of such control sequences differs depending upon the host organism. The term "regulatory sequence" is intended to include, at a minimum, all components whose presence is essential for expression, and can also include additional components whose presence is advantageous, for example, leader sequences and fusion partner sequences.

The term "vector" as used herein is intended to refer to a nucleic acid molecule capable of transporting another nucleic acid molecule to which it has been linked. The vector may be of any suitable type including, but not limited to, a phage, virus, plasmid, phagemid, cosmid, bacmid or even an artificial chromosome. Certain vectors are capable of autonomous replication in a host cell into which they are introduced (e.g., vectors having an origin of replication which functions in the host cell). Other vectors can be integrated into the genome of a host cell upon introduction into the host cell, and are thereby replicated along with the host genome. Moreover, certain preferred vectors are capable of directing the expression of certain genes of interest. Such vectors are referred to herein as "recombinant expression vectors" (or simply, "expression vectors"). Suitable vectors have regulatory sequences, such as promoters, enhancers, terminator sequences, and the like as desired and according to a particular host organism (e.g. bacterial cell, yeast cell). Typically, a recombinant vector as described herein comprises at least one "chimeric gene" or "expression cassette". Expression cassettes are generally DNA constructs preferably including (5' to 3' in the direction of transcription): a promoter region, a polynucleotide sequence, homologue, variant or fragment thereof of the present disclosure operably linked with the transcription initiation region, and a termination sequence including a stop signal for RNA polymerase and a polyadenylation signal. It is understood that all of these regions should be capable of operating in biological cells, such as prokaryotic or eukaryotic cells, to be transformed. The promoter region comprising the transcription initiation region, which preferably includes the RNA polymerase binding site, and the polyadenylation signal may be native to the biological cell to be transformed or may be derived from an alternative source, where the region is functional in the biological cell.

The term "host cell", as used herein, is intended to refer to a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A host cell may be an isolated cell or cell line grown in culture or may be a cell which resides in a living tissue or organism. In particular, host cells are of bacterial or fungal origin, but may also be of plant or mammalian origin. The wordings "host cell", "recombinant host cell", "expression host cell", "expression host system", "expression system", are intended to have the same meaning and are used interchangeably herein.

"G-protein coupled receptors" or "GPCRs" are polypeptides that share a common structural motif, having seven regions of between 22 to 24 hydrophobic amino acids that form seven alpha helices, each of which spans a membrane. Each span is identified by number, i.e., transmembrane-1 (TM1), transmembrane-2 (TM2), etc. The transmembrane helices are joined by regions of amino acids between transmembrane-2 and transmembrane-3, transmembrane-4 and transmembrane-5, and transmembrane-6 and transmembrane-7 on the exterior, or "extracellular" side, of the cell membrane, referred to as "extracellular" regions 1, 2 and 3 (EC1, EC2 and EC3), respectively. The transmembrane helices are also joined by regions of amino acids between transmembrane-1 and transmembrane-2, transmembrane-3 and transmembrane-4, and transmembrane-5 and transmembrane-6 on the interior, or "intracellular" side, of the cell membrane, referred to as "intracellular" regions 1, 2 and 3 (IC1, IC2 and IC3), respectively. The "carboxy" ("C") terminus of the receptor lies in the intracellular space within the cell, and the "amino" ("N") terminus of the receptor lies in the extracellular space outside of the cell. GPCR structure and classification is generally well known in the art, and further discussion of GPCRs may be found in Probst, DNA Cell Biol. 1992 11:1-20; Marchese et al Genomics 23: 609-618, 1994; and the following books: Jürgen Wess (Ed) Structure-Function Analysis of G Protein-Coupled Receptors published by Wiley Liss (1st edition; October 15, 1999); Kevin R. Lynch (Ed) Identification and Expression of G Protein-Coupled Receptors published by John Wiley & Sons (March 1998) and Tatsuya Haga (Ed), G Protein-Coupled Receptors, published by CRC Press (September 24, 1999); and Steve Watson (Ed) G-Protein Linked Receptor Factsbook, published by Academic Press (1st edition; 1994).

The term "biologically active", with respect to a GPCR, refers to a GPCR having a biochemical function (e.g., a binding function, a signal transduction function, or an ability to change conformation as a result of ligand binding) of a naturally occurring GPCR.

In general, the term "naturally-occurring" in reference to a GPCR means a GPCR that is naturally produced (e.g., by a wild type mammal such as a human). Such GPCRs are found in nature. The term "non-naturally occurring" in reference to a GPCR means a GPCR that is not naturally-occurring. Naturally-occurring GPCRs that have been made constitutively active through mutation, and variants of naturally-occurring transmembrane receptors, e.g., epitope-tagged GPCRs and GPCRs lacking their native N-terminus are examples of non-naturally occurring GPCRs. Non-naturally occurring versions of a naturally occurring GPCR are often activated by the same ligand as the naturally-occurring GPCR.

Non-limiting examples of either naturally-occurring or non-naturally occurring GPCRs within the context of the present disclosure are provided further herein, in particular for muscarinic acetylcholine receptors.

An "epitope", as used herein, refers to an antigenic determinant of a polypeptide. An epitope could comprise 3 amino acids in a spatial conformation, which is unique to the epitope. Generally an epitope consists of at least 4, 5, 6, 7 such amino acids, and more usually, consists of at least 8, 9, 10 such amino acids. Methods of determining the spatial conformation of amino acids are known in the art, and include, for example, x-ray crystallography and multi-dimensional nuclear magnetic resonance. A "conformational epitope", as used herein, refers to an epitope comprising amino acids in a spacial conformation that is unique to a folded 3-dimensional conformation of the polypeptide. Generally, a conformational epitope consists of amino acids that are discontinuous in the linear sequence that come together in the folded structure of the protein. However, a conformational epitope may also consist of a linear sequence of amino acids that adopts a conformation that is unique to a folded 3-dimensional conformation of the polypeptide (and not present in a denatured state).

The term "conformation" or "conformational state" of a protein refers generally to the range of structures that a protein may adopt at any instant in time. One of skill in the art will recognize that determinants of conformation or conformational state include a protein's primary structure as reflected in a protein's amino acid sequence (including modified amino acids) and the environment surrounding the protein. The conformation or conformational state of a protein also relates to structural features such as protein secondary structures (e.g., α-helix, β-sheet, among others), tertiary structure (e.g., the three dimensional folding of a polypeptide chain), and quaternary structure (e.g., interactions of a polypeptide chain with other protein subunits). Post-translational and other modifications to a polypeptide chain such as ligand binding, phosphorylation, sulfation, glycosylation, or attachments of hydrophobic groups, among others, can influence the conformation of a protein. Furthermore, environmental factors, such as pH, salt concentration, ionic strength, and osmolality of the surrounding solution, and interaction with other proteins and co-factors, among others, can affect protein conformation. The conformational state of a protein may be determined by either functional assay for activity or binding to another molecule or by means of physical methods such as X-ray crystallography, NMR, or spin labeling, among other methods. For a general discussion of protein conformation and conformational states, one is referred to Cantor and Schimmel, Biophysical Chemistry, Part I: The Conformation of Biological. Macromolecules,.W.H. Freeman and Company, 1980, and Creighton, Proteins: Structures and Molecular Properties, W.H. Freeman and Company, 1993.

A "functional conformation" or a "functional conformational state", as used herein, refers to the fact that proteins possess different conformational states having a dynamic range of activity, in particular ranging from no activity to maximal activity. It should be clear that "a functional conformational state" is meant to cover any conformational state of a protein, having any activity, including no activity, and is not meant to cover the denatured states of proteins. Non-limiting examples of functional conformations include active conformations, inactive conformations or basal conformations (as defined further herein). A particular class of functional conformations is defined as "druggable conformation" and generally refers to a unique therapeutically relevant conformational state of a target protein. As an illustration, the agonist-bound active conformation of the muscarinic acetylcholine receptor M2 corresponds to the druggable conformation of this receptor relating to pain and gliobastoma. It will thus be understood that druggability is confined to particular conformations depending on the therapeutic indication. More details are provided further herein.

As used herein, the terms "active conformation" and "active form" refer to a GPCR, particularly muscarinic acetylcholine receptor M2, that is folded in a way so as to be active. A GPCR can be placed into an active conformation using an agonist of the receptor. For example, a GPCR in its active conformation binds to heterotrimeric G protein and catalyzes nucleotide exchange of the G-protein to activate downstream signaling pathways. Activated GPCRs bind to the inactive, GDP-bound form of heterotrimeric G-proteins and cause the G-proteins to release their GDP so GTP can bind. There is a transient 'nucleotide-free' state that results from this process that enables GTP to bind. Once GTP is bound, the receptor and G-protein dissociate, allowing the GTP-bound G protein to activate downstream signaling pathways such as adenylyl cyclase, ion channels, RAS/MAPK, etc. The terms "inactive conformation" and "inactive form" refer to a GPCR, particularly muscarinic acetylcholine receptor M2, that is folded in a way so as to be inactive. A GPCR can be placed into an inactive conformation using an inverse agonist of the receptor. For example, a GPCR in its inactive conformation does not bind to heterotrimeric G protein with high affinity. The terms "active conformation" and "inactive conformation" will be illustrated further herein. As used herein, the term "basal conformation" refers to a GPCR, particularly muscarinic acetylcholine receptor M2, that is folded in a way that it exhibits activity towards a specific signaling pathway even in the absence of an agonist (also referred to as basal activity or constitutive activity). Inverse agonists can inhibit this basal activity. Thus, a basal conformation of a GPCR corresponds to a stable conformation or prominent structural species in the absence of ligands or accessory proteins.

The term "stabilizing" or "stabilized", with respect to a functional conformational state of a GPCR, as used herein, refers to the retaining or holding of a GPCR protein in a subset of the possible conformations that it could otherwise assume, due to the effects of the interaction of the GPCR with the binding agent described herein. Within this context, a binding agent that selectively binds to a specific conformation or conformational state of a protein refers to a binding agent that binds with a higher affinity to a protein in a subset of conformations or conformational states than to other conformations or conformational states that the protein may assume. One of skill in the art will recognize that binding agents that specifically or selectively bind to a specific conformation or conformational state of a protein will stabilize this specific conformation or conformational state, and its related activity. More details are provided further herein.

The term "affinity", as used herein, refers to the degree to which a ligand (as defined further herein) binds to a target protein so as to shift the equilibrium of target protein and ligand toward the presence of a complex formed by their binding. Thus, for example, where a GPCR and a ligand are combined in relatively equal concentration, a ligand of high affinity will bind to the available antigen on the GPCR so as to shift the equilibrium toward high concentration of the resulting complex. The dissociation constant is commonly used to describe the affinity between a ligand and a target protein. Typically, the dissociation constant is lower than 10-5 M. Preferably, the dissociation constant is lower than 10-6 M, more preferably, lower than 10-7 M. Most preferably, the dissociation constant is lower than 10-8 M. Other ways of describing the affinity between a ligand and its target protein are the association constant (Ka), the inhibition constant (Ki), or indirectly by evaluating the potency of ligands by measuring the half maximal inhibitory concentration (IC50) or half maximal effective concentration (EC50). The ligand may be a binding agent, preferably an immunoglobulin, such as an antibody, or an immunoglobulin fragment, such as a VHH or Nanobody, that binds a conformational epitope on a GPCR. It will be appreciated that within the scope of the present disclosure, the term "affinity" is used in the context of a binding agent, in particular an immunoglobulin or an immunoglobulin fragment, such as a VHH or Nanobody, that binds a conformational epitope of a target GPCR as well as in the context of a test compound (as defined further herein) that binds to a target GPCR, more particularly to an orthosteric or allosteric site of a target GPCR.

The term "specificity", as used herein, refers to the ability of a binding agent, in particular an immunoglobulin or an immunoglobulin fragment, such as a VHH or Nanobody, to bind preferentially to one antigen, versus a different antigen, and does not necessarily imply high affinity.

The terms "specifically bind" and "specific binding", as used herein, generally refers to the ability of a binding agent, in particular an immunoglobulin, such as an antibody, or an immunoglobulin fragment, such as a VHH or Nanobody, to preferentially bind to a particular antigen that is present in a homogeneous mixture of different antigens. In certain embodiments, a specific binding interaction will discriminate between desirable and undesirable antigens in a sample, in some embodiments more than about 10 to 100-fold or more (e.g., more than about 1000- or 10,000-fold). Within the context of the spectrum of conformational states of GPCRs, in particular muscarinic acetylcholine receptor M2, the terms particularly refer to the ability of a binding agent (as defined herein) to preferentially recognize and/or bind to a particular conformational state of a GPCR as compared to another conformational state.

As used herein, the term "conformation-selective binding agent" in the context of the present disclosure refers to a binding agent that binds to a target protein in a conformation-selective manner. A binding agent that selectively binds to a particular conformation or conformational state of a protein refers to a binding agent that binds with a higher affinity to a protein in a subset of conformations or conformational states than to other conformations or conformational states that the protein may assume. One of skill in the art will recognize that binding agents that selectively bind to a specific conformation or conformational state of a protein will stabilize or retain the protein it this particular conformation or conformational state. For example, an active conformation-selective binding agent will preferentially bind to a GPCR in an active conformational state and will not or to a lesser degree bind to a GPCR in an inactive conformational state, and will thus have a higher affinity for said active conformational state; or vice versa. The terms "specifically bind", "selectively bind", "preferentially bind", and grammatical equivalents thereof, are used interchangeably herein. The terms "conformational specific" or "conformational selective" are also used interchangeably herein.

The term "compound" or "test compound" or "candidate compound" or "drug candidate compound" as used herein describes any molecule, either naturally occurring or synthetic that is tested in an assay, such as a screening assay or drug discovery assay. As such, these compounds comprise organic or inorganic compounds. The compounds include polynucleotides, lipids or hormone analogs that are characterized by low molecular weights. Other biopolymeric organic test compounds include small peptides or peptide-like molecules (peptidomimetics) comprising from about 2 to about 40 amino acids and larger polypeptides comprising from about 40 to about 500 amino acids, such as antibodies, antibody fragments or antibody conjugates. Test compounds can also be protein scaffolds. For high-throughput purposes, test compound libraries may be used, such as combinatorial or randomized libraries that provide a sufficient range of diversity. Examples include, but are not limited to, natural compound libraries, allosteric compound libraries, peptide libraries, antibody fragment libraries, synthetic compound libraries, fragment-based libraries, phage-display libraries, and the like. A more detailed description can be found further in the specification.

As used herein, the term "ligand" means a molecule that specifically binds to a GPCR, in particular muscarinic acetylcholine receptor M2. A ligand may be, without the purpose of being limitative, a polypeptide, a lipid, a small molecule, an antibody, an antibody fragment, a nucleic acid, a carbohydrate. A ligand may be synthetic or naturally occurring. A ligand also includes a "native ligand" which is a ligand that is an endogenous, natural ligand for a native GPCR. Within the context of the present disclosure, a ligand may bind to a GPCR, either intracellularly or extracellularly. A ligand may be an agonist, a partial agonist, an inverse agonist, an antagonist, an allosteric modulator, and may bind at either the orthosteric site or at an allosteric site. A ligand may be a "conformation-selective ligand" or "conformation-specific ligand", meaning that such a ligand binds the GPCR in a conformation-selective manner. A conformation-selective ligand binds with a higher affinity to a particular conformation of the GPCR than to other conformations the GPCR may adopt. For the purpose of illustration, an agonist is an example of an active conformation-selective ligand, whereas an inverse agonist is an example of an inactive conformation-selective ligand. For the sake of clarity, a neutral antagonist is not considered as a conformation-selective ligand, since a neutral antagonist does not distinguish between the different conformations of a GPCR.

An "orthosteric ligand", as used herein, refers to a ligand (both natural and synthetic), that binds to the active site of a GPCR, in particular muscarinic acetylcholine receptor M2, and are further classified according to their efficacy or in other words to the effect they have on signaling through a specific pathway. As used herein, an "agonist" refers to a ligand that, by binding a receptor protein, increases the receptor's signaling activity. Full agonists are capable of maximal protein stimulation; partial agonists are unable to elicit full activity even at saturating concentrations. Partial agonists can also function as "blockers" by preventing the binding of more robust agonists. An "antagonist", also referred to as a "neutral antagonist", refers to a ligand that binds a receptor without stimulating any activity. An "antagonist" is also known as a "blocker" because of its ability to prevent binding of other ligands and, therefore, block agonist-induced activity. Further, an "inverse agonist" refers to an antagonist that, in addition to blocking agonist effects, reduces a receptor's basal or constitutive activity below that of the unliganded protein.

Ligands as used herein may also be "biased ligands" with the ability to selectively stimulate a subset of a receptor's signaling activities, for example in the case of GPCRs the selective activation of G-protein or β-arrestin function. Such ligands are known as "biased ligands", "biased agonists" or "functionally selective agonists". More particularly, ligand bias can be an imperfect bias characterized by a ligand stimulation of multiple receptor activities with different relative efficacies for different signals (non-absolute selectivity) or can be a perfect bias characterized by a ligand stimulation of one receptor protein activity without any stimulation of another known receptor protein activity.

Another kind of ligands is known as allosteric regulators. "Allosteric regulators" or otherwise "allosteric modulators", "allosteric ligands" or "effector molecules", as used herein, refer to ligands that bind at an allosteric site (that is, a regulatory site physically distinct from the protein's active site) of a GPCR, in particular muscarinic acetylcholine receptor M2. In contrast to orthosteric ligands, allosteric modulators are non-competitive because they bind receptor proteins at a different site and modify their function even if the endogenous ligand also is binding. Allosteric regulators that enhance the protein's activity are referred to herein as "allosteric activators" or "positive allosteric modulators" (PAMs), whereas those that decrease the protein's activity are referred to herein as "allosteric inhibitors" or otherwise "negative allosteric modulators" (NAMs).

As used herein, the terms "determining", "measuring", "assessing", "assaying" are used interchangeably and include both quantitative and qualitative determinations.

The term "antibody" is intended to mean an immunoglobulin or any fragment thereof that is capable of antigen binding. The term "antibody" also refers to single chain antibodies and antibodies with only one binding domain.

As used herein, the terms "complementarity determining region" or "CDR" within the context of antibodies refer to variable regions of either H (heavy) or L (light) chains (also abbreviated as VH and VL, respectively) and contains the amino acid sequences capable of specifically binding to antigenic targets. These CDR regions account for the basic specificity of the antibody for a particular antigenic determinant structure. Such regions are also referred to as "hypervariable regions." The CDRs represent non-contiguous stretches of amino acids within the variable regions but, regardless of species, the positional locations of these critical amino acid sequences within the variable heavy and light chain regions have been found to have similar locations within the amino acid sequences of the variable chains. The variable heavy and light chains of all canonical antibodies each have 3 CDR regions, each non- contiguous with the others (termed L1, L2, L3, H1, H2, H3) for the respective light (L) and heavy (H) chains. Immunoglobulin single variable domains, in particular Nanobodies, generally comprise a single amino acid chain that can be considered to comprise 4 "framework sequences or regions" or FRs and 3 "complementary determining regions" or CDRs. The nanobodies have 3 CDR regions, each non-contiguous with the others (termed CDR1, CDR2, CDR3). The delineation of the FR and CDR sequences can, for example, be based on the IMGT unique numbering system for V-domains and V-like domains (Lefranc et al. 2003).

### DETAILED DESCRIPTION

### Conformation-selective binding agents against muscarinic acetylcholine receptor and complexes comprising the same

Disclosed herein is a conformation-selective binding agent that is directed against and/or capable of specifically binding to a GPCR of the muscarinic acetylcholine receptor family (mAChRs).

The muscarinic acetylcholine receptors (mAChRs) belong to the superfamily of GPCRs (as defined herein), more particularly to the family A GPCRs, and include five subtypes, designated M1 to M5. Classically these receptors are sub-divided into two broad groups based on their primary coupling efficiency to G-proteins. The M2 and M4-muscarinic receptors are able to couple to Gi/o-proteins, whereas the M1, M3 and M5-muscarinic receptors couple to Gq/11-proteins and activate phospholipase C. The neurotransmitter acetylcholine (ACh) is a natural agonist for this family of receptors. The amino acid sequences (and the nucleotide sequences of the cDNAs which encode them) of the muscarinic acetylcholine receptors are readily available, for example by reference to GenBank (http://www.ncbi.nlm.nih.gov/entrez). HGNC standardized nomenclature to human genes, accession numbers of different isoforms from different organisms are available from Uniprot (www.uniprot.org). Moreover, a comprehensive overview of receptor nomenclature, pharmacological, functional and pathophysiological information on muscarinic acetylcholine receptors can be retrieved from the IUPHAR database (http://www.iuphar-db.org/). The terms "muscarinic acetylcholine receptor" and "muscarinic receptor" are used interchangeably herein.

Preferably, the conformation-selective binding agent described herein is directed against and/or specifically binds to a muscarinic acetylcholine receptor M2 (M2R). The nature of the muscarinic acetylcholine receptor, in particular muscarinic receptor M2, is not critical and can be from any organism including a fungus (including yeast), nematode, virus, insect, plant, bird (e.g. chicken, turkey), reptile or mammal (e.g., a mouse, rat, rabbit, hamster, gerbil, dog, cat, goat, pig, cow, horse, whale, monkey, camelid, or human). Preferably, the muscarinic acetylcholine receptor is of mammalian origin, even more preferably of human origin.

The conformation-selective binding agent described herein may specifically bind to human muscarinic acetylcholine receptor M2 (SEQ ID NO: 153), and/or mouse muscarinic acetylcholine receptor M2 (SEQ ID NO: 154), and/or rat muscarinic acetylcholine receptor M2 (SEQ ID NO: 155). Preferably, the conformation-selective binding agent binds to human muscarinic acetylcholine receptor M2 (SEQ ID NO: 153).

The conformation-selective binding agent described herein may not be directed against and/or not specifically bind to muscarinic acetylcholine receptor M3 (e.g. human muscarinic receptor M3; Uniprot identifier P20309). The conformation-selective binding agent described herein may not be directed against and/or not specifically bind to muscarinic acetylcholine receptor M4 (e.g. human muscarinic receptor M4; Uniprot identifier P08173). The conformation-selective binding agent described herein mat not be directed against and/or not specifically bind to muscarinic acetylcholine receptor M5 (e.g. human muscarinic receptor M5; Uniprot identifier P08912).The conformation-selective binding agent described herein may not be directed against and/or not specifically bind to muscarinic acetylcholine receptor M1 (e.g. human muscarinic receptor M1; Uniprot identifier P11229).

A prerequisite of the binding agent is its capability to specifically bind (as defined herein) to the muscarinic acetylcholine receptor, preferably muscarinic receptor M2. Thus, the binding agent may be directed against any conformational epitope (as defined herein) of the muscarinic receptor. A binding agent that specifically binds to a "conformational epitope" specifically binds to a tertiary (i.e., three dimensional) structure of a folded protein, and binds at much reduced (i.e., by a factor of at least 2, 5, 10, 50 or 100) affinity to the linear (i.e., unfolded, denatured) form of the protein. In particular, said conformational epitope can be part of an intracellular or extracellular region, or an intramembraneous region, or a domain or loop structure of the muscarinic receptor. Thus, the binding agent may be directed against an extracellular region, domain, loop or other extracellular conformational epitope of the muscarinic receptor, but is preferably directed against extracellular parts of the transmembrane domains or against extracellular loops that link the transmembrane domains. Alternatively, the binding agent may be directed against an intracellular region, domain, loop or other intracellular conformational epitope of the muscarinic receptor, but is preferably directed against intracellular parts of the transmembrane domains or against intracellular loops that link the transmembrane domains. The binding agent may also be directed against a conformational epitope that forms part of the binding site of a natural ligand, including but limited to an endogenous orthosteric agonist. The binding agent may also be directed against a conformational epitope, in particular an intracellular conformational epitope, that is comprised in a binding site for a downstream signaling protein, including but not-limited to a G protein binding site or a β-arrestin binding site. For example, the binding agent may bind to an intracellular conformational epitope of muscarinic receptor M2, the conformational epitope comprising at least one of the following amino acid residues: T56, N58, R121, C124, V125, P132, V133, R135, Y206, 1209, S213, S380, V385, T388, 1389, R381, C439, Y440, C443, A445, T446, whereby the amino acid numbering is as defined in the human muscarinic receptor M2R (SEQ ID NO: 153). Note that these residues are conserved in other species, including mouse M2R (SEQ ID NO: 154) and rat M2R (SEQ ID NO: 155), as can be readily derived from an alignment of these sequences, which is routine practice by persons skilled in the art.

It will be understood that the conformation-selective binding agent is capable of stabilizing the muscarinic receptor in a particular conformation. With the term "stabilizing", or grammatically equivalent terms, as defined hereinbefore, is meant an increased stability of a muscarinic receptor with respect to the structure (e.g. conformational state) and/or particular biological activity (e.g. intracellular signaling activity, ligand binding affinity, ...). In relation to increased stability with respect to structure and/or biological activity, this may be readily determined by either a functional assay for activity (e.g. Ca2+ release, cAMP generation or transcriptional activity, β-arrestin recruitment, ...) or ligand binding or by means of physical methods such as X-ray crystallography, NMR, or spin labeling, among other methods. The term "stabilize" also includes increased thermostability of the receptor under non-physiological conditions induced by denaturants or denaturing conditions. The term "thermostabilize", "thermostabilizing", "increasing the thermostability of", as used herein, refers to the functional rather than to the thermodynamic properties of a receptor and to the protein's resistance to irreversible denaturation induced by thermal and/or chemical approaches including but not limited to heating, cooling, freezing, chemical denaturants, pH, detergents, salts, additives, proteases or temperature. Irreversible denaturation leads to the irreversible unfolding of the functional conformations of the protein, loss of biological activity and aggregation of the denaturated protein. In relation to an increased stability to heat, this can be readily determined by measuring ligand binding or by using spectroscopic methods such as fluorescence, CD or light scattering that are sensitive to unfolding at increasing temperatures. It is preferred that the binding agent is capable of increasing the stability as measured by an increase in the thermal stability of a muscarinic receptor in a functional conformational state with at least 2°C, at least 5°C, at least 8°C, and more preferably at least 10°C or 15°C or 20°C. In relation to an increased stability to a detergent or to a chaotrope, typically the muscarinic receptor is incubated for a defined time in the presence of a test detergent or a test chaotropic agent and the stability is determined using, for example, ligand binding or a spectroscoptic method, optionally at increasing temperatures as discussed above. Otherwise, the binding agent is capable of increasing the stability to extreme pH of a functional conformational state of a muscarinic receptor. In relation to an extreme of pH, a typical test pH would be chosen for example in the range 6 to 8, the range 5.5 to 8.5, the range 5 to 9, the range 4.5 to 9.5, more specifically in the range 4.5 to 5.5 (low pH) or in the range 8.5 to 9.5 (high pH). The term "(thermo)stabilize", "(thermo)stabilizing", "increasing the (thermo)stability of", as used herein, applies to muscarinic receptors embedded in lipid particles or lipid layers (for example, lipid monolayers, lipid bilayers, and the like) and to muscarinic receptors that have been solubilized in detergent.

It is thus particularly envisaged that the conformation-selective binding agent described herein stabilizes the muscarinic receptor in a functional conformation upon binding of the binding agent. Preferably, the muscarinic receptor, more specifically muscarinic receptor M2, is stabilized in an active conformation upon binding of a binding agent that is conformation-selective for an active conformation. The term "active conformation", as used herein, refers to a spectrum of receptor conformations that allows signal transduction towards an intracellular effector system, such as G protein dependent signaling and/or G protein-independent signaling (e.g. β-arrestin signaling). An "active conformation" thus encompasses a range of ligand-specific conformations, including an agonist-specific active state conformation, a partial agonist-specific active state conformation or a biased agonist-specific active state conformation, so that it induces the cooperative binding of an intracellular effector protein. Preferably, the muscarinic receptor, more specifically muscarinic M2 receptor, is stabilized in an active conformation upon binding of an active conformation-selective binding agent, whereby the receptor is folded in a way that it is active by inducing G protein dependent signaling. Alternatively, the muscarinic receptor, more specifically muscarinic receptor M2, is stabilized in an inactive conformation upon binding of a binding agent that is conformation-selective for an inactive conformation. The term "inactive conformation", as used herein, refers to a spectrum of receptor conformations that does not allow or blocks signal transduction towards an intracellular effector system. An "inactive conformation" thus encompasses a range of ligand-specific conformations, including an inverse agonist-specific inactive state conformation, so that it prevents the cooperative binding of an intracellular effector protein. It will be understood that the site of binding of the ligand is not critical for obtaining an active or inactive conformation. Hence, orthosteric ligands as well as allosteric modulators may equally be capable of stabilizing a muscarinic receptor in an active or inactive conformation. For example, the binding agent that is capable of stabilizing the muscarinic receptor may bind at the orthosteric site or an allosteric site. IThe binding agent that is capable of stabilizing the muscarinic receptor may be an active conformation-selective binding agent, or an inactive conformation-selective binding agent, either by binding at the orthosteric site or at an allosteric site.

Generally, a conformation-selective binding agent that stabilizes an active conformation of a muscarinic receptor, more specifically muscarinic receptor M2, will increase or enhance the affinity of the receptor for an active conformation-selective ligand, such as an agonist, more specifically a full agonist, a partial agonist or a biased agonist, as compared to the receptor in the absence of the binding agent (or in the presence of a mock binding agent - also referred to as control binding agent or irrelevant binding agent - that is not directed against and/or does not specifically bind to the muscarinic receptor M2). Also, a binding agent that stabilizes an active conformation of a muscarinic receptor will decrease the affinity of the receptor for an inactive conformation-selective ligand, such as an inverse agonist, as compared to the receptor in the absence of the binding agent (or in the presence of a mock binding agent). In contrast, a binding agent that stabilizes an inactive conformation of a muscarinic receptor will enhance the affinity of the receptor for an inverse agonist and will decrease the affinity of the receptor for an agonist, particularly for a full agonist, a partial agonist or a biased agonist, as compared to the receptor in the absence of the binding agent (or in the presence of a mock binding agent). An increase or decrease in affinity for a ligand may be directly measured by and/or calculated from a decrease or increase, respectively in EC₅₀, IC₅₀, K_{d}, Kᵢ or any other measure of affinity or potency known to one of skill in the art. It is particularly preferred that the binding agent that stabilizes a particular conformation of a muscarinic receptor is capable of increasing or decreasing the affinity for a conformation-selective ligand at least 2 fold, at least 5 fold, at least 10 fold, at least 50 fold, and more preferably at least 100 fold, even more preferably at least 1000 fold or more, upon binding to the receptor. It will be appreciated that affinity measurements for conformation-selective ligands that trigger/inhibit particular signaling pathways may be carried out with any type of ligand, including natural ligands, small molecules, as well as biologicals; with orthosteric ligands as well as allosteric modulators; with single compounds as well as compound libraries; with lead compounds or fragments; etc.

A particularly preferred conformation-selective binding agent that is directed against and/or specifically binding to a muscarinic receptor, more specifically M2R, as described herein is a G protein mimetic. The term "G protein mimetic", as used herein, refers to a binding agent that, upon binding to a muscarinic receptor, enhances the affinity of the receptor for orthosteric or allosteric agonists, to a similar extend as upon binding of the natural G protein to the muscarinic receptor. Preferably, a binding agent that is a G protein mimetic will occupy the G protein binding site of a muscarinic receptor.

It will also be understood that the muscarinic acetylcholine receptor, more specifically M2R, to which the conformation-selective binding agents described herein will bind, can be a naturally occurring or non-naturally occurring (i.e., altered by man) receptor (as defined herein). In particular, wild type polymorphic variants and isoforms of the muscarinic acetylcholine receptor, as well as orthologs across different species are examples of naturally occurring proteins, and are found for example, and without limitation, in a mammal, more specifically in a human, or in a virus, or in a plant, or in an insect, amongst others). Such receptors are found in nature. For example, a "human muscarinic acetylcholine receptor M2" has an amino acid sequence that is at least 95% identical to (e.g., at least 95% or at least 98% identical to) the naturally occurring "human muscarinic acetylcholine receptor M2" of Genbank accession number AAA51570.1. Wild-type muscarinic acetylcholine receptors that have been mutated and other variants of naturally-occurring muscarinic acetylcholine receptors are examples of non-naturally occurring proteins. Non-limiting examples of non-naturally occurring muscarinic acetylcholine receptors include, without limitation, muscarinic acetylcholine receptors that have been made constitutively active through mutation, muscarinic acetylcholine receptors with a loop deletion, muscarinic acetylcholine receptors with an N- and/or C-terminal deletion, muscarinic acetylcholine receptors with a substitution, an insertion or addition, or any combination thereof, in relation to their amino acid or nucleotide sequence, or other variants of naturally-occurring muscarinic acetylcholine receptors. Also comprised within the scope of the present disclosure are muscarinic acetylcholine receptors comprising a chimeric or hybrid structure, for example a chimeric muscarinic acetylcholine receptor with an N- and/or C-terminus from one muscarinic acetylcholine receptor and loops of a second muscarinic acetylcholine receptor, or comprising a muscarinic acetylcholine receptor fused to a moiety, such as T4 lysozyme, Flavodoxin, Xylanase, Rubredoxin or cytochrome b as an utility in GPCR crystallization (Chun et al. 2012 and also described in patent applications WO2012/158555, WO2012/030735, WO2012/148586). According to specific examples within the scope of the present disclosure, a non-naturally occurring muscarinic acetylcholine receptor, in particular M2R, may have an amino acid sequence that is at least 80% identical to, at least 90% identical to, at least 95% identical to or at least 99% identical to, a corresponding naturally-occurring muscarinic acetylcholine receptor.

Thus, the conformation-selective binding agent is preferably capable of recognizing both a naturally-occurring as well as a non-naturally occurring muscarinic acetylcholine receptor, in particular M2R. This may be particularly advantageous in certain circumstances, and depending on the purpose or application. For example, and for illustration purposes only, to increase the probability of obtaining crystals of the muscarinic acetylcholine receptor stabilized in a particular conformation enabled by the conformation-selective binding agents described herein, it might be desired to perform some protein engineering without or only minimally affecting the conformation (e.g. active conformation with increased affinity for agonists). Or, alternatively or additionally, to increase cellular expression levels of a muscarinic acetylcholine receptor, or to increase the stability, one might also consider to introduce certain mutations in the receptor of interest.

The term "binding agent", as used herein, means the whole or part of a proteinaceous (protein, protein-like or protein containing) molecule that is capable of binding using specific intermolecular interactions to a muscarinic acetylcholine receptor, more specifically M2R. In particular, the term "binding agent" is not meant to include a naturally-occurring binding partner of the muscarinic acetylcholine receptor, such as a G protein, an arrestin, an endogenous ligand; or variants or derivatives (including fragments) thereof. More specifically, the term "binding agent" refers to a polypeptide, more particularly a protein domain. A suitable protein domain is an element of overall protein structure that is self-stabilizing and that folds independently of the rest of the protein chain and is often referred to as "binding domain". Such binding domains vary in length from between about 25 amino acids up to 500 amino acids and more. Many binding domains can be classified into folds and are recognizable, identifiable, 3-D structures. Some folds are so common in many different proteins that they are given special names. Non-limiting examples are binding domains selected from a 3- or 4-helix bundle, an armadillo repeat domain, a leucine-rich repeat domain, a PDZ domain, a SUMO or SUMO-like domain, a cadherin domain, an immunoglobulin-like domain, phosphotyrosine-binding domain, pleckstrin homology domain, src homology 2 domain, amongst others. A binding domain can thus be derived from a naturally occurring molecule, e.g. from components of the innate or adaptive immune system, or it can be entirely artificially designed.

In general, a binding domain can be immunoglobulin-based or it can be based on domains present in proteins, including but limited to microbial proteins, protease inhibitors, toxins, fibronectin, lipocalins, single chain antiparallel coiled coil proteins or repeat motif proteins. Particular examples of binding domains which are known in the art include, but are not limited to: antibodies, heavy chain antibodies (hcAb), single domain antibodies (sdAb), minibodies, the variable domain derived from camelid heavy chain antibodies (VHH or nanobodies), the variable domain of the new antigen receptors derived from shark antibodies (VNAR), alphabodies, protein A, protein G, designed ankyrin-repeat domains (DARPins), fibronectin type III repeats, anticalins, knottins, engineered CH2 domains (nanoantibodies), engineered SH3 domains, affibodies, peptides and proteins, lipopeptides (e.g. pepducins) (see, e.g., Gebauer & Skerra, 2009; Skerra, 2000; Starovasnik et al., 1997; Binz et al., 2004; Koide et al., 1998; Dimitrov, 2009; Nygren et al. 2008; WO2010066740). Frequently, when generating a particular type of binding domain using selection methods, combinatorial libraries comprising a consensus or framework sequence containing randomized potential interaction residues are used to screen for binding to a molecule of interest, such as a protein.

It is particularly envisaged that the binding agent described herein is derived from an innate or adaptive immune system. Preferably, said binding agent is derived from an immunoglobulin. Preferably, the binding agent described herein is derived from an antibody or an antibody fragment. The term "antibody" (Ab) refers generally to a polypeptide encoded by an immunoglobulin gene, or a functional fragment thereof, that specifically binds and recognizes an antigen, and is known to the person skilled in the art. An antibody is meant to include a conventional four-chain immunoglobulin, comprising two identical pairs of polypeptide chains, each pair having one "light" (about 25 kDa) and one "heavy" chain (about 50 kDa). Typically, in conventional immunoglobulins, a heavy chain variable domain (VH) and a light chain variable domain (VL) interact to form an antigen binding site. The term "antibody" is meant to include whole antibodies, including single-chain whole antibodies, and antigen-binding fragments. Antigen-binding fragments may be antigen-binding antibody fragments that include, but are not limited to, Fab, Fab' and F(ab')2, Fd, single-chain Fvs (scFv), single-chain antibodies, disulfide-linked Fvs (dsFv) and fragments comprising or consisting of either a VL or VH domain, and any combination of those or any other functional portion of an immunoglobulin peptide capable of binding to the target antigen. The term "antibodies" is also meant to include heavy chain antibodies, or fragments thereof, including immunoglobulin single variable domains, as defined further herein.

The term "immunoglobulin single variable domain" defines molecules wherein the antigen binding site is present on, and formed by, a single immunoglobulin domain (which is different from conventional immunoglobulins or their fragments, wherein typically two immunoglobulin variable domains interact to form an antigen binding site). It should however be clear that the term "immunoglobulin single variable domain" does comprise fragments of conventional immunoglobulins wherein the antigen binding site is formed by a single variable domain. Preferably, the binding agent within the scope of the present disclosure is an immunoglobulin single variable domain.

Generally, an immunoglobulin single variable domain will be an amino acid sequence comprising 4 framework regions (FR1 to FR4) and 3 complementary determining regions (CDR1 to CDR3), preferably according to the following formula (1): FR1-CDR1-FR2-CDR2-FR3-CDR3-FR4 (1), or any suitable fragment thereof (which will then usually contain at least some of the amino acid residues that form at least one of the complementarity determining regions). Immunoglobulin single variable domains comprising 4 FRs and 3 CDRs are known to the person skilled in the art and have been described, as a non-limiting example, in Wesolowski et al. 2009. Typical, but non-limiting, examples of immunoglobulin single variable domains include light chain variable domain sequences (e.g. a VL domain sequence) or a suitable fragment thereof, or heavy chain variable domain sequences (e.g. a VH domain sequence or VHH domain sequence) or a suitable fragment thereof, as long as it is capable of forming a single antigen binding unit. Thus, the binding agent is preferably an immunoglobulin single variable domain that is a light chain variable domain sequence (e.g. a VL domain sequence) or a heavy chain variable domain sequence (e.g. a VH domain sequence); more specifically, the immunoglobulin single variable domain is a heavy chain variable domain sequence that is derived from a conventional four-chain antibody or a heavy chain variable domain sequence that is derived from a heavy chain antibody. The immunoglobulin single variable domain may be a domain antibody, or a single domain antibody, or a "dAB" or dAb, or a Nanobody (as defined herein), or another immunoglobulin single variable domain, or any suitable fragment of any one thereof. For a general description of single domain antibodies, reference is made to the following book: "Single domain antibodies", Methods in Molecular Biology, Eds. Saerens and Muyldermans, 2012, Vol 911. The immunoglobulin single variable domains, generally comprise a single amino acid chain that can be considered to comprise 4 "framework sequences" or FR's and 3 "complementary determining regions" or CDR's (as defined hereinbefore). It should be clear that framework regions of immunoglobulin single variable domains may also contribute to the binding of their antigens (Desmyter et al 2002; Korotkov et al. 2009). The delineation of the CDR sequences (and thus also the FR sequences) can be based on the IMGT unique numbering system for V-domains and V-like domains (Lefranc et al. 2003). Alternatively, the delineation of the FR and CDR sequences can be done by using the Kabat numbering system as applied to VHH domains from Camelids in the article of Riechmann and Muyldermans (2000).

It should be noted that the immunoglobulin single variable domains as binding agent in their broadest sense are not limited to a specific biological source or to a specific method of preparation. The term "immunoglobulin single variable domain" encompasses variable domains of different origin, comprising mouse, rat, rabbit, donkey, human, shark, camelid variable domains. For example, the immunoglobulin single variable domains may be derived from shark antibodies (the so-called immunoglobulin new antigen receptors or IgNARs), more specifically from naturally occurring heavy chain shark antibodies, devoid of light chains, and are known as VNAR domain sequences. Preferably, the immunoglobulin single variable domains are derived from camelid antibodies. More preferably, the immunoglobulin single variable domains are derived from naturally occurring heavy chain camelid antibodies, devoid of light chains, and are known as VHH domain sequences or Nanobodies.

Preferably, the binding agent described herein is an immunoglobulin single variable domain that is a Nanobody (as defined further herein, and including but not limited to a VHH). The term "Nanobody" (Nb), as used herein, is a single domain antigen binding fragment. It particularly refers to a single variable domain derived from naturally occurring heavy chain antibodies and is known to the person skilled in the art. Nanobodies are usually derived from heavy chain only antibodies (devoid of light chains) seen in camelids (Hamers-Casterman et al. 1993; Desmyter et al. 1996) and consequently are often referred to as VHH antibody or VHH sequence. Camelids comprise old world camelids (*Camelus bactrianus* and *Camelus dromedarius*) and new world camelids (for example *Lama paccos, Lama glama, Lama guanicoe* and *Lama vicugna*). Nanobody® and Nanobodies® are registered trademarks of Ablynx NV (Belgium). For a further description of VHH's or Nanobodies, reference is made to the book "Single domain antibodies", Methods in Molecular Biology, Eds. Saerens and Muyldermans, 2012, Vol 911, in particular to the Chapter by Vincke and Muyldermans (2012), as well as to a non-limiting list of patent applications, which are mentioned as general background art, and include: WO 94/04678, WO 95/04079, WO 96/34103 of the Vrije Universiteit Brussel; WO 94/25591, WO 99/37681, WO 00/40968, WO 00/43507, WO 00/65057, WO 01/40310, WO 01/44301, EP 1 134 231 and WO 02/48193 of Unilever; WO 97/49805, WO 01/21817, WO 03/035694, WO 03/054016 and WO 03/055527 of the Vlaams Instituut voor Biotechnologie (VIB); WO 04/041867, WO 04/041862, WO 04/041865, WO 04/041863, WO 04/062551, WO 05/044858, WO 06/40153, WO 06/079372, WO 06/122786, WO 06/122787 and WO 06/122825, by Ablynx N.V. and the further published patent applications by Ablynx N .V. As will be known by the person skilled in the art, the Nanobodies are particularly characterized by the presence of one or more Camelidae "hallmark residues" in one or more of the framework sequences (according to Kabat numbering), as described for example in WO 08/020079, on page 75, Table A-3, incorporated herein by reference). It should be noted that the Nanobodies, disclosed herein in their broadest sense are not limited to a specific biological source or to a specific method of preparation. For example, Nanobodies, can generally be obtained: (1) by isolating the VHH domain of a naturally occurring heavy chain antibody; (2) by expression of a nucleotide sequence encoding a naturally occurring VHH domain; (3) by "humanization" of a naturally occurring VHH domain or by expression of a nucleic acid encoding a such humanized VHH domain; (4) by "camelization" of a naturally occurring VH domain from any animal species, and in particular from a mammalian species, such as from a human being, or by expression of a nucleic acid encoding such a camelized VH domain; (5) by "camelisation" of a "domain antibody" or "Dab" as described in the art, or by expression of a nucleic acid encoding such a camelized VH domain; (6) by using synthetic or semi-synthetic techniques for preparing proteins, polypeptides or other amino acid sequences known per se; (7) by preparing a nucleic acid encoding a Nanobody using techniques for nucleic acid synthesis known per se, followed by expression of the nucleic acid thus obtained; and/or (8) by any combination of one or more of the foregoing. A further description of Nanobodies, including humanization and/or camelization of Nanobodies, can be found e.g. in WO08/101985 and WO08/142164, as well as further herein. A particular class of Nanobodies binding conformational epitopes of native targets is called Xaperones and is particularly envisaged here. Xaperone™ is a trademark of VIB and VUB (Belgium). A Xaperone™ is a camelid single domain antibody that constrains drug targets into a unique, disease relevant druggable conformation.

Within the scope of the present disclosure, the term "immunoglobulin single variable domain" also encompasses variable domains that are "humanized" or "camelized", in particular Nanobodies that are "humanized" or "camelized". For example both "humanization" and "camelization" can be performed by providing a nucleotide sequence that encodes a naturally occurring VHH domain or VH domain, respectively, and then changing, in a manner known per se, one or more codons in said nucleotide sequence in such a way that the new nucleotide sequence encodes a "humanized" or "camelized" immunoglobulin single variable domains, respectively. This nucleic acid can then be expressed in a manner known per se, so as to provide the desired immunoglobulin single variable domains. Alternatively, based on the amino acid sequence of a naturally occurring VHH domain or VH domain, respectively, the amino acid sequence of the desired humanized or camelized immunoglobulin single variable domains, respectively, can be designed and then synthesized de novo using techniques for peptide synthesis known per se. Also, based on the amino acid sequence or nucleotide sequence of a naturally occurring VHH domain or VH domain, respectively, a nucleotide sequence encoding the desired humanized or camelized immunoglobulin single variable domains, respectively, can be designed and then synthesized de novo using techniques for nucleic acid synthesis known per se, after which the nucleic acid thus obtained can be expressed in a manner known per se, so as to provide the desired immunoglobulin single variable domains. Other suitable methods and techniques for obtaining the immunoglobulin single variable domains described herein and/or nucleic acids encoding the same, starting from naturally occurring VH sequences or preferably VHH sequences, will be clear from the skilled person, and may for example comprise combining one or more parts of one or more naturally occurring VH sequences (such as one or more FR sequences and/or CDR sequences), one or more parts of one or more naturally occurring VHH sequences (such as one or more FR sequences or CDR sequences), and/or one or more synthetic or semi-synthetic sequences, in a suitable manner, so as to provide a Nanobody as described herein or a nucleotide sequence or nucleic acid encoding the same.

The present disclosure encompasses conformational-selective binding agents, in particular conformational-selective immunoglobulin single variable domains, targeting the muscarinic acetylcholine receptor M2, comprising an amino acid sequence that comprises 4 framework regions (FR1 to FR4) and 3 complementarity determining regions (CDR1 to CDR3), according to the following formula (1):

FR1-CDR1-FR2-CDR2-FR3-CDR3-FR4 (1)

and wherein CDR1 is chosen from the group consisting of:
a) SEQ ID NOs: 31-41, 105-112,
b) A polypeptide that has at least 80% amino acid identity with SEQ ID NOs: 31-41, 105-112,
c) A polypeptide that has 3, 2 or 1 amino acid difference with SEQ ID NOs: 31-41, 105-112,
and wherein CDR2 is chosen from the group consisting of:
a) SEQ ID NOs: 53-63, 121-128,
b) A polypeptide that has at least 80% amino acid identity with SEQ ID NOs: 53-63, 121-128,
c) A polypeptide that has 3, 2 or 1 amino acid difference with SEQ ID NOs: 53-63, 121-128,
and wherein CDR3 is chosen from the group consisting of:
a) SEQ ID NOs: 75-85, 137-144,
b) A polypeptide that has at least 80% amino acid identity with SEQ ID NOs: 75-85, 137-144,
c) A polypeptide that has 3, 2 or 1 amino acid difference with SEQ ID NOs: 75-85, 137-144.

In particular, the conformation-selective immunoglobulin single variable domain directed against and/or specifically binding to the muscarinic acetylcholine receptor M2 described herein may be a Nanobody or VHH, wherein the Nanobody has an amino acid sequence selected from the group consisting of SEQ ID NOs: 1-19 or variants thereof. Particularly, the present disclosure provides for an immunoglobulin single variable domain comprising an amino acid sequence that comprises 4 framework regions (FR1 to FR4) and 3 complementarity determining regions (CDR1 to CDR3), according to the following formula (1):

FR1-CDR1-FR2-CDR2-FR3-CDR3-FR4 (1);

wherein CDR1 is SEQ ID NO: 31, and CDR2 is SEQ ID NO: 53, and CDR3 is SEQ ID NO: 75; or wherein CDR1 is SEQ ID NO: 32, and CDR2 is SEQ ID NO: 54, and CDR3 is SEQ ID NO: 76; or wherein CDR1 is SEQ ID NO: 33, and CDR2 is SEQ ID NO: 55, and CDR3 is SEQ ID NO: 77; or wherein CDR1 is SEQ ID NO: 34, and CDR2 is SEQ ID NO: 56, and CDR3 is SEQ ID NO: 78; or wherein CDR1 is SEQ ID NO: 35, and CDR2 is SEQ ID NO: 57, and CDR3 is SEQ ID NO: 79; or wherein CDR1 is SEQ ID NO: 36, and CDR2 is SEQ ID NO: 58, and CDR3 is SEQ ID NO: 80; or wherein CDR1 is SEQ ID NO: 37, and CDR2 is SEQ ID NO: 59, and CDR3 is SEQ ID NO: 81; or wherein CDR1 is SEQ ID NO: 38, and CDR2 is SEQ ID NO: 60, and CDR3 is SEQ ID NO: 82; or wherein CDR1 is SEQ ID NO: 39, and CDR2 is SEQ ID NO: 61, and CDR3 is SEQ ID NO: 83, or wherein CDR1 is SEQ ID NO: 40, and CDR2 is SEQ ID NO: 62, and CDR3 is SEQ ID NO: 84, or wherein CDR1 is SEQ ID NO: 41, and CDR2 is SEQ ID NO: 63, and CDR3 is SEQ ID NO: 85, or wherein CDR1 is SEQ ID NO: 105, and CDR2 is SEQ ID NO: 121, and CDR3 is SEQ ID NO: 137, or wherein CDR1 is SEQ ID NO: 106, and CDR2 is SEQ ID NO: 122, and CDR3 is SEQ ID NO: 138, or wherein CDR1 is SEQ ID NO: 107, and CDR2 is SEQ ID NO: 123, and CDR3 is SEQ ID NO: 139, or wherein CDR1 is SEQ ID NO: 108, and CDR2 is SEQ ID NO: 124, and CDR3 is SEQ ID NO: 140, or wherein CDR1 is SEQ ID NO: 109, and CDR2 is SEQ ID NO: 125, and CDR3 is SEQ ID NO: 141, or wherein CDR1 is SEQ ID NO: 110, and CDR2 is SEQ ID NO: 126, and CDR3 is SEQ ID NO: 142, or wherein CDR1 is SEQ ID NO: 111, and CDR2 is SEQ ID NO: 127, and CDR3 is SEQ ID NO: 143, or wherein CDR1 is SEQ ID NO: 112, and CDR2 is SEQ ID NO: 128, and CDR3 is SEQ ID NO: 144.

More preferably, the conformation-selective binding agents, in particular immunoglobulin single variable domains, directed against and/or specifically binding to muscarinic acetylcholine receptor M2 have an amino acid sequence chosen from the group consisting of SEQ ID NOs: 1-19. The conformation-selective binding agents described herein may be defined by SEQ ID NOs: 1-19.

In particular, non-limiting examples of conformation-selective binding agents directed against and/or specifically binding to muscarinic acetylcholine receptor M2, that are specifically characterized as G protein mimetics (as defined hereinbefore) immunoglobulin single variable domains that have an amino acid sequence chosen from the group consisting of SEQ ID NOs: 1-11. Thus, the conformation-selective binding agents directed against and/or specifically binding to muscarinic acetylcholine receptor M2, may have an amino acid sequence chosen from the group consisting of SEQ ID NOs: 1-11. Preferably, the conformation-selective binding agents described herein may have an amino acid sequence as defined by SEQ ID NO: 1. Non-limiting examples of conformation-selective binding agents directed against and/or specifically binding to muscarinic acetylcholine receptor M2, that are specifically characterized as binding to an extracellular conformational epitope are immunoglobulin single variable domains that have an amino acid sequence chosen from the group consisting of SEQ ID NOs: 12-19. Thus the conformation-selective binding agents directed against and/or specifically binding to muscarinic acetylcholine receptor M2, may have an amino acid sequence chosen from the group consisting of SEQ ID NOs: 12-19.

Also within the scope of the disclosure are natural or synthetic analogs, mutants, variants, alleles, parts or fragments (herein collectively referred to as "variants") of the immunoglobulin single variable domains, in particular the nanobodies, as defined herein, and in particular variants of the immunoglobulin single variable domains of SEQ ID NOs: 1-19 (see Tables 1-2). Generally, in such variants, one or more amino acid residues may have been replaced, deleted and/or added, compared to the immunoglobulin single variable domains as defined herein. Such substitutions, insertions or deletions may be made in one or more of the FR's and/or in one or more of the CDR's, and in particular variants of the FR's and CDR's of the immunoglobulin single variable domains of SEQ ID NOs: 1-19 (see Tables 1-2). Variants, as used herein, are sequences wherein each or any framework region and each or any complementarity determining region shows at least 80% identity, preferably at least 85% identity, more preferably 90% identity, even more preferably 95% identity or, still even more preferably 99% identity with the corresponding region in the reference sequence (i.e. FR1_variant versus FR1_reference, CDR1_variant versus CDR1_reference, FR2_variant versus FR2_reference, CDR2_variant versus CDR2_reference, FR3_variant versus FR3_reference, CDR3_variant versus CDR3_reference, FR4_variant versus FR4_reference), as can be measured electronically by making use of algorithms such as PILEUP and BLAST (50, 51). Software for performing BLAST analyses is publicly available through the National Center for Biotechnology Information (http://www/ncbi.nlm.nih.gov/). It will be understood that for determining the degree of amino acid identity of the amino acid sequences of the CDRs of one or more sequences of the immunoglobulin single variable domains, the amino acid residues that form the framework regions are disregarded. Similarly, for determining the degree of amino acid identity of the amino acid sequences of the FRs of one or more sequences of the immunoglobulin single variable domains described herein, the amino acid residues that form the complementarity regions are disregarded. Such variants of immunoglobulin single variable domains may be of particular advantage since they may have improved potency/affinity.

By means of non-limiting examples, a substitution may for example be a conservative substitution (as described herein) and/or an amino acid residue may be replaced by another amino acid residue that naturally occurs at the same position in another VHH domain. Thus, any one or more substitutions, deletions or insertions, or any combination thereof, that either improve the properties of the immunoglobulin single variable domains or that do not detract from the desired properties or from the balance or combination of desired properties of the immunoglobulin single variable domain (i.e. to the extent that the immunoglobulin single variable domains is no longer suited for its intended use) are included within the scope of the disclosure. A skilled person will generally be able to determine and select suitable substitutions, deletions or insertions, or suitable combinations of thereof, based on the disclosure herein and optionally after a limited degree of routine experimentation, which may for example involve introducing a limited number of possible substitutions and determining their influence on the properties of the immunoglobulin single variable domains thus obtained.

Also encompassed within the scope of the present disclosure are immunoglobulin single variable domains that are in a "multivalent" form and are formed by bonding, chemically or by recombinant DNA techniques, together two or more monovalent immunoglobulin single variable domains. Non-limiting examples of multivalent constructs include "bivalent" constructs, "trivalent" constructs, "tetravalent" constructs, and so on. The immunoglobulin single variable domains comprised within a multivalent construct may be identical or different. In another example, the immunoglobulin single variable domains described herein are in a "multispecific" form and are formed by bonding together two or more immunoglobulin single variable domains, of which at least one with a different specificity. Non-limiting examples of multi-specific constructs include "bi-specific" constructs, "tri-specific" constructs, "tetra-specific" constructs, and so on. To illustrate this further, any multivalent or multispecific (as defined herein) immunoglobulin single variable domain described herein may be suitably directed against two or more different epitopes on the same antigen, for example against two or more different epitopes of the muscarinic acetylcholine receptor M2; or may be directed against two or more different antigens, for example against an epitope of muscarinic acetylcholine receptor M2 and an epitope of a natural binding partner of the muscarinic acetylcholine receptor M2 (e.g. G protein, β-arrestin). In particular, a monovalent immunoglobulin single variable domain as described herein is such that it will bind to the target receptor with an affinity less than 500 nM, preferably less than 200 nM, more preferably less than 10 nM, such as less than 500 pM. Multivalent or multispecific immunoglobulin single variable domains as described herein may also have (or be engineered and/or selected for) increased avidity and/or improved selectivity for the desired receptor, and/or for any other desired property or combination of desired properties that may be obtained by the use of such multivalent or multispecific immunoglobulin single variable domains. Such multivalent or multispecific binding domains may also have (or be engineered and/or selected for) improved efficacy in modulating signaling activity of a GPCR (see also further herein). It will be appreciated that the multivalent or multispecific binding domains as described herein may additionaly be suitably directed to a different antigen, such as, but not limiting to, a ligand interacting with a muscarinic acetylcholine receptor or one or more downstream signaling proteins.

Further, and depending on the host organism used to express the binding agent as described herein, deletions and/or substitutions within the binding agent may be designed in such a way that e.g. one or more sites for post-translational modification (such as one or more glycosylation sites) are removed, as will be within the ability of the person skilled in the art. Alternatively, substitutions or insertions may be designed so as to introduce one or more sites for attachment of functional groups (as described further herein).

It is also expected that the conformation-selective binding agent will generally be capable of binding to all naturally occurring or synthetic analogs, variants, mutants, alleles, parts, fragments, and isoforms of a muscarinic acetylcholine receptor, in particular M2R; or at least to those analogs, variants, mutants, alleles, parts, fragments, and isoforms of a muscarinic acetylcholine receptor that contain one or more antigenic determinants or epitopes that are essentially the same as the antigenic determinant(s) or epitope(s) to which the binding agents as described herein bind to a muscarinic acetylcholine receptor.

Also provided herein is a complex comprising a muscarinic receptor, preferably muscarinic receptor M2, and a conformation-selective binding agent that is directed against and/or specifically binds to the muscarinic receptor. As a non-limiting example, a stable complex may be purified by size exclusion chromatography. The complex as described above may further comprise at least one other conformation-selective receptor ligand (as defined herein). Non-limiting examples of conformation-selective receptor ligands include full agonists, partial agonists, inverse agonists, natural binding partners, allosteric modulators, and the like. To illustrate this further, without the purpose of being limitative, agonists of muscarinic receptor M2 are known in the art and include xanomeline, oxotremorine, acetylcholine, carbachol, pilocarpine, furmethide, bethanechol, amongst others. Antagonists of muscarinic receptor M2 are known in the art and include atropine, tripitramine, propantheline, scopolamine, amonst others. Inverse agonists of muscarinic receptor M2 are known in the art and include tolterodine, oxybutynin, darifenacin, amongst others. Allosteric modulators of muscarinic receptor M2 are known in the art and include staurosporine, vincamine, brucine, gallamine, amongst others. Further examples can be found in the IUPHAR database (http://www.iuphar-db.org/).

The conformation-selective binding agent and/or the complex as described herein may be in a solubilized form, such as in a detergent. Alternatively, the conformation-selective binding agent and/or the complex may be immobilized to a solid support. Non-limiting examples of solid supports as well as methods and techniques for immobilization are described further in the detailed description. In still another example, the conformation-selective binding agent and/or complex as described herein is in a cellular composition, including an organism, a tissue, a cell, a cell line, or in a membrane composition or liposomal composition derived from said organism, tissue, cell or cell line. Examples of membrane or liposomal compositions include, but are not limited to organelles, membrane preparations, viruses, Virus Like Lipoparticles, and the like. It will be appreciated that a cellular composition, or a membrane or liposomal composition may comprise natural or synthetic lipids.The complex may be crystalline. So, a crystal of the complex is also provided, as well as methods of making said crystal, which are described in greater detail below. Preferably, a crystalline form of a complex as described herein and a receptor ligand is envisaged.

### Screening and selection of conformational-selective binding agents against muscarinic acetylcholine receptors

Conformation-selective binding agents, in particular immunoglobulin single variable domains, can be identified in several ways, and will be illustrated hereafter in a non-limiting way for VHHs. Although naive or synthetic libraries of VHHs (for examples of such libraries, see WO9937681, WO0043507, WO0190190, WO03025020 and WO03035694) may contain conformational binders against a muscarinic receptor in a functional conformation, a preferred example includes the immunization of a *Camelidae* with a muscarinic receptor in a functional conformation, optionally bound to a receptor ligand, to expose the immune system of the animal with the conformational epitopes that are unique to the receptor in that particular conformation (for example, agonist-bound muscarinic receptor so as to raise antibodies directed against the receptor in its active conformational state). Optionally, a particular ligand can be coupled to the receptor of interest by chemical cross-linking. Thus, as further described herein, such VHH sequences can preferably be generated or obtained by suitably immunizing a species of Camelid with a muscarinic receptor, preferably a receptor in a functional conformational state (i.e. so as to raise an immune response and/or heavy chain antibodies directed against said receptor), by obtaining a suitable biological sample from said Camelid (such as a blood sample, or any sample of B-cells), and by generating VHH sequences directed against said receptor, starting from said sample. Such techniques will be clear to the skilled person. Yet another technique for obtaining the desired VHH sequences involves suitably immunizing a transgenic mammal that is capable of expressing heavy chain antibodies (i.e. so as to raise an immune response and/or heavy chain antibodies directed against a muscarinic receptor in a functional conformational state), obtaining a suitable biological sample from said transgenic mammal (such as a blood sample, or any sample of B-cells), and then generating VHH sequences directed against said receptor starting from said sample, using any suitable technique known per se. For example, for this purpose, the heavy chain antibody-expressing mice and the further methods and techniques described in WO02085945 and in WO04049794 can be used.

For the immunization of an animal with a muscarinic acetylcholine receptor, the receptor may be produced and purified using conventional methods that may employ expressing a recombinant form of the protein in a host cell, and purifying the protein using affinity chromatography and/or antibody-based methods.The baculovirus/Sf-9 system may be employed for expression, although other expression systems (e.g., bacterial, yeast or mammalian cell systems) may also be used. Exemplary methods for expressing and purifying GPCRs like the muscarinic acetylcholine receptor are described in, for example, Kobilka (1995), Eroglu et al (2002), Chelikani et al (2006) and the book "Identification and Expression of G Protein-Coupled Receptors" (Kevin R. Lynch (Ed.), 1998), among many others. A GPCR such as a muscarinic acetylcholine receptor may also be reconstituted in phospholipid vesicles. Likewise, methods for reconstituting an active GPCR in phospholipid vesicles are known, and are described in: Luca et al (2003), Mansoor et al (2006), Niu et al. (2005), Shimada et al. (2002), and Eroglu et al. (2003), among others. In certain cases, the GPCR and phospholipids may be reconstituted at high density (e.g., 1 mg receptor per mg of phospholipid).The phospholipids vesicles may be tested to confirm that the GPCR is active. In many cases, a GPCR may be present in the phospholipid vesicle in both orientations (in the normal orientation, and in the "upside down" orientation in which the intracellular loops are on the outside of the vesicle). Other immunization methods include, without limitation, the use of complete cells expressing a muscarinic acetylcholine receptor or fractions thereof, vaccination with a nucleic acid sequence encoding a muscarinic acetylcholine receptor (e.g. DNA vaccination), immunization with viruses or virus like particles expressing a muscarinic acetylcholine receptor, amongst others (e.g. as described in WO2010070145, WO2011083141).

Any suitable animal, in particular a mammal such as a rabbit, mouse, rat, camel, sheep, cow, pig, amongst others, or a bird such as a chicken or turkey, or a fish, such as a shark, may be immunized using any of the techniques well known in the art suitable for generating an immune response.

The selection for VHHs or Nanobodies, as a non-limiting example, specifically binding to a conformational epitope of a functional conformational state of a muscarinic receptor may for example be performed by screening a set, collection or library of cells that express heavy chain antibodies on their surface (e.g. B-cells obtained from a suitably immunized Camelid), or bacteriophages that display a fusion of genlll and Nanobody at their surface, or yeast cells that display a fusion of the mating factor protein Aga2p, by screening of a (naive or immune) library of VHH sequences or Nanobody sequences, or by screening of a (naive or immune) library of nucleic acid sequences that encode VHH sequences or Nanobody sequences, which may all be performed in a manner known per se, and which method may optionally further comprise one or more other suitable steps, such as, for example and without limitation, a step of affinity maturation, a step of expressing the desired amino acid sequence, a step of screening for binding and/or for activity against the desired antigen (in this case, the muscarinic receptor in a particular conformation), a step of determining the desired amino acid sequence or nucleotide sequence, a step of introducing one or more humanizing substitutions, a step of formatting in a suitable multivalent and/or multispecific format, a step of screening for the desired biological and/or physiological properties (i.e. using a suitable assay known in the art), and/or any combination of one or more of such steps, in any suitable order.

Various methods may be used to determine specific binding (as defined hereinbefore) between the binding agent and a target muscarinic receptor, including for example, enzyme linked immunosorbent assays (ELISA), flow cytometry, radioligand binding assays, surface Plasmon resonance assays, phage display, and the like, which are common practice in the art, for example, in discussed in Sambrook et al. (2001), Molecular Cloning, A Laboratory Manual. Third Edition. Cold Spring Harbor Laboratory Press, Cold Spring Harbor, NY, and are further illustrated in the Example section. It will be appreciated that for this purpose often a unique label or tag will be used, such as a peptide label, a nucleic acid label, a chemical label, a fluorescent label, or a radio isotope label, as described further herein.

A particularly preferred way of selecting for conformation-selective binding agents is as described in, for example, WO 2012/007593. In an alternative example, selection for conformation-selective binding agents can also be performed by using cell sorting to select, from a population of cells comprising a library of cell-surface tethered extracellular binding agents, cells that are specifically bound to either the muscarinic receptor in its active conformation or the muscarinic receptor in its inactive conformation, but not both. Without the purpose of being limitative, selection for conformation-selective binding agents is also further illustrated in the Example section.

### Modifications of conformational-selective binding agents

The conformation-selective binding agents described herein may be further modified and/or may comprise (or can be fused to) other moieties, as described further herein. Examples of modifications, as well as examples of amino acid residues within the binding agent described herein that can be modified (i.e. either on the protein backbone but preferably on a side chain), methods and techniques that can be used to introduce such modifications and the potential uses and advantages of such modifications will be clear to the skilled person. For example, such a modification may involve the introduction (e.g. by covalent linking or in another suitable manner) of one or more functional groups, residues or moieties into or onto the binding agent. Examples of such functional groups and of techniques for introducing them will be clear to the skilled person, and can generally comprise all functional groups and techniques mentioned in the art as well as the functional groups and techniques known per se for the modification of pharmaceutical proteins, and in particular for the modification of antibodies or antibody fragments (including ScFv's and single domain antibodies), for which reference is for example made to Remington's Pharmaceutical Sciences, 16th ed., Mack Publishing Co., Easton, PA (1980). Such functional groups may for example be linked directly (for example covalently) to the binding agent, or optionally via a suitable linker or spacer, as will again be clear to the skilled person.

One of the most widely used techniques for increasing the half-life and/or reducing immunogenicity of pharmaceutical proteins comprises attachment of a suitable pharmacologically acceptable polymer, such as poly(ethyleneglycol) (PEG) or derivatives thereof (such as methoxypoly(ethyleneglycol) or mPEG). Generally, any suitable form of pegylation can be used, such as the pegylation used in the art for antibodies and antibody fragments (including but not limited to (single) domain antibodies and ScFv's); reference is made to for example Chapman, Nat. Biotechnol., 54, 531-545 (2002); by Veronese and Harris, Adv. Drug Deliv. Rev. 54, 453-456 (2003), by Harris and Chess, Nat. Rev. Drug. Discov., 2, (2003) and in WO04060965. Various reagents for pegylation of proteins are also commercially available, for example from Nektar Therapeutics, USA. Preferably, site-directed pegylation is used, in particular via a cysteine-residue (see for example Yang et al., Protein Engineering, 16, 10, 761-770 (2003). For example, for this purpose, PEG may be attached to a cysteine residue that naturally occurs in an binding agent, or the binding agent may be modified so as to suitably introduce one or more cysteine residues for attachment of PEG, or an amino acid sequence comprising one or more cysteine residues for attachment of PEG may be fused to the N- and/or C-terminus of an binding agent, all using techniques of protein engineering known per se to the skilled person. Preferably, for the binding agents described herein, a PEG can be used with a molecular weight of more than 5000, such as more than 10,000 and less than 200,000, such as less than 100,000; for example in the range of 20,000-80,000. Another, usually less preferred modification comprises N-linked or O-linked glycosylation, usually as part of co-translational and/or post-translational modification, depending on the host cell used for expressing the immunoglobulin single variable domain or polypeptide. Another technique for increasing the half-life of a binding agent may comprise the engineering into bifunctional constructs (for example, one Nanobody against the target M2R and one against a serum protein such as albumin) or into fusions of binding agents with peptides (for example, a peptide against a serum protein such as albumin).

A usually less preferred modification comprises N-linked or O-linked glycosylation, usually as part of co-translational and/or post-translational modification, depending on the host cell used for expressing the binding agent described herein.

Yet another modification may comprise the introduction of one or more detectable labels or other signal-generating groups or moieties, depending on the intended use of the labeled binding agent. Suitable labels and techniques for attaching, using and detecting them will be clear to the skilled person, and for example include, but are not limited to, fluorescent labels, (such as IRDye800, VivoTag800, fluorescein, isothiocyanate, rhodamine, phycoerythrin, phycocyanin, allophycocyanin, o-phthaldehyde, and fluorescamine and fluorescent metals such as Eu or others metals from the lanthanide series), phosphorescent labels, chemiluminescent labels or bioluminescent labels (such as luminal, isoluminol, theromatic acridinium ester, imidazole, acridinium salts, oxalate ester, dioxetane or GFP and its analogs), radio-isotopes, metals, metals chelates or metallic cations or other metals or metallic cations that are particularly suited for use in in vivo, in vitro or in situ diagnosis and imaging, as well as chromophores and enzymes (such as malate dehydrogenase, staphylococcal nuclease, delta- V-steroid isomerase, yeast alcohol dehydrogenase, alpha-glycerophosphate dehydrogenase, triose phosphate isomerase, biotinavidin peroxidase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholine esterase). Other suitable labels will be clear to the skilled person, and for example include moieties that can be detected using NMR or ESR spectroscopy. Such labelled binding agents of the disclosure may for example be used for in vitro, in vivo or in situ assays (including immunoassays known per se such as ELISA, RIA, EIA and other "sandwich assays", etc.) as well as in vivo diagnostic and imaging purposes, depending on the choice of the specific label. As will be clear to the skilled person, another modification may involve the introduction of a chelating group, for example to chelate one of the metals or metallic cations referred to above. Suitable chelating groups for example include, without limitation, 2,2',2"-(10-(2-((2,5-dioxopyrrolidin-1-yl)oxy)-2-oxoethyl)-1,4,7,10-tetraazacyclododecane-1,4,7-triyl)triacetic acid (DOTA), 2,2'-(7-(2-((2,5-dioxopyrrolidin-1-yl)oxy)-2-oxoethyl)-1,4,7-triazonane-1,4-diyl)diacetic acid (NOTA), diethyl- enetriaminepentaacetic acid (DTPA) or ethylenediaminetetraacetic acid (EDTA). Yet another modification may comprise the introduction of a functional group that is one part of a specific binding pair, such as the biotin-(strept)avidin binding pair. Such a functional group may be used to link the binding agent to another protein, polypeptide or chemical compound that is bound to the other half of the binding pair, i.e. through formation of the binding pair. For example, a binding agent as described herein may be conjugated to biotin, and linked to another protein, polypeptide, compound or carrier conjugated to avidin or streptavidin. For example, such a conjugated binding agent may be used as a reporter, for example in a diagnostic system where a detectable signal-producing agent is conjugated to avidin or streptavidin. Such binding pairs may for example also be used to bind the binding agent described herein to a carrier, including carriers suitable for pharmaceutical purposes. One non-limiting example are the liposomal formulations described by Cao and Suresh, Journal of Drug Targetting, 8, 4, 257 (2000). Such binding pairs may also be used to link a therapeutically active agent to the binding agent described herein.

In case conformation-selective binding agents are modified by linking particular functional groups, residues or moieties (as described hereinabove) to the binding agent, then often linker molecules will be used. Preferred "linker molecules" or "linkers" are peptides of 1 to 200 amino acids length, and are typically, but not necessarily, chosen or designed to be unstructured and flexible. For instance, one can choose amino acids that form no particular secondary structure. Or, amino acids can be chosen so that they do not form a stable tertiary structure. Or, the amino acid linkers may form a random coil. Such linkers include, but are not limited to, synthetic peptides rich in Gly, Ser, Thr, Gin, Glu or further amino acids that are frequently associated with unstructured regions in natural proteins (Dosztányi, Z., Csizmok, V., Tompa, P., & Simon, I. (2005). IUPred: web server for the prediction of intrinsically unstructured regions of proteins based on estimated energy content. Bioinformatics (Oxford, England), 21(16), 3433-4.). Non-limiting examples of suitable linker sequences include (GS)5 (GSGSGSGSGS; SEQ ID NO: 156), (GS)10 (GSGSGSGSGSGSGSGSGSGS; SEQ ID NO: 157), (G4S)3 (GGGGSGGGGSGGGGS; SEQ ID NO: 158), llama IgG2 hinge (AHHSEDPSSKAPKAPMA; SEQ ID NO: 159) or human IgA hinge (SPSTPPTPSPSTPPAS; SEQ ID NO: 160) linkers.

Thus, the amino acid (AA) linker sequence may be a peptide of between 0 and 200 AA, between 0 and 150 AA, between 0 and 100 AA, between 0 and 90 AA, between 0 and 80 AA, between 0 and 70 AA, between 0 and 60 AA, between 0 and 50 AA, between 0 and 40 AA, between 0 and 30 amino acids, between 0 and 20 AA, between 0 and 10 amino acids, between 0 and 5 amino acids. Examples of sequences of short linkers include, but are not limited to, PPP, PP or GS.

For certain applications, it may be advantageous that the linker molecule comprises or consists of one or more particular sequence motifs. For example, a proteolytic cleavage site can be introduced into the linker molecule such that detectable label or moiety can be released. Useful cleavage sites are known in the art, and include a protease cleavage site such as Factor Xa cleavage site having the sequence IEGR (SEQ ID NO: 161), the thrombin cleavage site having the sequence LVPR (SEQ ID NO: 162), the enterokinase cleaving site having the sequence DDDDK (SEQ ID NO: 163), or the PreScission cleavage site LEVLFQGP (SEQ ID NO: 164).

Alternatively, in case the binding agent is linked to a detectable label or moiety using chemoenzymatic methods for protein modification, the linker moiety may exist of different chemical entities, depending on the enzymes or the synthetic chemistry that is used to produce the covalently coupled molecule in vivo or in vitro (reviewed in: Rabuka 2010, Curr Opin Chem Biol 14: 790-796).

### Expression systems

The disclosure also relates to a nucleic acid molecule comprising a nucleic acid sequence encoding any of the conformation-selective binding agents as described hereinbefore. Further, the present disclosure also envisages expression vectors comprising nucleic acid sequences encoding any of the conformation-selective binding agents described herein, as well as host cells expressing such expression vectors. Suitable expression systems include constitutive and inducible expression systems in bacteria or yeasts, virus expression systems, such as baculovirus, semliki forest virus and lentiviruses, or transient transfection in insect or mammalian cells. The cloning and/or expression of the conformation-selective binding agents described herein can be done according to techniques known by the skilled person in the art.

The "host cell" according to the present disclosure can be of any prokaryotic or eukaryotic organism. Preferably, the host cell is a eukaryotic cell and can be of any eukaryotic organism, but in particular examples yeast, plant, mammalian and insect cells are envisaged. The nature of the cells used will typically depend on the ease and cost of producing the binding agent, the desired glycosylation properties, the origin of the binding agent, the intended application, or any combination thereof.

Mammalian cells may for instance be used for achieving complex glycosylation, but it may not be cost-effective to produce proteins in mammalian cell systems. Plant and insect cells, as well as yeast typically achieve high production levels and are more cost-effective, but additional modifications may be needed to mimic the complex glycosylation patterns of mammalian proteins. Yeast cells are often used for expression of proteins because they can be economically cultured, give high yields of protein, and when appropriately modified are capable of producing proteins having suitable glycosylation patterns. Further, yeast offers established genetics allowing for rapid transformations, tested protein localization strategies, and facile gene knock-out techniques. Insect cells are also an attractive system to express GPCRs including muscarinic receptors because insect cells offer an expression system without interfering GPCRs and with a limited set of G-proteins. Eukaryotic cell or cell lines for protein production are well known in the art, including cell lines with modified glycosylation pathways, and non-limiting examples will be provided hereafter.

Animal or mammalian host cells suitable for harboring, expressing, and producing proteins for subsequent isolation and/or purification include Chinese hamster ovary cells (CHO), such as CHO-K1 (ATCC CCL-61), DG44 (Chasin et al., 1986; Kolkekar et al., 1997), CHO-K1 Tet-On cell line (Clontech), CHO designated ECACC 85050302 (CAMR, Salisbury, Wiltshire, UK), CHO clone 13 (GEIMG, Genova, IT), CHO clone B (GEIMG, Genova, IT), CHO-K1/SF designated ECACC 93061607 (CAMR, Salisbury, Wiltshire, UK), RR-CHOK1 designated ECACC 92052129 (CAMR, Salisbury, Wiltshire, UK), dihydrofolate reductase negative CHO cells (CHO/-DHFR, Urlaub and Chasin, 1980), and dp12.CHO cells (U.S. Pat. No. 5,721,121); monkey kidney CV1 cells transformed by SV40 (COS cells, COS-7, ATCC CRL-1651); human embryonic kidney cells (e.g., 293 cells, or 293T cells, or 293 cells subcloned for growth in suspension culture, Graham et al., 1977, J. Gen. Virol., 36:59, or GnTI KO HEK293S cells, Reeves et al. 2002); baby hamster kidney cells (BHK, ATCC CCL-10); monkey kidney cells (CV1, ATCC CCL-70); African green monkey kidney cells (VERO-76, ATCC CRL-1587; VERO, ATCC CCL-81); mouse sertoli cells (TM4, Mather, 1980, Biol. Reprod., 23:243-251); human cervical carcinoma cells (HELA, ATCC CCL-2); canine kidney cells (MDCK, ATCC CCL-34); human lung cells (W138, ATCC CCL-75); human hepatoma cells (HEP-G2, HB 8065); mouse mammary tumor cells (MMT 060562, ATCC CCL-51); buffalo rat liver cells (BRL 3A, ATCC CRL-1442); TRI cells (Mather, 1982); MCR 5 cells; FS4 cells. For example, the cells may be mammalian cells selected from Hek293 cells or COS cells.

Exemplary non-mammalian cell lines include, but are not limited to, insect cells, such as Sf9 cells/baculovirus expression systems (e.g. review Jarvis, Virology Volume 310, Issue 1, 25 May 2003, Pages 1-7), plant cells such as tobacco cells, tomato cells, maize cells, algae cells, or yeasts such as Saccharomyces species, Schizosaccharomyces species, Hansenula species, Yarrowia species or Pichia species. For example, the eukaryotic cells may be yeast cells from a Saccharomyces species (e.g. Saccharomyces cerevisiae), Schizosaccharomyces sp. (for example Schizosaccharomyces pombe), a Hansenula species (e.g. Hansenula polymorpha), a Yarrowia species (e.g. Yarrowia lipolytica), a Kluyveromyces species (e.g. Kluyveromyces lactis), a Pichia species (e.g. Pichia pastoris), or a Komagataella species (e.g. Komagataella pastoris). For example, the eukaryotic cells may be Pichia cells, and most particularly Pichia pastoris cells.

Transfection of target cells (e.g. mammalian cells) can be carried out following principles outlined by Sambrook and Russel (Molecular Cloning, A Laboratory Manual, 3rd Edition, Volume 3, Chapter 16, Section 16.1-16.54). In addition, viral transduction can also be performed using reagents such as adenoviral vectors. Selection of the appropriate viral vector system, regulatory regions and host cell is common knowledge within the level of ordinary skill in the art. The resulting transfected cells are maintained in culture or frozen for later use according to standard practices.

Accordingly, another aspect of the disclosure relates to a method for producing a conformation-selective binding agent as described herein, the method comprising at least the steps of:
a) Expressing in a suitable cellular expression system (as defined hereinabove) a nucleic acid encoding a conformation-selective binding agent as described herein, and optionally
b) Isolating and/or purifying said binding agent.

The above described conformation-selective binding agents as well as the complexes comprising the same are particularly useful for screening and drug discovery (in its broadest sense), all of which is now detailed further herein.

### Applications

The herein described conformation-selective binding agents can be used in a variety of contexts and applications, for example and without limitation, (1) for capturing and/or purification of a muscarinic acetylcholine receptor, more specifically M2R, whereby upon binding, the conformation-selective binding agent maintains the receptor in a particular conformation; (2) for co-crystallization studies and high-resolution structural analysis of a muscarinic acetylcholine receptor, more specifically M2R, in complex with the conformation-selective binding agent, and optionally additionally bound to another conformation-selective receptor ligand; (3) for ligand screening, and (structure-based) drug discovery; (4) as therapeutics and/or diagnostics, all of which will be described into further detail below.

### Capturing, separation and purification methods for muscarinic acetylcholine receptor in a functional conformation

The disclosure further provides a method for capturing and/or purifying a muscarinic acetylcholine receptor, more specifically M2R, in a functional conformation by making use of any of the above described conformation-selective binding agents. Capturing and/or purifying a receptor in a functional conformation will allow subsequent crystallization, ligand characterization and compound screening, immunizations, amongst others.

Thus, the disclosure relates to the use of a conformation-selective binding agent as described herein to capture a muscarinic acetylcholine receptor in an active or inactive conformation. Optionally, but not necessarily, capturing of a receptor in a particular conformation as described above may include capturing a receptor in complex with another conformation-selective receptor ligand (e.g. an orthosteric ligand, an allosteric ligand, a natural binding partner such as a G protein or an arrestin, and the like).

In accordance, the disclosure also provides a method of capturing a muscarinic acetylcholine receptor, in particular M2R, in a functional conformation, said method comprising the steps of:
(i) bringing a conformation-selective binding agent as described herein into contact with a solution comprising a muscarinic acetylcholine receptor, more specifically M2R, and
(ii) allowing the binding agent to specifically bind to the muscarinic acetylcholine receptor M2, whereby muscarinic acetylcholine receptor M2 is captured in a functional conformation.

More specifically, the disclosure also envisages a method of capturing a muscarinic acetylcholine receptor, in particular M2R, in a functional conformation, said method comprising the steps of:
(i) applying a solution containing a muscarinic acetylcholine receptor, more specifically M2R, in a plurality of conformations to a solid support possessing an immobilized conformation-selective binding agent as described herein, and
(ii) allowing the binding agent to specifically bind to the muscarinic acetylcholine receptor M2, whereby muscarinic acetylcholine receptor M2 is captured in a functional conformation, and
(iii) removing weakly bound or unbound molecules.

It will be appreciated that any of the methods as described above may further comprise the step of isolating the complex formed in step (ii) of the above described methods, said complex comprising the conformation-selective binding agent and the muscarinic acetylcholine receptor in a particular conformation.

The above methods for isolating/purifying muscarinic receptors include, without limitation, affinity-based methods such as affinity chromatography, affinity purification, immunoprecipitation, protein detection, immunochemistry, surface-display, size exclusion chromatography, ion exchange chromatography, amongst others, and are all well-known in the art.

### Crystallography and applications in structure-based drug design

An aspect of the present disclosure relates to the usefulness of the conformation-selective binding agents described herein in X-ray crystallography of muscarinic acetylcholine receptors, in particular M2, and its applications in structure-based drug design. With the inactive-state structures of muscarinic acetylcholine receptor M2 and M3 that are available in the art, pharmaceutical chemists now have experimental data to guide the development of ligands for several active therapeutic targets. However, the value of these high-resolution structures for in silico screening is limited. On the other hand, and as a matter of illustration, agonist-bound receptor crystals may provide three-dimensional representations of the active states of muscarinic acetylcholine receptors. These structures will help clarify the conformational changes connecting the ligand-binding and G-protein-interaction sites, and lead to more precise mechanistic hypotheses and eventually new therapeutics. Given the conformational flexibility inherent to ligand-activated GPCRs and the greater heterogeneity exhibited by agonist-bound receptors, stabilizing such a state is not easy. Such efforts can benefit from the stabilization of the agonist-bound receptor conformation by the addition of binding agents that are specific for an active conformational state of the receptor. In that regard, it is a particular advantage of the conformation-selective binding agents that they show G-protein like behavior and exhibit cooperative properties with respect to agonist binding (see also Example section). This will also be of great advantage to help guide drug discovery. Especially methods for acquiring structures of receptors bound to lead compounds that have pharmacological or biological activity and whose chemical structure is used as a starting point for chemical modifications in order to improve potency, selectivity, or pharmacokinetic parameters are very valuable and are provided herein. Persons of ordinary skill in the art will recognize that the conformation-selective binding agent described herein is particularly suited for co-crystallization of receptor:binding agent with lead compounds that are selective for the druggable conformation induced by the binding agent because this binding agent is able to substantially increase the affinity for conformation-selective receptor ligands.

It is thus a particular advantage of the conformation-selective binding agents described herein that the binding agent binds a conformational epitope on the receptor, thus stabilizing the receptor in that particular conformation, reducing its conformational flexibility and increasing its polar surface, facilitating the crystallization of a receptor:binding agent complex. The conformation-selective binding agents described herein are unique tools to increase the probability of obtaining well-ordered crystals by minimizing the conformational heterogeneity in the target muscarinic acetylcholine receptor.

Thus, it is envisaged to use the conformation-selective binding agents described herein for crystallization purposes. Advantageously, crystals can be formed of a complex of a conformation-selective binding agent and the muscarinic acetylcholine receptor, wherein the receptor is trapped in a particular receptor conformation, more particularly a therapeutically relevant receptor conformation (e.g. an active conformation), as ensured by the choice of a conformationally-selective binding agent. The binding agent will also reduce the flexibility of extracellular regions upon binding the receptor to grow well-ordered crystals. In particular, immunoglobulin single variable domains, including Nanobodies, are especially suitable binding agents for this purpose because they bind conformational epitopes and are composed of one single rigid globular domain, devoid of flexible linker regions unlike conventional antibodies or fragments derived such as Fab's.

Thus, the present disclosure provides for conformation-selective binding agents useful as tools for crystallizing a complex of a conformation-selective binding agent and a muscarinic acetylcholine receptor to which the binding agent will specifically bind, and eventually to solve the structure of the complex. The present disclosure also envisages to crystallize a complex of conformation-selective binding agent, a muscarinic acetylcholine receptor to which the binding agent will specifically bind, and another conformation-selective receptor ligand (as defined hereinbefore). Thus, the complex comprising the conformation-selective binding agent described herein and the muscarinic acetylcholine receptor maintained in a particular conformation, may be crystallized using any of a variety of specialized crystallization methods for membrane proteins, many of which are reviewed in Caffrey (2003 & 2009). In general terms, the methods are lipid-based methods that include adding lipid to the complex prior to crystallization. Such methods have previously been used to crystallize other membrane proteins. Many of these methods, including the lipidic cubic phase crystallization method and the bicelle crystallization method, exploit the spontaneous self-assembling properties of lipids and detergent as vesicles (vesicle-fusion method), discoidal micelles (bicelle method), and liquid crystals or mesophases (in meso or cubic-phase method). Lipidic cubic phases crystallization methods are described in, for example: Landau et al. 1996; Gouaux 1998; Rummel et al. 1998; Nollert et al. 2004, Rasmussen et al. 2011, which publications are incorporated by reference for disclosure of those methods. Bicelle crystallization methods are described in, for example: Faham et al. 2005; Faham et al. 2002, which publications are incorporated by reference for disclosure of those methods.

The disclosure alos relates to the use of a conformation-selective binding agent as described herein to solve the structure of a muscarinic acetylcholine receptor in complex with a conformation-selective binding agent, and optionally in complex with another conformation-selective receptor ligand. "Solving the structure" as used herein refers to determining the arrangement of atoms or the atomic coordinates of a protein, and is often done by a biophysical method, such as X-ray crystallography.

In many cases, obtaining a diffraction-quality crystal is the key barrier to solving its atomic-resolution structure. Thus, the herein described conformation-selective binding agents can be used to improve the diffraction quality of the crystals so that the crystal structure of the receptor:binding agent complex can be solved.

In accordance, the present disclosure encompasses a method of determining the crystal structure of a muscarinic acetylcholine receptor, in particular M2R, in a functional conformation, the method comprising the steps of:
a) Providing a conformation-selective binding agent as described herein and muscarinic acetylcholine receptor M2, and optionally a receptor ligand, and
b) Allowing the formation of a complex of the binding agent, the muscarinic acetylcholine receptor M2 and optionally a receptor ligand,
c) crystallizing said complex of step b) to form a crystal
Said determining of the crystal structure may be done by a biophysical method such as X-ray crystallography. The method may further comprise a step for obtaining the atomic coordinates of the crystal (as defined hereinbefore).

### Ligand screening and drug discovery

Other applications are particularly envisaged that can make use of the conformation-selective binding agents described herein, including compound screening and immunizations, which will be described further herein.

In the process of compound screening, lead optimization and drug discovery (including antibody discovery), there is a requirement for faster, more effective, less expensive and especially information-rich screening assays that provide simultaneous information on various compound characteristics and their effects on various cellular pathways (i.e. efficacy, specificity, toxicity and drug metabolism). Thus, there is a need to quickly and inexpensively screen large numbers of compounds in order to identify new specific ligands of a protein of interest, preferably conformation-selective ligands, which may be potential new drug candidates. The present disclosure solves this problem by providing conformation-selective binding agents that stabilize or lock a muscarinic acetylcholine receptor, in particular M2R, in a functional conformation, preferably in an active conformation. This will allow to quick and reliably screen for and differentiate between receptor agonists, inverse agonists, antagonists and/or modulators as well as inhibitors of muscarinic acetylcholine receptors, so increasing the likelihood of identifying a ligand with the desired pharmacological properties. In particular, the conformation-selective binding agents, the complexes comprising the same, the host cells comprising the same, as well as host cell cultures or membrane preparations derived thereof are provided, for which specific preferences have been described hereinbefore, are particularly suitable for this purpose, and can then be used as immunogens or selection reagents for screening in a variety of contexts.

To illustrate this further, the conformation-selective binding agents described herein that recognize the active conformation of muscarinic acetylcholine receptor M2 will preferably be used in screening assays to screen for agonists because they increase the affinity of the receptor for agonists, relative to inverse agonists or antagonists. Reciprocally, binding agents that stabilize the inactive state conformation of muscarinic acetylcholine receptor M2 will increase the affinity for an inverse agonist, relative to agonists or antagonists. Such binding agents will preferably be used to screen for inverse agonists.

Thus, the present disclosure encompasses the use of the conformation-selective binding agents, complexes comprising the same, host cells comprising the same, host cell cultures, or membrane preparations derived thereof, as described hereinbefore, in screening and/or identification programs for conformation-selective binding partners of a muscarinic acetylcholine receptor, in particular M2R, which ultimately might lead to potential new drug candidates.

For example, the a method of identifying conformation-selective compounds is disclosed, the method comprising the steps of
(i) Providing a complex comprising a muscarinic acetylcholine receptor, in particular M2R, and a conformation-selective binding agent specifically binding to the receptor, and
(ii) Providing a test compound, and
(iii) Evaluating the selective binding of the test compound to the M2R comprised in the complex.

Specific preferences for the conformation-selective binding agents, complexes, host cells, host cell cultures and membrane preparations thereof are as defined above.

Preferably, the conformation-selective binding agent, the muscarinic acetylcholine receptor or the complex comprising the conformation-selective binding agent and the muscarinic acetylcholine receptor, as used in any of the screening methods described herein, are provided as whole cells, or cell (organelle) extracts such as membrane extracts or fractions thereof, or may be incorporated in lipid layers or vesicles (comprising natural and/or synthetic lipids), high-density lipoparticles, or any nanoparticle, such as nanodisks, or are provided as virus or virus-like particles (VLPs), so that sufficient functionality of the respective proteins is retained. Methods for preparations of GPCRs from membrane fragments or membrane-detergent extracts are reviewed in detail in Cooper (2004). Alternatively, the receptor and/or the complex may also be solubilized in detergents. Non-limiting examples of solubilized receptor preparations are also provided in the Example section.

Screening assays for drug discovery can be solid phase (e.g. beads, columns, slides, chips or plates) or solution phase assays, e.g. a binding assay, such as radioligand binding assays. In high-throughput assays, it is possible to screen up to several thousand different compounds in a single day in 96-, 384- or 1536-well formats. For example, each well of a microtiter plate can be used to run a separate assay against a selected potential modulator, or, if concentration or incubation time effects are to be observed, every 5-10 wells can test a single modulator. Thus, a single standard microtiter plate can assay about 96 modulators. It is possible to assay many plates per day; assay screens for up to about 6.000, 20.000, 50.000 or more different compounds are possible today. Preferably, a screening for muscarinic receptor conformation-selective compounds will be performed starting from host cells, or host cell cultures, or membrane preparations derived thereof.

Various methods may be used to determine binding between the stabilized muscarinic receptor and a test compound, including for example, flow cytometry, radioligand binding assays, enzyme linked immunosorbent assays (ELISA), surface Plasmon resonance assays, chip-based assays, immunocytofluorescence, yeast two-hybrid technology and phage display which are common practice in the art, for example, in Sambrook et al. (2001), Molecular Cloning, A Laboratory Manual. Third Edition. Cold Spring Harbor Laboratory Press, Cold Spring Harbor, NY. Other methods of detecting binding between a test compound and a membrane protein include ultrafiltration with ion spray mass spectroscopy/HPLC methods or other (bio)physical and analytical methods. Fluorescence Energy Resonance Transfer (FRET) methods, for example, well known to those skilled in the art, may also be used. It will be appreciated that a bound test compound can be detected using a unique label or tag associated with the compound, such as a peptide label, a nucleic acid label, a chemical label, a fluorescent label, or a radio isotope label, as described further herein.

The above described method of identifying conformation-selective compounds may be performed by a ligand binding assay or competition assay, even more preferably a radioligand binding or competition assay. Most preferably, the above described method of identifying conformation-selective compounds may be performed in a comparative assay, more specifically, a comparative ligand competition assay, even more specifically a comparative radioligand competition assay.

In case the above described method is performed in a comparative assay, it will be understood that the method will comprise the step of comparing the binding of a test compound for M2R is stabilized by a conformation-selective binding agent in a functional conformation of interest, preferably an active conformation, with the binding of the test compound to a control. Within the scope of the disclosure, the control can be the corresponding M2R in the absence of a conformation-selective binding agent or in the presence of a mock binding agent (also referred to as control binding agent or irrelevant binding moiety), which is a binding agent that is not directed to and/or does not specifically bind to the corresponding M2R.

The step of evaluating the selective binding of the test compound to the receptor in any of the above described methods may be done by measuring and/or calculating the affinity (as defined herein) of the test compound for the receptor.

Often high-throughput screening for conformation-selective binding partners of receptors will be preferred. This may be facilitated by immobilization of a either the conformation-selective binding agent described herein, the muscarinic acetylcholine receptor or the complex comprising the conformation-selective binding agent and the muscarinic acetylcholine receptor, onto a suitable solid surface or support that can be arrayed or otherwise multiplexed. Non-limiting examples of suitable solid supports include beads, columns, slides, chips or plates.

More particularly, the solid supports may be particulate (e. g. beads or granules, generally used in extraction columns) or in sheet form (e. g. membranes or filters, glass or plastic slides, microtitre assay plates, dipstick, capillary fill devices or such like) which can be flat, pleated, or hollow fibres or tubes. The following matrices are given as examples and are not exhaustive, such examples could include silica (porous amorphous silica), i. e. the FLASH series of cartridges containing 60A irregular silica (32-63 um or 35-70 um) supplied by Biotage (a division of Dyax Corp.), agarose or polyacrylamide supports, for example the Sepharose range of products supplied by Amersham Pharmacia Biotech, or the Affi-Gel supports supplied by Bio-Rad. In addition there are macroporous polymers, such as the pressure-stable Affi-Prep supports as supplied by Bio-Rad. Other supports that could be utilised include; dextran, collagen, polystyrene, methacrylate, calcium alginate, controlled pore glass, aluminium, titanium and porous ceramics. Alternatively, the solid surface may comprise part of a mass dependent sensor, for example, a surface plasmon resonance detector. Further examples of commercially available supports are discussed in, for example, Protein Immobilisation, R.F. Taylor ed.., Marcel Dekker, Inc., New York, (1991).

Immobilization may be either non-covalent or covalent. In particular, non-covalent immobilization or adsorption on a solid surface of the conformation-selective binding agent, the muscarinic acetylcholine receptor or the complex comprising the conformation-selective binding agent and the muscarinic acetylcholine receptor, may occur via a surface coating with any of an antibody, or streptavidin or avidin, or a metal ion, recognizing a molecular tag attached to the binding agent, according to standard techniques known by the skilled person (e.g. biotin tag, Histidine tag, etc.).

In particular, the conformation-selective binding agent, the muscarinic acetylcholine receptor or the complex comprising the conformation-selective binding agent and the muscarinic acetylcholine receptor, may be attached to a solid surface by covalent cross-linking using conventional coupling chemistries. A solid surface may naturally comprise cross-linkable residues suitable for covalent attachment or it may be coated or derivatised to introduce suitable cross-linkable groups according to methods well known in the art.Sufficient functionality of the immobilised protein may be retained following direct covalent coupling to the desired matrix via a reactive moiety that does not contain a chemical spacer arm. Further examples and more detailed information on immobilization methods of antibody (fragments) on solid supports are discussed in Jung et al. (2008); similarly, membrane receptor immobilization methods are reviewed in Cooper (2004); both herein incorporated by reference.

Advances in molecular biology, particularly through site-directed mutagenesis, enable the mutation of specific amino acid residues in a protein sequence. The mutation of a particular amino acid (in a protein with known or inferred structure) to a lysine or cysteine (or other desired amino acid) can provide a specific site for covalent coupling, for example. It is also possible to reengineer a specific protein to alter the distribution of surface available amino acids involved in the chemical coupling (Kallwass et al, 1993), in effect controlling the orientation of the coupled protein. A similar approach can be applied to the conformation-selective binding agents described herein, as well as to the conformationally-stabilized muscarinic receptors, whether or not comprised in the complex, so providing a means of oriented immobilisation without the addition of other peptide tails or domains containing either natural or unnatural amino acids. In case of an antibody or an antibody fragment, such as a Nanobody, introduction of mutations in the framework region is preferred, minimising disruption to the antigen-binding activity of the antibody (fragment).

Conveniently, the immobilised proteins may be used in immunoadsorption processes such as immunoassays, for example ELISA, or immunoaffinity purification processes by contacting the immobilised proteins with a test sample according to standard methods conventional in the art. Alternatively, and particularly for high-throughput purposes, the immobilized proteins can be arrayed or otherwise multiplexed. Preferably, the immobilised proteins described herein are used for the screening and selection of compounds that selectively bind to a particular conformation of a muscarinic receptor, particularly M2R.

It will be appreciated that either the conformation-selective binding agent or the target muscarinic receptor may be immobilized, depending on the type of application or the type of screening that needs to be done. Also, the choice of the conformation-selective binding agent (targeting a particular conformational epitope of the receptor), will determine the orientation of the receptor and accordingly, the desired outcome of the compound identification, e.g. compounds specifically binding to extracellular parts, intramembranal parts or intracelllular parts of said conformationally stabilized receptor.

Alternatively, the test compound (or a library of test compounds) may be immobilized on a solid surface, such as a chip surface, whereas the conformation-selective binding agent and muscarinic receptor are provided, for example, in a detergent solution or in a membrane-like preparation.

Accordingly, a solid support to which is immobilized a conformation-selective binding agent as described herein is provided herein for use in any of the above screening methods.

Most preferably, neither the conformation-selective binding agent, nor the muscarinic receptor, nor the test compound are immobilized, for example in phage-display selection protocols in solution, or radioligand binding assays.

The compounds to be tested can be any small chemical compound, or a macromolecule, such as a protein, a sugar, nucleic acid or lipid. Typically, test compounds will be small chemical compounds, peptides, antibodies or fragments thereof. It will be appreciated that in some instances the test compound may be a library of test compounds. In particular, high-throughput screening assays for therapeutic compounds such as agonists, antagonists or inverse agonists and/or modulators form part of the disclosure. For high-throughput purposes, compound libraries or combinatorial libraries may be used such as allosteric compound libraries, peptide libraries, antibody libraries, fragment-based libraries, synthetic compound libraries, natural compound libraries, phage-display libraries and the like. Methodologies for preparing and screening such libraries are known to those of skill in the art.

The test compound may optionally be covalently or non-covalently linked to a detectable label. Suitable detectable labels and techniques for attaching, using and detecting them will be clear to the skilled person, and include, but are not limited to, any composition detectable by spectroscopic, photochemical, biochemical, immunochemical, electrical, optical or chemical means. Useful labels include magnetic beads (e.g. dynabeads), fluorescent dyes (e.g. all Alexa Fluor dyes, fluorescein isothiocyanate, Texas red, rhodamine, green fluorescent protein and the like), radiolabels (e.g. ³H, ¹²⁵I, ³⁵S, ¹⁴C, or ³²P), enzymes (e.g. horse radish peroxidase, alkaline phosphatase), and colorimetric labels such as colloidal gold or colored glass or plastic (e.g. polystyrene, polypropylene, latex, etc.) beads. Means of detecting such labels are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with a substrate and detecting the reaction product produced by the action of the enzyme on the substrate, and colorimetric labels are detected by simply visualizing the colored label. Other suitable detectable labels were described earlier.

Thus, the test compound as used in any of the above screening methods may be selected from the group comprising a polypeptide, a peptide, a small molecule, a natural product, a peptidomimetic, a nucleic acid, a lipid, lipopeptide, a carbohydrate, an antibody or any fragment derived thereof, such as Fab, Fab' and F(ab')2, Fd, single-chain Fvs (scFv), single-chain antibodies, disulfide-linked Fvs (dsFv) and fragments comprising either a VL or VH domain, a heavy chain antibody (hcAb), a single domain antibody (sdAb), a minibody, the variable domain derived from camelid heavy chain antibodies (VHH or Nanobody), the variable domain of the new antigen receptors derived from shark antibodies (VNAR), a protein scaffold including an alphabody, protein A, protein G, designed ankyrin-repeat domains (DARPins), fibronectin type III repeats, anticalins, knottins, engineered CH2 domains (nanoantibodies), as defined hereinbefore.

For example, high throughput screening methods may involve providing a combinatorial chemical or peptide library containing a large number of potential therapeutic ligands. Such "combinatorial libraries" or "compound libraries" are then screened in one or more assays, as described herein, to identify those library members (particular chemical species or subclasses) that display a desired characteristic activity. A "compound library" is a collection of stored chemicals usually used ultimately in high-throughput screening A "combinatorial library" is a collection of diverse chemical compounds generated by either chemical synthesis or biological synthesis, by combining a number of chemical "building blocks" such as reagents. Preparation and screening of combinatorial libraries are well known to those of skill in the art. The compounds thus identified can serve as conventional "lead compounds" or can themselves be used as potential or actual therapeutics. Thus, the screening methods as described herein above may further comprise modifying a test compound which has been shown to selectively bind to a muscarinic receptor in a particular conformation, and determining whether the modified test compound binds to the receptor when residing in the particular conformation.

It may be determined whether the compound alters the binding of the muscarinic receptor to a receptor ligand (as defined herein). Binding of a receptor to its ligand can be assayed using standard ligand binding methods known in the art as described herein. For example, a ligand may be radiolabelled or fluorescently labeled. The assay may be carried out on whole cells or on membranes obtained from the cells or aqueous solubilized receptor with a detergent. The compound will be characterized by its ability to alter the binding of the labeled ligand (see also Example section). The compound may decrease the binding between the receptor and its ligand, or may increase the binding between the receptor and its ligand, for example by a factor of at least 2 fold, 3 fold, 4 fold, 5 fold, 10 fold, 20 fold, 30 fold, 50 fold, 100 fold.

Thus, a complex comprising a conformation-selective binding agent as described herein, a muscarinic receptor and a receptor ligand may be used in any of the above screening methods. Preferably, the receptor ligand is chosen from the group comprising a small molecule, a polypeptide, an antibody or any fragment derived thereof, a natural product, and the like. More preferably, the receptor ligand is a full agonist, or a partial agonist, a biased agonist, an antagonist, or an inverse agonist, as described hereinbefore.

The test compound as used in any of the above screening methods may be provided as a biological sample. In particular, the sample can be any suitable sample taken from an individual. For example, the sample may be a body fluid sample such as blood, serum, plasma, spinal fluid.

In addition to establishing binding to a muscarinic receptor, in particular M2R, in a particular conformation of interest, it will also be desirable to determine the functional effect of a compound on the receptor. For example, the compounds may bind to the muscarinic receptor resulting in the modulation (activation or inhibition) of the biological function of the receptor, in particular the downstream receptor signaling. This modulation of intracellular signaling can occur ortho- or allosterically. The compounds may bind to the muscarinic receptor so as to activate or increase receptor signaling; or alternatively so as to decrease or inhibit receptor signaling. The compounds may also bind to the muscarinic receptor in such a way that they block off the constitutive activity of the receptor. The compounds may also bind to the muscarinic receptor in such a way that they mediate allosteric modulation (e.g. bind to the receptor at an allosteric site). In this way, the compounds may modulate the receptor function by binding to different regions in the receptor (e.g. at allosteric sites). Reference is for example made to George et al. 2002; Kenakin 2002; Rios et al. 2001. The compounds may also bind to the muscarinic receptor in such a way that they prolong the duration of the receptor-mediated signaling or that they enhance receptor signaling by increasing receptor-ligand affinity. Further, the compounds may also bind to the muscarinic receptor in such a way that they inhibit or enhance the assembly of receptor functional homomers or heteromers. The efficacy of the compounds and/or compositions comprising the same, can be tested using any suitable in vitro assay, cell-based assay, in vivo assay and/or animal model known per se, or any combination thereof, depending on the specific disease or disorder involved.

It will be appreciated that the conformation-selective binding agents, complexes, host cells and derivatives thereof, as described herein, may be further engineered and are thus particularly useful tools for the development or improvement of cell-based assays. Cell-based assays are critical for assessing the mechanism of action of new biological targets and biological activity of chemical compounds. For example, without the purpose of being limitative, current cell-based assays for GPCRs include measures of pathway activation (Ca²⁺ release, cAMP generation or transcriptional activity); measurements of protein trafficking by tagging GPCRs and downstream elements with GFP; and direct measures of interactions between proteins using Forster resonance energy transfer (FRET), bioluminescence resonance energy transfer (BRET) or yeast two-hybrid approaches.

Further, it may be particularly advantageous to immunize an animal with a complex comprising a muscarinic receptor, particularly M2R, and a conformation-selective binding agent that is directed against and/or specifically binds to said receptor, or with a host cell comprising said complex, or derivative thereof, in order to raise antibodies, preferably conformation-selective antibodies against the muscarinic receptor. Thus, such immunization methods are also envisaged here. Methods for raising antibodies *in vivo* are known in the art, and are also described hereinbefore. Any suitable animal, e.g., a warm-blooded animal, in particular a mammal such as a rabbit, mouse, rat, camel, sheep, cow, shark, or pig or a bird such as a chicken or turkey, may be immunized using any of the techniques well known in the art suitable for generating an immune response. Following immunization, expression libraries encoding immunoglobulin genes, or portions thereof, expressed in bacteria, yeast, filamentous phages, ribosomes or ribosomal subunits or other display systems, can be made according to well-known techniques in the art. Further to that, the antibody libraries that are generated comprise a collection of suitable test compounds for use in any of the screening methods as described hereinbefore. The antibodies that have been raised as described herein above may also be useful diagnostic tools to specifically detect muscarinic receptors in a particular conformation, and thus also form part of the present disclosure.

The complex comprising the muscarinic receptor, in particular M2R, and the conformation-selective binding agent that is directed against and/or specifically binds to the muscarinic receptor may be used for the selection of conformation-selective binding agents including antibodies or antibody fragments that bind the receptor by any of the screening methods as described above. Persons of ordinary skill in the art will recognize that such binding agents, as a non-limiting example, can be selected by screening a set, collection or library of cells that express binding agents on their surface, or bacteriophages that display a fusion of genlll and binding agent at their surface, or yeast cells that display a fusion of the mating factor protein Aga2p, or by ribosome display amongst others.

### Therapeutic and diagnostic applications

A further aspect relates to a pharmaceutical composition comprising a therapeutically effective amount of a conformation-selective binding agent as described herein and at least one of a pharmaceutically acceptable carrier, adjuvant or diluents.

A 'carrier', or 'adjuvant', in particular a 'pharmaceutically acceptable carrier' or 'pharmaceutically acceptable adjuvant' is any suitable excipient, diluent, carrier and/or adjuvant which, by themselves, do not induce the production of antibodies harmful to the individual receiving the composition nor do they elicit protection. So, pharmaceutically acceptable carriers are inherently non-toxic and nontherapeutic, and they are known to the person skilled in the art. Suitable carriers or adjuvantia typically comprise one or more of the compounds included in the following non- exhaustive list: large slowly metabolized macromolecules such as proteins, polysaccharides, polylactic acids, polyglycolic acids, polymeric amino acids, amino acid copolymers and inactive virus particles. Carriers or adjuvants may be, as a non-limiting example, Ringer's solution, dextrose solution or Hank's solution. Non aqueous solutions such as fixed oils and ethyl oleate may also be used. A preferred excipient is 5% dextrose in saline. The excipient may contain minor amounts of additives such as substances that enhance isotonicity and chemical stability, including buffers and preservatives.

The administration of a conformation-selective binding agent as described herein or a pharmaceutical composition thereof may be by way of oral, inhaled or parenteral administration. The binding agent may be delivered through intrathecal or intracerebroventricular administration. The active compound may be administered alone or preferably formulated as a pharmaceutical composition. An amount effective to treat a certain disease or disorder that express the antigen recognized by the protein binding domain depends on the usual factors such as the nature and severity of the disorder being treated and the weight of the mammal. However, a unit dose will normally be in the range of 0.1 mg to 1 g, for example, to 0.1 to 500 mg, for example 0.1 to 50 mg, or 0.1 to 2 mg of protein binding domain or a pharmaceutical composition thereof. Unit doses will normally be administered once a month, once a week, bi-weekly, once or more than once a day, for example 2, 3, or 4 times a day, more usually 1 to 3 times a day. It is greatly preferred that the binding agent or a pharmaceutical composition thereof is administered in the form of a unit-dose composition, such as a unit dose oral, parenteral, or inhaled composition. Such compositions are prepared by admixture and are suitably adapted for oral, inhaled or parenteral administration, and as such may be in the form of tablets, capsules, oral liquid preparations, powders, granules, lozenges, reconstitutable powders, injectable and infusable solutions or suspensions or suppositories or aerosols. Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art. Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate. These solid oral compositions may be prepared by conventional methods of blending, filling, tabletting or the like. Repeated blending operations may be used to distribute the active agent throughout those compositions employing large quantities of fillers. Such operations are, of course, conventional in the art. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents. Oral formulations also include conventional sustained release formulations, such as tablets or granules having an enteric coating. Preferably, compositions for inhalation are presented for administration to the respiratory tract as a snuff or an aerosol or solution for a nebulizer, or as a microfine powder for insuflation, alone or in combination with an inert carrier such as lactose. In such a case the particles of active compound suitably have diameters of less than 50 microns, preferably less than 10 microns, for example between 1 and 5 microns, such as between 2 and 5 microns. A favored inhaled dose will be in the range of 0.05 to 2 mg, for example 0.05 to 0.5 mg, 0.1 to 1 mg or 0.5 to 2 mg. For parenteral administration, fluid unit dose forms are prepared containing a compound of the present disclosure and a sterile vehicle. The active compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound. Where appropriate, small amounts of bronchodilators for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included. As is common practice, the compositions will usually be accompanied by written or printed directions for use in the medical treatment concerned.

In the case of a biological, delivery of conformation-selective binding agents into cells may be performed as described for peptides, polypeptides and proteins. If the antigen is extracellular or an extracellular domain, the binding agent may exert its function by binding to this domain, without need for intracellular delivery. The binding agents as described herein may target intracellular conformational epitopes of the muscarinic receptor. To use these binding agents as effective and safe therapeutics inside a cell, intracellular delivery may be enhanced by protein transduction or delivery systems know in the art. Protein transduction domains (PTDs) have attracted considerable interest in the drug delivery field for their ability to translocate across biological membranes. The PTDs are relatively short (1-35 amino acid) sequences that confer this apparent translocation activity to proteins and other macromolecular cargo to which they are conjugated, complexed or fused (Sawant and Torchilin 2010). The HIV-derived TAT peptide (YGRKKRRQRRR), for example, has been used widely for intracellular delivery of various agents ranging from small molecules to proteins, peptides, range of pharmaceutical nanocarriers and imaging agents. Alternatively, receptor-mediated endocytic mechanisms can also be used for intracellular drug delivery. For example, the transferrin receptor-mediated internalization pathway is an efficient cellular uptake pathway that has been exploited for site-specific delivery of drugs and proteins (Qian et al. 2002). This is achieved either chemically by conjugation of transferrin with therapeutic drugs or proteins or genetically by infusion of therapeutic peptides or proteins into the structure of transferrin. Naturally existing proteins (such as the iron-binding protein transferrin) are very useful in this area of drug targeting since these proteins are biodegradable, nontoxic, and non-immunogenic. Moreover, they can achieve site-specific targeting due to the high amounts of their receptors present on the cell surface. Still other delivery systems include, without the purpose of being limitative, polymer- and liposome-based delivery systems.

The efficacy of the conformation-selective binding agents described herein, and of compositions comprising the same, can be tested using any suitable in vitro assay, cell-based assay, in vivo assay and/or animal model known per se, or any combination thereof, depending on the specific disease or disorder involved.

Another aspect of the disclosure relates to the use of the conformation-selective binding agent or the pharmaceutical composition as described hereinbefore to modulate M2R signaling activity.

The conformation-selective binding agents as described herein may bind to the muscarinic receptor so as to activate or increase receptor signaling; or alternatively so as to decrease or inhibit receptor signaling. The binding agents described herein may also bind to the receptor in such a way that they block off the constitutive activity of the receptor. The binding agents described herein may also bind to the receptor in such a way that they mediate allosteric modulation (e.g. bind to the receptor at an allosteric site). In this way, the binding agents of described herein may modulate the receptor function by binding to different regions in the receptor (e.g. at allosteric sites). Reference is for example made to George et al. (2002), Kenakin (2002) and Rios et al. (2001). The binding agents described herein may also bind to the receptor in such a way that they prolong the duration of the receptor-mediated signaling or that they enhance receptor signaling by increasing receptor-ligand affinity. Further, the binding agents described herein may also bind to the receptor in such a way that they inhibit or enhance the assembly of receptor functional homomers or heteromers.

For example, the conformation-selective binding agent or the pharmaceutical composition as described hereinbefore may block G-protein mediated signaling.

The disclosure also envisages the conformation-selective binding agent or the pharmaceutical composition as described hereinbefore for use in the treatment of a muscarinic receptor-related disease, in particular an M2R-related disease.

It will thus be understood that certain of the above-described conformation-selective binding agents may have therapeutic utility and may be administered to a subject having a condition in order to treat the subject for the condition. The therapeutic utility for a conformation-selective binding agent may be determined by the muscarinic receptor to which the binding agent binds in that signaling via that receptor is linked to the condition. A conformation-selective binding agent may be employed for the treatment of a muscarinic receptor-mediated condition, in particular an M2R-mediated condition, such as Alzheimer's disease and cognitive impairments, pain, IBD, gliomablastoma, amongst others. Further exemplary muscarinic receptor-related conditions at the On-line Mendelian Inheritance in Man database found at the world wide website of the NCBI. So, the use of a conformation-selective biding agent or of a pharmaceutical composition for the treatment of a muscarinic receptor-related disease or disorder, in particular an M2R-related disease or disorder is also provided.

The conformation-selective binding agents may be employed as co-therapeutic agents for use in combination with other drug substances, for example as potentiators of therapeutic activity of such drugs or as a means of reducing required dosaging or potential side effects of such drugs. A conformation-selective binding agent may be mixed with the other drug substance in a fixed pharmaceutical composition or it may be administered separately, before, simultaneously with or after the other drug substance. In general terms, these protocols involve administering to an individual suffering from a muscarinic receptor-related disease or disorder, in particular an M2R-related disease or disorder, an effective amount of a conformation-selective binding agent that modulates a muscarinic receptor, in particular M2R, to modulate the receptor in the host and treat the individual for the disorder.

Where a reduction in activity of a muscarinic receptor, particularly M2R, is desired, one or more compounds that decrease the activity of the receptor may be administered, whereas when an increase in activity of a muscarinic receptor, particularly M2R, is desired, one or more compounds that increase the activity of the receptor activity may be administered.

A variety of individuals are treatable according to the subject methods. Generally such individuals are mammals or mammalian, where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In many examples, the individuals will be humans. Subject treatment methods are typically performed on individuals with such disorders or on individuals with a desire to avoid such disorders.

The conformation-selective binding agents may also be useful for the diagnosis or prognosis of a muscarinic receptor-related disease, in particular an M2R-related disease or disorder, as described hereinbefore.

### Kit of parts

Still another aspect relates to a kit comprising a conformation-selective binding agent targeting a muscarinic receptor, in particular M2R, or a kit comprising a host cell or a host cell culture or a membrane preparation comprising a conformation-selective binding agent targeting a muscarinic receptor as described herein. The kit may further comprise a combination of reagents such as buffers, molecular tags, vector constructs, reference sample material, as well as a suitable solid supports, and the like. Such a kit may be useful for any of the applications as described herein. For example, the kit may comprise (a library of) test compounds useful for compound screening applications.

### EXAMPLES

### Methods to the Examples

**M2 muscarinic receptor expression and purification.** The human M2 muscarinic receptor gene was modified to remove glycosylation sites, and to add an amino-terminal FLAG tag and a carboxy-terminal 8xHis tag. In addition, residues 233 - 374 of intracellular loop 3 were deleted. This region has previously been shown to be unstructured (Ichiyama et al. 2006) and is not essential for G protein coupling (Shapiro et al. 1989). Human M2 muscarinic receptor bearing an amino-terminal FLAG epitope tag and carboxy-terminal 8xHis tag was expressed in *Sf9* cells using the BestBac baculovirus system (Expression Systems; Davis, CA). Cells were infected at a density of 4 x 10⁶ cells/mL, then incubated for two days at 27 °C. Receptor was extracted and purified in the manner described previously for the M3 muscarinic receptor (Kruse et al. 2012). Briefly, receptor was first purified by Ni-NTA chromatography, FLAG affinity chromatography, then size exclusion chromatography. 1 µM atropine was included in all buffers. Receptor was then labeled with a 5-fold molar excess of biotin-NHS ester (Sigma-Aldrich; St. Louis, MO) in buffer containing 25 mM HEPES pH 7.2. Following a 30 minute incubation at room temperature and a 30 minute incubation on ice, unreacted label was quenched with 50 mM Tris pH 8. Directly labeled samples with fluorophore-NHS esters were prepared similarly. Receptor was then desalted into buffer containing either 10 µM tiotropium, 10 µM iperoxo, or buffer containing no ligand. Receptor eluted in buffer containing no ligand was treated with 50 µM iperoxo mustard (derivative of iperoxo, that allows covalent binding to M2 receptor) for 20 minutes at room temperature. Samples were then concentrated, aliquoted, and flash frozen with 20% (v/v) glycerol.

**Llama immunization samples.** M2 receptor was prepared as described above, and bound to iperoxo by including it at a 10µM starting at FLAG wash steps and in all subsequent buffers. Receptor was reconstituted into phospholipid vesicles composed of DOPC (1,2-dioleoyl-sn-glycero-3-phosphocholine, Avanti Polar Lipids) and Lipid A in a 10:1 (w:w) ratio, then aliquoted at 1 mg/mL receptor concentration and frozen in 100 µL aliquots prior to injection.

**Llama immunization.** One llama (Lama glama) was immunized for six weeks with 1 mg receptor in total. Peripheral blood lymphocytes were isolated from the immunized animal to extract total RNA. cDNA was prepared using 50 µg of total RNA and 2.5 µg of oligo-dN6primer. Nanobody open reading frames were amplified as described (Conrath et al. 2001).

**Post-immune M2 llama nanobody yeast library construction.** Starting from a PCR nanobody library, nanobody Nanobody VHH fragments were amplified by PCR using the primers pYalNB80AMPF (CATTTTCAATTAAGATGCAG TTACTTCGCT GTTTTTCAAT ATTTTCTGTT ATTGCTAGCG TTTTAGCAAT GGCCCAGGTG CAGCTGCAGG AG; SEQ ID NO: 165) and pYalNB80AMPR 5CCACCAGATC CACCACCACC CAAGGTCTTCT TCGGAGATAA GCTTTTGTTC GGATCCTGAG GAGACGGTGA CCTGGGTCCC; SEQ ID NO: 166). The PCR products were then cotransformed with linearized pYal into yeast strain EBY100, yielding a library size of 0.6x10⁸ transformants.

**Selections of M2 Gi-mimetic nanobodies from post-immune M2 llama nanobody library.** For the first round of selection, counter-selection was performed against the β2 adrenergic receptor to remove yeast-clones that bind non-specifically to membrane proteins or to secondary staining reagents. 1.0x10⁹ of induced yeast were washed with PBEM buffer and then stained in 5 mL of PBEM buffer containing 1 µM biotinylated β2 receptor liganded with carazolol for one hour at 4° C. Yeast were washed with PBEM buffer and then stained with streptavidin-647 as a secondary reagent in PBEM buffer for 15 minutes at 4°C. Yeast were washed again with PBEM buffer and magnetically-labeled with anti-647 microbeads (Miltenyi) in 4.75 mL PBEM buffer for 15 minutes at 4°C. Positively labeled yeast, cells that bind the β2 receptor, were then removed by application to an LD column (Miltenyi); the cleared flow-through was then used for subsequent selection. Positive selection for clones recognizing the active-state of the M2 receptor was performed by staining the yeast with 2 µM biotinylated M2 receptor liganded with the agonist iperoxo in 5 mL PBEM buffer supplemented with 2 µM iperoxo for one hour at 4°C. Yeast were then washed, stained with streptavidin-647, and magnetically-labeled with anti-647 microbeads, including 1 µM iperoxo in the PBEM buffer at all steps. Magnetic separation of M2 receptor binding yeast clones was performed using an LS column (Miltenyi) following the manufacturer's instructions. Magnetically sorted yeast were resuspended in SDCAA medium and cultured at 30°C. Rounds 2-4 were selected in a similar manner, counter-selecting against 1 µM biotinylated β2 receptor + carazolol and positively selecting using 1 µM biotinylated M2 receptor + iperoxo. For these rounds, the scale was reduced ten-fold to 1 x 10⁸ induced yeast and staining volumes of 0.5 mL.

Conformational selection was performed for rounds 5-9. For rounds 5-8, yeast were stained with 1 µM biotinylated M2 receptor pre-incubated with the high-affinity antagonist tiotropium for one hour at 4°C. Yeast were then fluorescently labeled with either streptavidin-647 or streptavidin-PE, and magnetically labeled with the corresponding anti-647 or anti-PE microbeads (Miltenyi). Only yeast cells binding the tiotropium loaded M2 receptor are labeled and depletion of inactive-state binders was carried out using an LS column. The cleared yeast were then positively selected by staining with 0.5 µM (rounds 5-7) or 0.1 µM (round 8) biotinylated M2 receptor pre-bound to iperoxo for one hour at 4° C. Yeast were then fluorescently-labeled with either streptavidin-PE or streptavidin-647, using the fluorophore distinct from counter-selection in the previous step. Yeast cells binding the iperoxo loaded M2 receptor are labeled and magnetic separation of agonist-occupied M2 receptor was performed using an LS column, as for steps 1-4. For round 9, two-color FACS was performed. Induced yeast were simultaneously stained with 1 µM Alexa647-labeled M2 receptor reacted with iperoxo mustard and 1 µM Alexa488-labeled M2 receptor pre-bound with tiotropium for one hour at 4°C. Alexa647 positive/Alexa488 negative yeast were purified using a FACS Jazz cell (BD Biosciences) sorter. Post-sorted yeast were plated onto SDCAA-agar plates and the nanobody-encoding sequences of several colonies were sequenced. Full sequences of clones confirmed to enhance agonist affinity are Nb9-1 (SEQ ID NO: 8), Nb9-8 (SEQ ID NO: 10) and Nb9-20 (SEQ ID NO: 11).

### Selections of functional nanobodies with M2 Gi mimetic nanobody Nb9-8.

Selections were initiated with the yeast remaining after the first four rounds of selection for the M2 Gi mimetic nanobody prior to conformational selection. For rounds 5 & 6, yeast were precleared using MACS against 500 nM PE-labeled streptavidin tetramers conjugated to biotinylated Nb9-8, removing clones that bind Nb9-8 directly. Tetramers were formed by preincubating 2 µM biotinylated Nb9-8 with 0.5 µM streptavidin-PE in PBEM buffer on ice for 10 minutes. The yeast were then positively selected with 500 nM streptavidin-PE/Nb9-8 tetramers after first staining the yeast with 1 µM Alexa488-labeled M2 receptor reacted with iperoxo mustard. Magnetic separation with MACS was accomplished using anti-PE microbeads and an LS column. To further select for clones binding at extracellular, allosteric/orthosteric site of the M2 receptor, for rounds 7 and 8 counter-selection was performed against 1 µM biotinylated M2 receptor occupied with iperoxo in the presence of 2mM of the allosteric/orthosteric ligand gallamine. Positive selection for M2 receptor in the absence of gallamine was then performed using 1 µM biotinylated M2 receptor occupied with iperoxo and MACS for round 7 and 1 µM Alexa488-labeled M2 receptor reacted with iperoxo mustard and FACS for round 8.

**Expression of MBP-nanobody fusions in *E. coli.*** Nanobody sequences were subcloned into a modified pMalp2x vector (New England Biolabs), containing an aminoterminal, 3C protease-cleavable maltose binding protein (MBP) tag and a carboxy-terminal 8xHistidine tag. Plasmids were transformed into BL21(DE3) cells and protein expression induced in Terrific Broth by addition of IPTG to 1 mM at an OD600 of 0.8. After 24 hours of incubation at 22°C, cells were harvested and periplasmic protein obtained by osmotic shock. MBP-nanobody fusions were purified by Ni-NTA chromatography and MBP was removed using 3C protease. Cleaved MBP was separated from the 8xHis tagged nanobodies by an additional Ni-NTA purification step. The 8xHis tag was subsequently removed using carboxypeptidase A. To obtain biotinylated nanobodies, proteins were expressed with a carboxy-terminal biotin acceptor peptide tag (GLNDIFEAQKIEWHE) and purified as described above. The purified proteins were biotinylated *in vitro* with BirA ligase and then repurified from the reaction mixture by size exclusion chromatography.

**Expression and purification of G protein.** Heterotrimeric Gᵢ was prepared by expression using a single baculovirus for the human Gαᵢ₁ subunit and a second, bicistronic virus for human G_{β1} and G_{γ2} subunits. G protein was expressed in HighFive insect cells, and then purified as described previously for Gₛ (Rasmussen et al. 2011). In brief, G protein was extracted with cholate, purified by Ni-NTA chromatography, detergent exchanged into dodecyl maltoside buffer, and then purified by ion exchange and dialyzed prior to use.

**M2 receptor radioligand binding assays.** M2 receptor was expressed and purified as described above. Receptor was then reconstituted into HDL particles consisting of apolipoprotein A1 and a 3:2 (mol:mol) mixture of the lipids POPC:POPG (1-palmitoyl-2-oleoyl-sn-glycero-3-phosphocholine: 1-palmitoyl-2-oleoyl-sn-glycero-3-phosphocholine and 1-hexadecanoyl-2-(9Z-octadecenoyl)-sn-glycero-3-phospho-(1'-rac-glycerol) respectively, Avanti Polar Lipids). Binding reactions contained 50 fmol functional receptor, 0.6 nM 3H N-methyl scopolamine (NMS), 100 mM NaCl, 20 mM HEPES pH 7.5, 0.1% BSA, and ligands and nanobodies as indicated. Single point allosteric effects of nanobodies were measured in the presence of 10 nM iperoxo. Concentration-dependent effects of nanobodies were measured in the presence of 10nM iperoxo. All reactions were 500 µL in volume. Reactions were mixed and then incubated for two hours. Samples were then filtered on a 48-well harvester (Brandel) onto a filter which had been pretreated with 0.1% polyethylenimine. All measurements were taken by liquid scintillation counting, and experiments were performed at least in triplicate.

**Crystallization samples.** M2 receptor for crystallization was prepared as described above. When bound to FLAG resin, the sample was washed with a mix of dodecyl maltoside buffer (DDM) and buffer containing 0.2% lauryl maltose neopentyl glycol detergent (MNG; Anatrace). These buffers were mixed first in a 1:1 ratio (DDM:MNG buffer), then 1:4, and 1:10 ratios. At each step the 5 mL column was washed with 10 mL of buffer at a 1 mL/min flow rate, and all buffers contained 1 µM atropine. Finally, the column was washed with 10 mL MNG buffer, and then 10 mL of low detergent buffer with agonist (0.01 % MNG, 0.001 % cholesterol hemisuccinate, 20 mM HEPES pH 7.5, 100 mM NaCl, 10 IlM iperoxo). The sample was eluted, mixed with a 1.5-fold stoichiometric excess of Nb9-8 and a second nanobody, NbB4. This nanobody binds to an epitope different from Nb9-8, but was not resolved in the crystal structure. Following mixing, the sample was incubated 30 min on ice, then concentrated and purified by size exclusion in low detergent buffer. Eluted protein was concentrated to A₂₈₀ = 96, and frozen in liquid nitrogen in 7 µL aliquots.

**Crystallization.** Purified M2 receptor was reconstituted into lipidic cubic phase by mixing with a 1.5 fold excess by mass of 10:1 (w:w) monoolein cholesterol lipid mix. Protein and lipid were loaded into glass syringes (Art Robbins Instruments, Sunnyvale, CA), and then mixed 100 times by the coupled syringe method (Caffrey and Cherezov 2009). Samples of 30 - 100 nL in volume were spotted onto 96 well glass plates and overlaid en bloc with 600 nL precipitant solution for each well. Precipitant solution consisted of 10 - 20% PEG300, 100 mM HEPES pH 7.2 - 7.9, 1.2% 1,2,3-heptanetril, and 20 80 mM EDTA pH 8.0.

**Data collection.** Grids of crystals were rastered at Advanced Photon Source beamlines 23ID-B and 23ID-D. Initial rastering was performed with an 80 µm by 30 µm beam with 5-fold attenuation and 1 sec exposure, and regions with strong diffraction were sub-rastered with a 10 µm collimated beam with equivalent X-ray dose. Data collection was similarly performed with a 10 µm beam, but with no attenuation and exposures of typically 1 - 5 s. An oscillation width of 1 - 2 degrees was used in each case, and wedges of 5 -10 degrees were compiled to create the final data sets.

**Data reduction and refinement.** Diffraction data were processed in *HKL2000* (Otwinowski and Minor, 1997), and statistics are summarized in Fig. 6. The structure was solved using molecular replacement with the structure of the inactive M2 receptor (PDB ID: 3UON) and Nb80 (PDB ID: 3POG) as search models in *Phaser* (McCoy et al. 2007). The resulting structure was iteratively refined in *PheniX* (Afonine et al. 2012) and manually rebuilt in *Coot* (Emsley et al. 2004). Final refinement statistics are summarized in Fig. 6. Figures were prepared in *PyMol* (Schrödinger).

### Example 1. Conformational selections of M2 Gi-mimetic nanobodies by yeast display

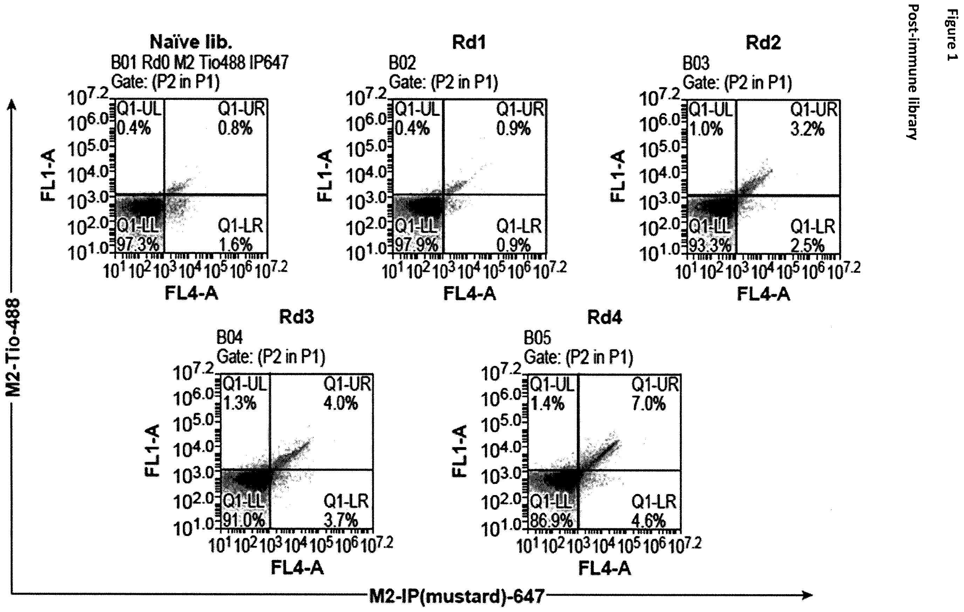

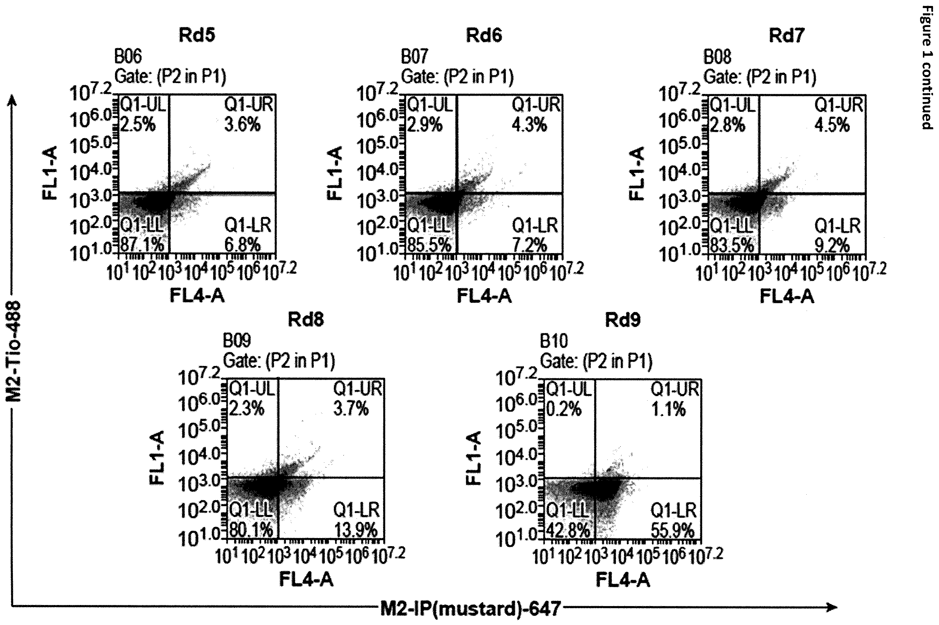

Conformationally specific Gi mimetic proteins were identified for the M2 muscarinic receptor (for experimental details, see also section "Methods to the Examples" as described above). First, llamas were immunized with M2 receptor bound to the agonist iperoxo, and a phage-displayed post-immune single variable domain (VHH) library was constructed. Unlike the case of the β2AR, standard biopanning techniques were unsuccessful in identifying conformationally-selective M2 receptor binding nanobodies. In order to specifically isolate such nanobodies, we employed a conformational selection strategy using yeast surface display. A post-immune library of llama nanobody variants was displayed on the surface of yeast and selected for the ability to bind to the M2 receptor occupied with an agonist, iperoxo. Four rounds of selections were first performed by MACS, selecting each round with agonist-bound receptor after first counter-selecting against an unrelated membrane protein (β2 adrenergic receptor). This was followed by several rounds of conformational selection using MACS where the yeast were first counter-selected against antagonist (tiotropium)-occupied M2 receptor followed by positive selection with agonist (iperoxo)-occupied M2 receptor. For the ninth and final round of selection, a FACS-based selection was employed. Yeast were simultaneously stained with Alexa647-labeled M2 receptor bound with the covalent agonist iperoxo mustard and with Alexa488-labeled M2 receptor bound to tiotropium. Yeast cells positive only for the Alexa647 label were purified, thus selecting those variants preferentially binding agonist-occupied receptor. The staining of the library during the selection process as a whole shows enrichment of nanobody variants that bind to M2 receptor occupied by the agonist iperoxo, but not to M2 receptor bound to the antagonist tiotropium, particularly after applying conformational selection in rounds 5-9 (Fig. 1).

### Example 2. Radioligand binding assays confirm that selected nanobodies stabilize the active state of M2 receptor

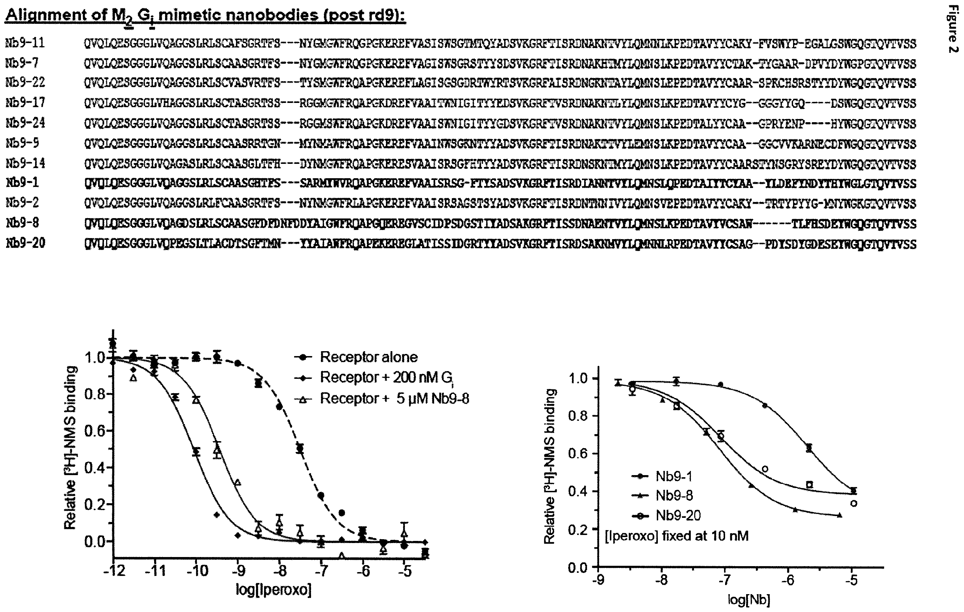

To determine whether the Nanobody variants that specifically stain agonist-bound M2 receptor are able to stabilize the M2 receptor active state, a binding assay was performed. Due to the allosteric properties of GPCRs, molecules that stabilize the active conformation of a receptor also increase agonist affinity. Several conformationally specific binders were isolated and were tested for their ability to induce an increase in the affinity of the non-covalent agonist iperoxo. Results for one of these, Nanobody clone Nb9-8, are shown in Fig. 2. Furthermore, Nb9-8 and other conformationally specific binders displayed a dose-dependent effect on agonist ability to displace a radioactive probe (Fig. 2). Nb9-8 was the most potent, with an EC50 of approximately 100 nM. At high concentrations, Nb9-8 enhanced the affinity of the M2 receptor for iperoxo to almost the same extent as that observed in the presence of the heterotrimeric G protein Gi (Fig. 2).

### Example 3. Gi mimetic nanobodies facilitate crystallization of agonist bound M2 receptor.

Initial crystallization attempts with M2 receptor bound to agonists were unsuccessful. Several attempts were made, either by fusing the M2 receptor to an amino-terminal T4 Lysozyme (T4L) or by inserting T4L into the third intracellular loop, as originally described for the β2-adrenergic receptor (Rosenbaum et al. 2007). This is most likely due to the flexibility of the intracellular receptor surface in the absence of a stabilizing protein. Therefore, a Gi protein mimetic nanobody for the M2 receptor was used to enable crystallization of the M2 receptor in its active conformation.

M2 receptor was purified in the presence of 10 µM iperoxo, and we were able to obtain crystals of iperoxo-bound M2 receptor in complex with Nb9-8 by lipidic meso phase crystallography (for experimental details, see also section "Methods to the Examples" as described above). The structure was solved by microdiffraction at Advanced Photon Source beamlines 23ID-B and 23ID-D. While several GPCRs have been crystallized in complex with agonists, only the β2AR and rhodopsin show a fully active state with adequate space to allow G protein binding (Rasmussen et al 2011; Park et al. 2008). As anticipated based on functional studies, the M2 receptor in complex with Nb9-8 shows similar structural changes, with Nb9-8 binding to the intracellular surface of the receptor (Fig. 5). Coordinates and structure factors for the active M2 receptor in complex with Nb9-8 and iperoxo are deposited in the Protein Data Bank.

### Example 4. Binding epitope of M2 Gi mimetic nanobody Nb9-8

Nb9-8 binds an intracellular cavity of M2R (SEQ ID NO: 153). The binding epitope is composed of the following elements: the side chains of residues T56 and N58 in the intracellular loop linking TM1 and TM2, the side chains of R121, C124 and V125 of TM3, the side chains of P132, V133 and R135 of the intracellular loop linking TM3 and TM4, the side chains of Y206, 1209 and S213 of TM5, the side chains of S380, V385, T388 and 1389 and the main chain atoms of R381 that are part of TM6, the main chain atoms of C439 and Y440 and the side chains of C443, A445 and T446 of TM7.

### Example 5. Selection for functional Nanobodies using M2 Gi mimetic nanobody Nb9-8.

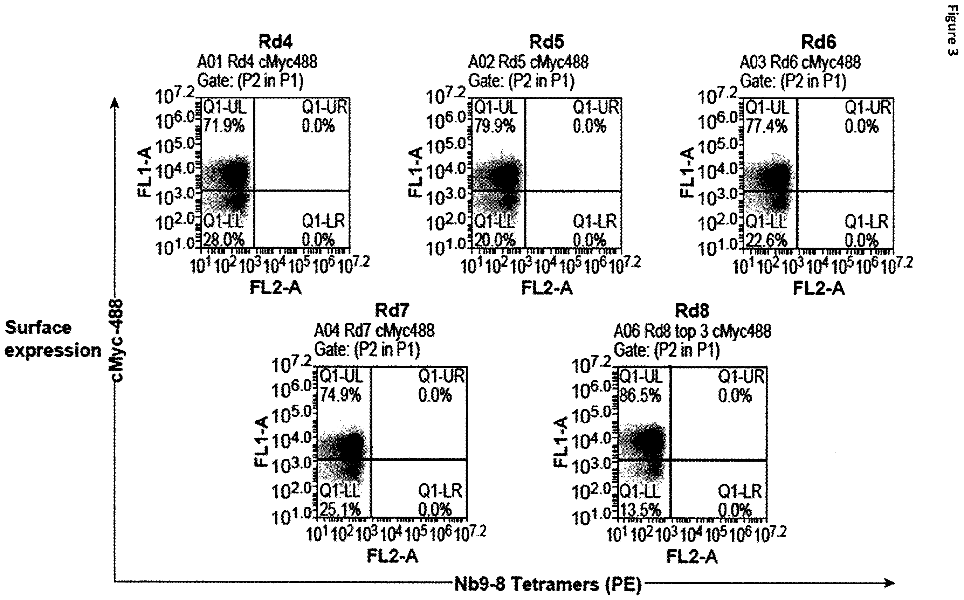

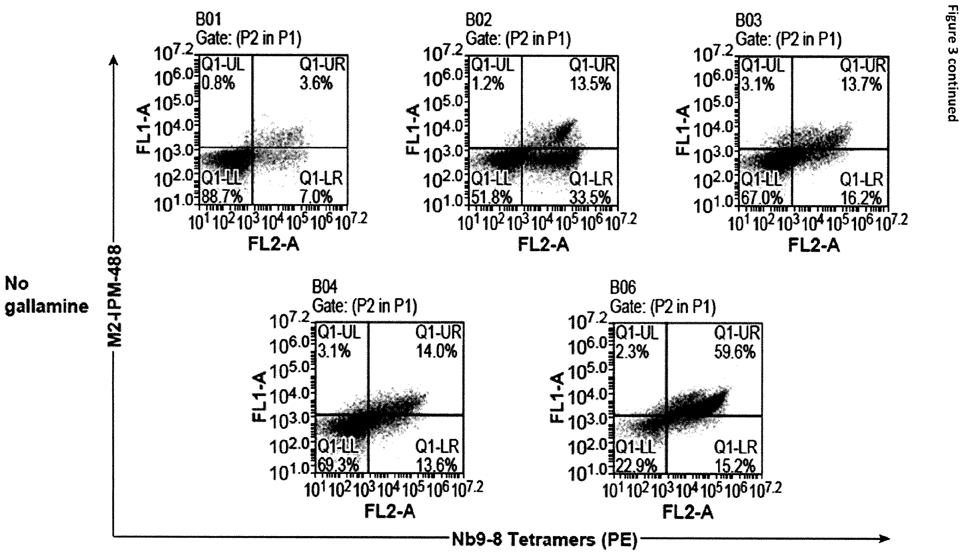

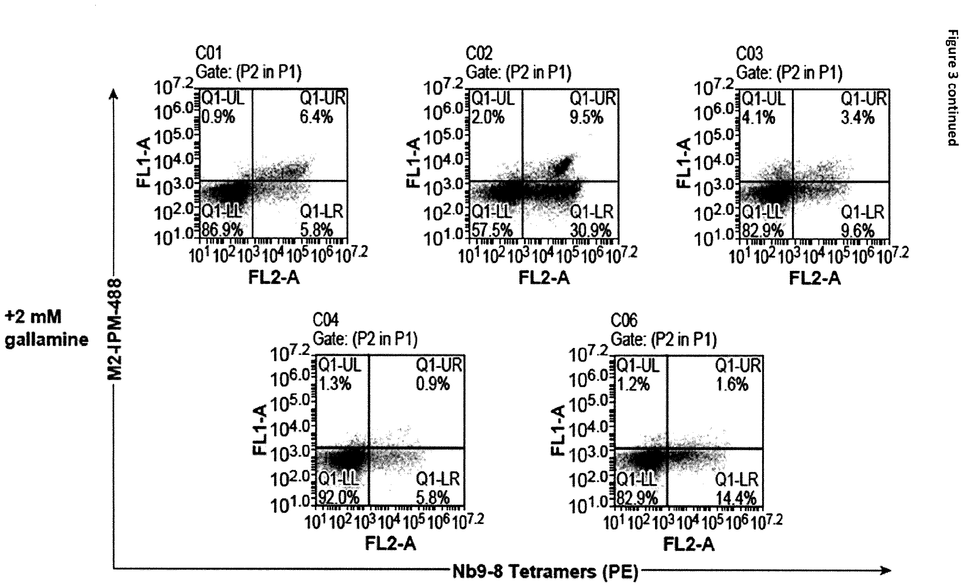

To select for functional ligands to the M2 receptor, the library resulting from the first four rounds of MACS selection described above was subjected to further selections to identify Nanobody variants that bind to the extracellular side of the receptor. First, two rounds of MACS selections were performed by selecting for the ability of variants to recruit Nb9-8 in the presence of M2 receptor, while counter-selecting for variants that bind to Nb9-8 in the absence of the M2 receptor. This selection strategy enriches for clones that either induce or are compatible with an active conformation of the M2 receptor, but that also bind to a site distinct from that of Nb9-8. To further select for variants that bind specifically to the extracellular side of the M2 receptor, counter-selection was performed against M2 receptor in the presence of the allosteric muscarinic ligand gallamine, while positively selecting those clones binding M2 receptor in the absence of gallamine. The staining of the selection process as a whole shows enrichment of Nanobody variants that bind to the M2 receptor and Nb9-8 simultaneously (Fig. 3). Furthermore, these clones are sensitive to the presence of gallamine, suggesting that they bind at the allosteric/orthosteric site of the receptor. The allosteric binding properties of several of these clones were measured by a binding assay (Fig. 4). Among the characterized variants, clone B4 (SEQ ID NO: 16) and others caused a decrease in the binding of the radioligand N-methylscopolamine only in the presence of agonist, consistent with the ability of the clone to bind at the allosteric site of the M2 receptor.

**Table 1. List of Nanobodies**

| **Nanobody reference number** | SEQ ID NO | **AMINO ACID SEQUENCE** |
|---|---|---|
| Nb9-8 | 1 | |
| Nb9-1 | 2 | |
| Nb9-11 | 3 | |
| Nb9-7 | 4 | |
| Nb9-22 | 5 | |
| Nb9-17 | 6 | |
| Nb9-24 | 7 | |
| Nb9-9 | 8 | |
| Nb9-14 | 9 | |
| Nb9-2 | 10 | |
| Nb9-20 | 11 | |
| Nb_C3 | 12 | |
| Nb_H4 | 13 | |
| Nb_El | 14 | |
| Nb_A2 | 15 | |
| Nb_B4 | 16 | |
| Nb_D3 | 17 | |
| Nb_DI | 18 | |
| Nb_HI | 19 | |

**Table 2. FRs and CDRs of M2R conformation-selective Nanobodies**

| | | **FR1** | | **CDR1** | | **FR2** | | **CDR2** | | **FR3** | | **CDR3** | | **FR4** | |
|---|---|---|---|---|---|---|---|---|---|---|---|---|---|---|---|
| **Nanobody reference number** | **SEQ ID NO** | | **SEQ ID NO** | | **SEQ ID NO** | | **SEQ** ID **NO** | | **SEQ ID NO** | | **SEQ ID NO** | | **SEQ ID NO** | | **SEQ ID NO** |
| Nb9-8 | 1 | | 20 | | 31 | | 42 | | 53 | | 64 | | 75 | | 86 |
| Nb9-1 | 2 | | 21 | | 32 | | 43 | | 54 | | 65 | | 76 | | 87 |
| Nb9-11 | 3 | | 22 | | 33 | | 44 | | 55 | | 66 | | 77 | | 88 |
| Nb9-7 | 4 | | 23 | | 34 | | 45 | | 56 | | 67 | | 78 | | 89 |
| Nb9-22 | 5 | | 24 | | 35 | | 46 | | 57 | | 68 | | 79 | | 90 |
| Nb9-17 | 6 | | 25 | | 36 | | 47 | | 58 | | 69 | | 80 | | 91 |
| Nb9-24 | 7 | | 26 | | 37 | | 48 | | 59 | | 70 | | 81 | | 92 |
| Nb9-9 | 8 | | 27 | | 38 | | 49 | | 60 | | 71 | | 82 | | 93 |
| Nb9-14 | 9 | | 28 | | 39 | | 50 | | 61 | | 72 | | 83 | | 94 |
| Nb9-2 | 10 | | 29 | | 40 | | 51 | | 62 | | 73 | | 84 | | 95 |
| Nb9-20 | 11 | | 30 | | 41 | | 52 | | 63 | | 74 | | 85 | | 96 |
| Nb_C3 | 12 | | 97 | | 105 | | 113 | | 121 | | 129 | | 137 | | 145 |
| Nb_H4 | 13 | | 98 | | 106 | | 114 | | 122 | | 130 | | 138 | | 146 |
| Nb_EI | 14 | | 99 | | 107 | | 115 | | 123 | | 131 | | 139 | | 147 |
| Nb_A2 | 15 | | 100 | | 108 | | 116 | | 124 | | 132 | | 140 | | 148 |
| Nb_B4 | 16 | | 101 | | 109 | | 117 | | 125 | | 133 | | 141 | | 149 |
| Nb_D3 | 17 | | 102 | | 110 | | 118 | | 126 | | 134 | | 142 | | 150 |
| Nb_DI | 18 | | 103 | | 111 | | 119 | | 127 | | 135 | | 143 | | 151 |
| Nb_HI | 19 | | 104 | | 112 | | 120 | | 128 | | 136 | | 144 | | 152 |

**Table 3. Examples of M2 muscarinic acetylcholine receptors**

| **Protein/subunit** | **Accession number** | **AA sequence** |
|---|---|---|
| | **(SEQ ID NO)** | |
| human M2 receptor | 153 | |
| P08172 (ACM2_HUMAN) | | |
| mouse M2 receptor | 154 | |
| Q9ERZ4 (ACM2_MOUSE) | | |
| Rat M2 receptor | 155 | |
| P10980 (ACM2_RAT) | | |

### REFERENCES

- Afonine, P. V. et al. Towards automated crystallographic structure refinement with phenix.refine. Acta Crystal/ogr D Bioi Crystal/ogr 68, 352-367, doi:l0.1107/s0907444912001308 (2012).
- Binz et al., Nature Biotech., 22: 575-582 (2004)
- Caffrey (2003). Membrane protein crystallization. J Struct. Biol. 2003 142:108-32.
- Caffrey, M. & Cherezov, V. Crystallizing membrane proteins using lipidic mesophases. Nat Protoc 4,706-731, doi:10.1038/nprot.2009.31 (2009).
- Chasin et al., 1986, Som. Cell Molec. Genet., 12:555-556.
- Chelikani et al Protein Sci. 2006 15:1433-40
- Choe, H. W. et al. Crystal structure of meta rhodopsin II. Nature 471,651-655, (2011).
- Chun, E., A. A. Thompson, W. Liu, C. B. Roth, M. T. Griffith, V. Katritch, J. Kunken, F. Xu, V. Cherezov, M. A. Hanson and R. C. Stevens (2012). "Fusion partner toolchest for the stabilization and crystallization of G protein-coupled receptors." Structure 20(6): 967-976.
- Conrath K. E., M. Lauwereys, M. Galleni et al., Antimicrob Agents Chemother 45 (10), 2807 (2001).
- Conrath, K E. et 01. Beta-lactamase inhibitors derived from single-domain antibody fragments elicited in the camelidae. Antimicrob Agents Chemother 45,2807-2812, doi:10.1128/aac.45.10.2807-2812.2001 (2001).
- Cooper M.A. (2004) J. Mol. Recognit. 17: 286-315.
- Desmyter A, Spinelli S, Payan F, Lauwereys M, Wyns L, Muyldermans S, Cambillau C. Three camelid VHH domains in complex with porcine pancreatic alpha-amylase. Inhibition and versatility of binding topology. J Biol Chem. 2002 Jun 28;277(26):23645-50.
- Desmyter A, Transue TR, Ghahroudi MA, Thi MH, Poortmans F, Hamers R, Muyldermans S, Wyns L. Crystal structure of a camel single-domain VH antibody fragment in complex with lysozyme. Nat Struct Biol. 1996 Sep;3(9):803-11.
- Deupi, X. et al. Stabilized G protein binding site in the structure of constitutively active metarhodopsin-II. Proc Natl Acad Sci USA 109, 119-124, doi:10.1073/pnas.1114089108 (2012).
- Deveraux et al. 1984, Nucleic Acids Research 12, 387-395
- Digby, G. J., Shirey, J. K. & Conn, P. J. Allosteric activators of muscarinic receptors as novel approaches for treatment of CNS disorders. Mol Biosyst 6, 1345-1354, doi:10.1039/c002938f (2010).
- Dimitrov DS. Engineered CH2 domains (nanoantibodies). MAbs. 2009 Jan-Feb;1(1):26-8.
- Dosztányi, Z., Csizmok, V., Tompa, P., & Simon, I. (2005)
- Emsley, P. & Cowtan, K Coot: model-building tools for molecular graphics. Acta Crystal/ogr D Bioi Crystal/ogr 60,2126-2132, doi:l0.1107/s0907444904019158 (2004).
- Eroglu et al EMBO 2002 3: 491^96
- Eroglu et al Proc. Natl. Acad. Sci. 2003 100: 10219-10224
- Faham et al Crystallization of bacteriorhodopsin from bicelle formulations at room temperature. Protein Sci. 2005 14:836-40. 2005
- Faham et al, Bicelle crystallization: a new method for crystallizing membrane proteins yields a monomeric bacteriorhodopsin structure. J Mol Biol. 2002 Feb 8;316(1): 1-6.
- Gebauer M, Skerra A. Engineered protein scaffolds as next-generation antibody therapeutics. Curr Opin Chem Biol. 2009 Jun;13(3):245-55.
- George et al, Nat Rev Drug Discov 1:808-820 (2002)
- Gouaux, It's not just a phase: crystallization and X-ray structure determination of bacteriorhodopsin in lipidic cubic phases. Structure. 1998 6:5-10;
- Haga, K. et al. Structure of the human M2 muscarinic acetylcholine receptor bound to an antagonist. Nature 482,547-551, doi:10.1038/nature10753 (2012). Kruse, A. C. et al. Structure and dynamics of the M3 muscarinic acetylcholine receptor. Nature 482, 552-556 (2012).
- Hamers-Casterman, C., T. Atarhouch, S. Muyldermans et al. Naturally occurring antibodies devoid of light chains. Nature 363, 446-448, doi:10.1038/363446a0 (1993).
- Ichiyama, S. et of. The structure of the third intracellular loop of the muscarinic acetylcholine receptor M2 subtype. FEBS Lett 580,23-26, doi:10.1016/j.febslet.2005.11.042 (2006).
- Jung Y, Jeong JY, Chung BH. Recent advances in immobilization methods of antibodies on solid supports. Analyst. 2008 Jun;133(6):697-701. doi: 10.1039/b800014j. Epub 2008 Apr 17.
- Kallwass et al, Biotechnol. Lett., 15 (1), 29-34,1993
- Kenakin, Trends Pharmacol Sci 25:186-192 (2002)
- Keov, P., Sexton, P. M. & Christopoulos, A. Allosteric modulation of G proteincoupled receptors: a pharmacological perspective. Neuropharmacology 60, 24-35, doi:10.1016/j.neuropharm.2010.07.010 (2011).
- Kobilka, B. K. Amino and carboxyl terminal modifications to facilitate the production and purification of a G protein-coupled receptor. Anal Biochem 231, 269-271 (1995).
- Koide et al, J. Mol. Biol., 284: 1141-1151 (1998)
- Koide, S. (2009). Engineering of recombinant crystallization chaperones. Current Opinion in Structural Biology, 19, 449.
- Kolkekar et al., 1997, Biochemistry, 36:10901-10909.
- Korotkov KV, Pardon E, Steyaert J, Hol WG. Crystal structure of the N-terminal domain of the secretin GspD from ETEC determined with the assistance of a nanobody. Structure. 2009 Feb 13;17(2):255-65.
- Kruse, A. C. et al. Structure and dynamics of the M3 muscarinic acetylcholine receptor. Nature 482, 552-556 (2012).
- Kubo, T. et al. Primary structure of porcine cardiac muscarinic acetylcholine receptor deduced from the cDNA sequence. FEBS Lett 209,367-372 (1986).
- Landau et al, Lipidic cubic phases: a novel concept for the crystallization of membrane proteins. Proc. Natl. Acad. Sci. 1996 93:14532-5
- Lefranc, M. P., C. Pommie, et al. (2003). "IMGT unique numbering for immunoglobulin and T cell receptor variable domains and lg superfamily V-like domains." Developmental and Comparative Immunology 27(1): 55-77.
- Luca et al Proc. Natl. Acad. Sci. 2003 100 :10706-I 1
- Mansoor et al Proc. Natl. Acad. Sci. 2006 103: 3060-3065
- Mather, 1980, Biol. Reprod., 23:243-251
- Mather, 1982, Annals NYAcad. Sci., 383:44-68
- McCoy, A. J. et al. Phaser crystallographic software.] Appl Crystal/ogr 40, 658-674, doi:10.1107/s0021889807021206 (2007).
- Mohr, K., Trankle, C. & Holzgrabe, U. Structure/activity relationships of M2 muscarinic allosteric modulators. Receptors Channels 9, 229-240 (2003).
- Niu et al, Biophys J. 2005 89: 1833-1840
- Nollert et al Lipidic cubic phases as matrices for membrane protein crystallization Methods. 2004 34:348-53
- Nygaard, R. et a1. The dynamic process of beta(2)-adrenergic receptor activation. Cell 152, 532-542, doi:10.1016/j.cel1.2013.01.008 (2013).
- Nygren, P-A. (2008) Alternative binding proteins: affibody binding proteins developed from a small three-helix bundle scaffold. FEBS J. 275, 2668-2676.
- Otwinowski, Z. & Minor, W. in Methods in Enzymology Vol. Volume 276 (ed Charles W. Carter, Jr.) 307-326 (Academic Press, 1997).
- Park JH, Scheerer P, Hofmann KP, Choe HW, Ernst OP. Crystal structure of the ligand-free G-protein-coupled receptor opsin. Nature. 2008 Jul 10;454(7201):183-7. doi: 10.1038/nature07063. Epub 2008 Jun 18.
- Peterson, G. L., Herron, G. S., Yamaki, M., Fullerton, D. S. & Schimerlik, M. I. Purification of the muscarinic acetylcholine receptor from porcine atria. Proc Natl Acad Sci USA 81, 4993-4997 (1984).
- Qian ZM, Li H, Sun H and Ho K (2002). Targeted drug delivery via the transferring receptor-mediated endocytosis pathway. Pharmacol Rev 54, 561-587.
- Rasmussen, S. G. et al. Crystal structure of the beta2 adrenergic receptor-Gs protein complex. Nature 477,549-555, doi:10.1038/nature10361 (2011a).
- Rasmussen, S. G., H. J. Choi, J. J. Fung, E. Pardon, P. Casarosa, P. S. Chae, B. T. Devree, D. M. Rosenbaum, F. S. Thian, T. S. Kobilka, A. Schnapp, I. Konetzki, R. K. Sunahara, S. H. Gellman, A. Pautsch, J. Steyaert, W. I. Weis and B. K. Kobilka (2011b). "Structure of a nanobody-stabilized active state of the beta(2) adrenoceptor." Nature 469(7329): 175-180.
- Reeves et al. 2002, PNAS, 99: 13419
- Riechmann and Muyldermans J. Immunol. Methods 2000; 240: 185-195.
- Rios et al., Pharmacol Ther 92:71-87 (2001)).
- Rosenbaum, D. M., V. Cherezov, M. A. Hanson, S. G. Rasmussen, F. S. Thian, T. S. Kobilka, H. J. Choi, X. J. Yao, W. I. Weis, R. C. Stevens and B. K. Kobilka (2007). "GPCR engineering yields high-resolution structural insights into beta2-adrenergic receptor function." Science 318(5854): 1266-1273.
- Rummel et al, Lipidic Cubic Phases: New Matrices for the Three-Dimensional Crystallization of Membrane Proteins. J. Struct. Biol. 1998 121 :82-91;
- Sawant R, Torchilin V. Intracellular transduction using cell-penetrating peptides. Mol Biosyst. 2010 Apr;6(4):628-40. Epub 2009 Dec 21.
- Scheerer, P. et a1. Crystal structure of opsin in its G-protein-interacting conformation. Nature 455, 497-502, doi:10.1038/nature07330 (2008).
- Shapiro, R A. & Nathanson, N. M. Deletion analysis of the mouse m1 muscarinic acetylcholine receptor: effects on phosphoinositide metabolism and down-regulation. Biochemistry 28, 8946-8950 (1989).
- Shimada et al J. Biol. Chem. 2002 277:31774-80
- Skerra, J. Molecular Recognition, 13:167-187 (2000).
- Starovasnik MA, Braisted AC, Wells JA. Structural mimicry of a native protein by a minimized binding domain.Proc Natl Acad Sci USA. 1997 Sep 16; 94(19):10080-5.
- Urlaub and Chasin, 1980, Proc. Natl. Acad. Sci. USA, 77:4216
- Wesolowski, J., Alzogaray, V., Reyelt, J., Unger, M., Juarez, K., Urrutia, M., Cauerhiff, A., Danquah, W., Rissiek, B., Scheuplin, F., Schwarz, N., Adriouch, S., Boyer, O., Seman, M., Licea, A., Serreze, D.V., Goldbaum, F.A., Haag, F. and Koch-Nolte, F. (2009). Single domain antibodies: promising experimental and therapeutic tools in infection and immunity. Med. Microbiol. Immunol. 198, 157-174.
- Wess, J., Eglen, R M. & Gautam, D. Muscarinic acetylcholine receptors: mutant mice provide new insights for drug development. Nat Rev. Drug Discov. 6,721-733 (2007).
- Yao XJ, Vélez Ruiz G, Whorton MR, Rasmussen SG, DeVree BT, Deupi X, Sunahara RK, Kobilka B. The effect of ligand efficacy on the formation and stability of a GPCR-G protein complex. Proc Natl Acad Sci USA. 2009 Jun 9;106(23):9501-6.

## Claims

1. An active conformation-selective VHH antibody that is directed against and capable of specifically binding to an active conformation of a muscarinic receptor M2 (M2R), wherein the VHH antibody comprises an amino acid sequence that comprises four framework regions (FR1 to FR4) and three complementary determining regions (CDR1 to CDR3) according to the formula: FR1-CDR1-FR2-CDR2-FR3-CDR3-FR4, wherein CDR1 is SEQ ID NO: 31, and CDR2 is SEQ ID NO: 53, and CDR3 is SEQ ID NO: 75; or wherein CDR1 is SEQ ID NO: 32, and CDR2 is SEQ ID NO: 54, and CDR3 is SEQ ID NO: 76; or wherein CDR1 is SEQ ID NO: 41, and CDR2 is SEQ ID NO: 63, and CDR3 is SEQ ID NO: 85.

2. The VHH antibody of claim 1, wherein the VHH antibody comprises an amino acid sequence selected from the group consisting of SEQ ID NO:1, SEQ ID NO:2 and SEQ ID NO:11.

3. The VHH antibody of claim 1, wherein the VHH antibody is a G protein mimetic.

4. The VHH antibody of any of claims 1 to 3, wherein the VHH antibody is comprised in a polypeptide.

5. The VHH antibody of any of claims 1 to 4, wherein the VHH antibody is immobilized on a solid support.

6. A complex, preferably a crystalline complex, comprising muscarinic receptor M2 (M2R) and an active conformation-selective M2R VHH antibody according to any one of claims 1 to 5, and optionally at least one other conformation-selective receptor ligand.

7. A non-cellular composition, preferably a membrane composition, comprising the complex of claim 6.

8. An isolated cellular composition comprising the complex of claim 6.

9. A nucleic acid molecule comprising a nucleic acid sequence encoding an amino acid sequence of a VHH antibody of any of claims 1-5.

10. A host cell comprising the nucleic acid sequence of claim 9.

11. A method of identifying conformation-selective compounds targeting muscarinic receptor M2, the method comprising the steps of
(i) Providing a complex comprising a muscarinic receptor M2 (M2R) and a conformation-selective M2R VHH antibody according to any one of claims 1 to 5, wherein M2R is in an active conformation, and
(ii) Providing a test compound, and
(iii) Evaluating the selective binding of the test compound to the M2R comprised in the complex.

12. Use of the VHH antibody of any of claims 1-5 as a means for crystallization.

13. Use of the VHH antibody of any of claims 1-5 or the complex of claim 6 as a means for compound screening and drug discovery or as a means for capturing and/or purification of molecules.

14. A pharmaceutical composition comprising a therapeutically effective amount of an active conformation-selective VHH antibody of any of claims 1-5 and at least one of a pharmaceutically acceptable carrier, adjuvant or diluents.

15. *In vitro* use of the active conformation-selective VHH antibody of any of claims 1-5 to modulate M2R signaling activity.

## Patentansprüche

1. Aktiver konformationsselektiver VHH-Antikörper,
der sich gegen eine aktive Konformation eines muskarinischen Rezeptors M2 (M2R) richtet und sich spezifisch daran binden kann, wobei der VHH-Antikörper eine Aminosäuresequenz umfasst, die vier Rahmen-Regionen (FR1 bis FR4) und drei komplementäre Bestimmungsregionen (CDR1 bis CDR3) umfasst, entsprechend der Formel: FR1-CDR1-FR2-CDR2-FR3-CDR3-FR4, wobei CDR1 SEQ ID NO: 31 ist, und CDR2 SEQ ID NO: 53 ist, und CDR3 SEQ ID NO: 75 ist; oder wobei CDR1 SEQ ID NO: 32 ist, und CDR2 SEQ ID NO: 54 ist, und CDR3 SEQ ID NO: 76 ist; oder wobei CDR1 SEQ ID NO: 41 ist, und CDR2 SEQ ID NO: 63 ist, und CDR3 SEQ ID NO: 85 ist.

2. VHH-Antikörper nach Anspruch 1, wobei der VHH-Antikörper eine Aminosäuresequenz umfasst, die aus folgender Gruppe ausgewählt wurde: SEQ ID NO:1, SEQ ID NO:2 und SEQ ID NO:11.

3. VHH-Antikörper nach Anspruch 1, wobei der VHH-Antikörper G-Protein-mimetisch ist.

4. VHH-Antikörper nach einem der Ansprüche 1 bis 3, wobei der VHH-Antikörper in einem Polypeptid enthalten ist.

5. VHH-Antikörper nach einem der Ansprüche 1 bis 4, wobei der VHH-Antikörper in einem festen Träger immobilisiert ist.

6. Komplex, vorzugsweise ein kristalliner Komplex, mit Muskarinrezeptor M2 (M2R) und einem konformationsselektiven aktiven Antikörper M2R VHH nach einem der Ansprüche 1 bis 5, und gegebenenfalls mindestens einem anderen konformationsselektiven Rezeptorliganden.

7. Nicht-zelluläre Zusammensetzung, vorzugsweise eine Membranzusammensetzung, die den Komplex nach Anspruch 6 umfasst.

8. Isolierte zelluläre Zusammensetzung, die den Komplex nach Anspruch 6 umfasst.

9. Nukleinsäuremolekül, das eine Nukleinsäuresequenz umfasst, die eine Aminosäuresequenz eines VHH-Antikörpers nach einem der Ansprüche 1 bis 5 kodiert.

10. Wirtszelle, welche die Nukleinsäuresequenz nach Anspruch 9 umfasst.

11. Verfahren zur Identifizierung von konformationsselektiven Verbindungen, die auf den Muskarinrezeptor M2 abzielen, wobei das Verfahren die folgenden Schritte umfasst:
(i) Bereitstellen eines Komplexes, der einen Muskarinrezeptor M2 (M2R) und einen konformationsselektiven M2R-VHH-Antikörper nach einem der Ansprüche 1 bis 5 umfasst, wobei M2R in einer aktiven Konformation vorliegt, und
(ii) Bereitstellen einer Testverbindung und
(iii) Bewertung der selektiven Bindung der Testverbindung an den im Komplex enthaltenen M2R.

12. Verwendung des VHH-Antikörpers nach einem der Ansprüche 1 bis 5 als Mittel zur Kristallisation.

13. Verwendung des VHH-Antikörpers nach einem der Ansprüche 1 bis 5 oder des Komplexes nach Anspruch 6 als Mittel zum Screening von Verbindungen und Entdecken von Medikamenten oder als Mittel zum Einfangen und / oder Reinigen von Molekülen.

14. Pharmazeutische Zusammensetzung, die eine therapeutisch wirksame Menge eines aktiven konformationsselektiven VHH-Antikörpers nach einem der Ansprüche 1 bis 5 und mindestens einen pharmazeutisch verträglichen Träger, Hilfsmittel und/oder Verdünnungsmittel umfasst.

15. In-vitro-Verwendung des aktiven konformationsselektiven VHH-Antikörpers nach einem der Ansprüche 1 bis 5 zur Modulation der M2R-Signalaktivität

## Revendications

1. Anticorps VHH sélectif pour une conformation active qui est dirigé contre et qui est capable de se lier spécifiquement à une conformation active d'un récepteur muscarinique M2 (M2R), où l'anticorps VHH comprend une séquence d'acides aminés qui comprend quatre régions charpentes (FR1 à FR4) et trois régions déterminantes complémentaires (CDR1 à CDR3) selon la formule: FR1-CDR1-FR2-CDR2-FR3-CDR3-FR4, où CDR1 est la SEQ ID NO: 31, et CDR2 est la SEQ ID NO: 53, et CDR3 est la SEQ ID NO: 75; ou où CDR1 est la SEQ ID NO: 32, et CDR2 est la SEQ ID NO: 54, et CDR3 est la SEQ ID NO: 76; ou où CDR1 est la SEQ ID NO: 41, et CDR2 est la SEQ ID NO: 63, et CDR3 est la SEQ ID NO: 85.

2. Anticorps VHH selon la revendication 1, où l'anticorps VHH comprend une séquence d'acides aminés sélectionnée du groupe constitué par la SEQ ID NO:1, la SEQ ID NO:2 et la SEQ ID NO:11.

3. Anticorps VHH selon la revendication 1, où l'anticorps VHH est un mimétique de la protéine G.

4. Anticorps VHH selon l'une quelconque des revendications 1 à 3, où l'anticorps VHH est compris dans un polypeptide.

5. Anticorps VHH selon l'une quelconque des revendications 1 à 4, où l'anticorps VHH est immobilisé sur un support solide.

6. Complexe, de préférence un complexe cristallin, comprenant le récepteur muscarinique M2 (M2R) et un anticorps VHH sélectif pour une conformation active de M2R selon l'une quelconque des revendications 1 à 5, et éventuellement au moins un autre ligand de récepteur sélectif pour une conformation.

7. Composition non cellulaire, de préférence une composition membranaire, comprenant le complexe selon la revendication 6.

8. Composition cellulaire isolée comprenant le complexe selon la revendication 6.

9. Molécule d'acide nucléique comprenant une séquence d'acides nucléiques codifiant une séquence d'acides aminés d'un anticorps VHH selon l'une quelconque des revendications 1-5.

10. Cellule hôte comprenant la séquence d'acides nucléiques selon la revendication 9.

11. Procédé d'identification de composés sélectifs pour une conformation dirigés au récepteur muscarinique M2, le procédé comprenant les étapes de
(i) fournir un complexe comprenant un récepteur muscarinique M2 (M2R) et un anticorps VHH sélectif pour une conformation active de M2R selon l'une quelconque des revendications 1 à 5, où le M2R est dans une conformation active, et
(ii) fournir un composé d'essai, et
(iii) évaluer la liaison sélective du composé d'essai au M2R compris dans le complexe.

12. Utilisation de l'anticorps VHH selon l'une quelconque des revendications 1-5 en tant que moyen de cristallisation.

13. Utilisation de l'anticorps VHH selon l'une quelconque des revendications 1-5 ou du complexe de la revendication 6 en tant que moyen de sélection de composés et de découverte de médicaments ou en tant que moyen de capture et/ou de purification de molécules.

14. Composition pharmaceutique comprenant une quantité thérapeutiquement efficace d'un anticorps VHH sélectif pour une conformation active selon l'une quelconque des revendications 1-5 et au moins un parmi un support, un adjuvant ou un diluant pharmaceutiquement acceptables.

15. Utilisation *in vitro* de l'anticorps VHH sélectif pour une conformation active selon l'une quelconque des revendications 1-5 pour moduler l'activité de signalisation de M2R.
